# EUROPÄISCHE PATENTSCHRIFT

(11) **EP 2 791 105 B1**
(45) Veröffentlichungstag und Bekanntmachung des Hinweises auf die Patenterteilung: **18.03.2020**
(21) Anmeldenummer: 12788444.3
(22) Anmeldetag: 15.11.2012
(51) Int. Cl.: C07C 209/10, C07C 211/55, H01L 51/00

(54) **VERBINDUNGEN FÜR ELEKTRONISCHE VORRICHTUNGEN**
COMPOUNDS FOR ELECTRONIC DEVICES
COMPOSÉS DESTINÉS À DES DISPOSITIFS ÉLECTRONIQUES

(30) Priorität: 12.12.2011 EP 11009779
(43) Veröffentlichungstag der Anmeldung: 22.10.2014
(73) Patentinhaber: Merck Patent GmbH, 64293 Darmstadt (DE)
(72) Erfinder: STOESSEL, Philipp, 60487 Frankfurt am Main (DE); VOGES, Frank, 67098 Bad Duerkheim (DE); BUESING, Arne, 65929 Frankfurt am Main (DE); PFLUMM, Christof, 64291 Darmstadt (DE); MUJICA-FERNAUD, Teresa, 64283 Darmstadt (DE); WIRGES, Christian, 64287 Darmstadt (DE)
(86) Internationale Anmeldenummer: PCT/EP2012/004744
(87) Internationale Veröffentlichungsnummer: WO 2013/087142

(56) Entgegenhaltungen:
- EP-A2- 0 376 311
- EP-A2- 1 683 804
- JP-A- H1 095 972
- JP-A- 2003 201 472
- JP-A- 2009 147 276
- JP-A- 2011 084 717
- US-A- 4 992 350
- US-A1- 2009 072 718
- US-A1- 2009 102 368
- US-A1- 2010 099 890
- US-A1- 2010 237 339
- US-A1- 2011 198 581
- SIYI WANG ET AL: "N,N-Diarylanilinosquaraines and their application to organic photovoltaics", CHEMISTRY OF MATERIALS, Bd. 23, 18. Oktober 2011 (2011-10-18), Seiten 4789-4798, XP002691608, AMERICAN CHEMICAL SOCIETY, WASHINGTON, DC ISSN: 1520-5002
- CUI-HUA ZHAO ET AL: "Highly solid-state emissive para-terphenyls laterally substituted with a diphenylamino group", CHEMICAL COMMUNICATIONS, Bd. 47, 2011, Seiten 5518-5520, XP002691609, SEINSTITUTE OF INORGANIC AND PHYSICAL CHEMISTRY, STOCKHOLM, ISSN: 0366-5607
- D. HELLWINKEL ET AL: "Ringschlussreaktionen von 2'-heterosubstituierten Biphenyl-2-diazonium-Salzen zu (spiro)cyclischen Tetraarylammonium-Salzen und Tribenz(b.d.f) azepinen", CHEMISCHE BERICHTE., Bd. 105, 1972, Seiten 880-906, XP002691610, DEVERLAG CHEMIE GMBH. WEINHEIM. ISSN: 0009-2940

## Beschreibung

Gegenstand der Erfindung sind Arylaminoverbindungen sowie ihre Verwendung in elektronischen Vorrichtungen, beispielsweise organischen Elektrolumineszenzvorrichtungen. Weiterer Gegenstand der Erfindung sind elektronische Vorrichtungen enthaltend eine oder mehrere der genannten Verbindungen, beispielsweise als Lochtransportmaterialien in einer entsprechenden Funktionsschicht der Vorrichtung. Weiterhin Gegenstand der Erfindung ist ein Verfahren zur Herstellung der genannten Verbindungen, sowie eine Formulierung enthaltend eine oder mehrere der genannten Verbindungen.

Die Entwicklung von funktionellen Verbindungen zur Verwendung in elektronischen Vorrichtungen ist von hohem Interesse. Ziel ist hierbei insbesondere die Entwicklung von Verbindungen, mit denen verbesserte Eigenschaften der elektronischen Vorrichtungen in einem oder mehreren relevanten Punkten erzielt werden können, wie beispielsweise Leistungseffizienz, Lebensdauer oder Farbkoordinaten des emittierten Lichts.

Unter dem Begriff elektronische Vorrichtung werden gemäß der vorliegenden Erfindung unter anderem organische integrierte Schaltungen (OICs), organische Feld-Effekt-Transistoren (OFETs), organische Dünnfilmtransistoren (OTFTs), organische lichtemittierende Transistoren (OLETs), organische Solarzellen (OSCs), organische optische Detektoren, organische Photorezeptoren, organische Feld-Quench-Devices (OFQDs), organische lichtemittierende elektrochemische Zellen (OLECs), organische Laserdioden (O-Laser) und organische Elektrolumineszenzvorrichtungen (OLEDs) verstanden.

Von besonderem Interesse ist die Bereitstellung von Verbindungen zur Verwendung in den zuletzt genannten, als OLEDs bezeichneten elektronischen Vorrichtungen. Der allgemeine Aufbau sowie das Funktionsprinzip von OLEDs ist dem Fachmann bekannt und unter anderem in US 4539507, US 5151629, EP 0676461 und WO 1998/27136 beschrieben. EP376311 offenbart Verbindungen, die sich in der Position des zweiten Phenylrings unterscheiden.

Betreffend die Leistungsdaten von OLEDs sind noch weitere Verbesserungen erforderlich, insbesondere in Hinblick auf eine breite kommerzielle Verwendung, beispielsweise in Anzeigevorrichtungen oder als Lichtquellen. Von besonderer Bedeutung sind in diesem Zusammenhang die Lebensdauer, die Effizienz und die Betriebsspannung der OLEDs sowie die realisierten Farbwerte. Insbesondere bei blau emittierenden OLEDs besteht Verbesserungspotential bezüglich der Lebensdauer der Vorrichtungen. Zudem ist es wünschenswert, dass die Verbindungen zur Verwendung als Funktionsmaterialien in elektronischen Vorrichtungen eine hohe thermische Stabilität und eine hohe Glasübergangstemperatur aufweisen und sich unzersetzt sublimieren lassen.

In diesem Zusammenhang besteht insbesondere Bedarf an alternativen Lochtransportmaterialien. Bei Lochtransportmaterialien gemäß dem Stand der Technik steigt im Allgemeinen die Spannung mit der Schichtdicke der Lochtransportschicht an. In der Praxis wäre häufig eine höhere Schichtdicke der Lochtransportschicht wünschenswert, dies hat jedoch oftmals eine höhere Betriebsspannung und schlechtere Leistungsdaten zur Folge. In diesem Zusammenhang besteht Bedarf an neuen Lochtransportmaterialien, die eine hohe Ladungsträgerbeweglichkeit aufweisen, so dass dickere Lochtransportschichten mit lediglich geringem Anstieg der Betriebsspannung realisiert werden können.

Weiterhin besteht Bedarf an Lochtransportmaterialien, welche sich einfach und kostengünstig herstellen lassen. Nochmals weiterhin besteht Bedarf an Lochtransportmaterialien, welche in hochreiner Form hergestellt werden können.

Im Stand der Technik bekannt (JP 1995/053955 A) ist die Verwendung von Triarylamino-Verbindungen wie beispielsweise Tris-(para-biphenyl)amin oder Tris-(para-triphenyl)amin in OLEDs.

Weiterhin im Stand der Technik bekannt (WO 2006/123667 A1 und JP 2010/222268 A) ist die Verwendung von Triarylamino-Verbindungen, welche para- und meta-Verknüpfungen der einzelnen aromatischen Ringe aufweisen, in OLEDs.

Nochmals weiterhin wird in JP 2007/91719 A die Verwendung von Triarylamino-Verbindungen, welche eine 1,3,5-Triphenyl-substituierte Phenylgruppe als Arylgruppe aufweisen, in OLEDs offenbart.

Es besteht jedoch weiterhin Bedarf an Verbindungen, welche sich als Funktionsmaterialien in OLEDs, insbesondere als Lochtransportmaterialien eignen.

Es wurde nun gefunden, dass sich Verbindungen der unten gezeigten Formel (I) hervorragend als Funktionsmaterialien in OLEDs verwenden lassen.

Gegenstand der Erfindung ist somit eine Verbindung der Formel (I) 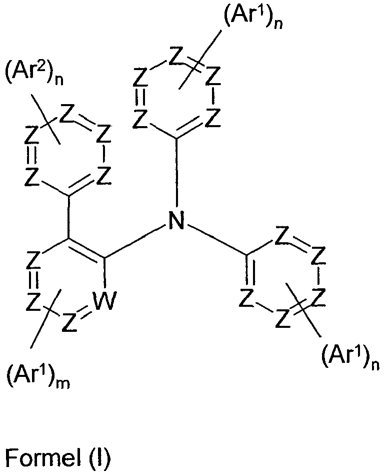 wobei für die auftretenden Symbole und Indices gilt:
- Z: ist bei jedem Auftreten gleich oder verschieden CR¹ oder N, wobei Z gleich C ist, wenn eine Gruppe Ar¹ oder Ar² gebunden ist;
- W: ist gleich CH oder N;
- Ar¹: ist bei jedem Auftreten gleich oder verschieden ein aromatisches Ringsystem mit 6 bis 30 aromatischen Ringatomen, welches mit einem oder mehreren Resten R¹ substituiert sein kann;
- Ar²: ist bei jedem Auftreten gleich oder verschieden ein aromatisches Ringsystem mit 6 bis 24 aromatischen Ringatomen, welches mit einem oder mehreren Resten R¹ substituiert sein kann;
- R¹: ist bei jedem Auftreten gleich oder verschieden H, D, F, Cl, Br, I, C(=O)R², CN, Si(R²)₃, NO₂, P(=O)(R²)₂, S(=O)R², S(=O)₂R², eine geradkettige Alkyl-, Alkoxy- oder Thioalkylgruppe mit 1 bis 20 C-Atomen oder eine verzweigte oder cyclische Alkyl-, Alkoxy- oder Thioalkylgruppe mit 3 bis 20 C-Atomen oder eine Alkenyl- oder Alkinylgruppe mit 2 bis 20 C-Atomen, wobei die oben genannten Gruppen jeweils mit einem oder mehreren Resten R² substituiert sein können und wobei eine oder mehrere CH₂-Gruppen in den oben genannten Gruppen durch -R²C=CR²-, -C≡C-, Si(R²)₂, C=O, C=S, C=NR², -C(=O)O-, -C(=O)NR²-, NR², P(=O)(R²), -O-, -S-, SO oder SO₂ ersetzt sein können und wobei ein oder mehrere H-Atome in den oben genannten Gruppen durch D, F, Cl, Br, I, CN oder NO₂ ersetzt sein können, oder ein aromatisches Ringsystem mit 6 bis 30 aromatischen Ringatomen, das jeweils durch einen oder mehrere Reste R² substituiert sein kann, wobei zwei oder mehr Reste R¹ miteinander verknüpft sein können und einen Ring bilden können;
- R²: ist bei jedem Auftreten gleich oder verschieden H, D, F, Cl, Br, I, C(=O)R³, CN, Si(R³)₃, NO₂, P(=O)(R³)₂, S(=O)R³, S(=O)₂R³, eine geradkettige Alkyl-, Alkoxy- oder Thioalkylgruppe mit 1 bis 20 C-Atomen oder eine verzweigte oder cyclische Alkyl-, Alkoxy- oder Thioalkylgruppe mit 3 bis 20 C-Atomen oder eine Alkenyl- oder Alkinylgruppe mit 2 bis 20 C-Atomen, wobei die oben genannten Gruppen jeweils mit einem oder mehreren Resten R³ substituiert sein können und wobei eine oder mehrere CH₂-Gruppen in den oben genannten Gruppen durch -R³C=CR³-, -C≡C-, Si(R³)₂, C=O, C=S, C=NR³, -C(=O)O-, -C(=O)NR³-, NR³, P(=O)(R³), -O-, -S-, SO oder SO₂ ersetzt sein können und wobei ein oder mehrere H-Atome in den oben genannten Gruppen durch D, F, Cl, Br, I, CN oder NO₂ ersetzt sein können, oder ein aromatisches Ringsystem mit 6 bis 30 aromatischen Ringatomen, das jeweils durch einen oder mehrere Reste R³ substituiert sein kann, wobei zwei oder mehr Reste R² miteinander verknüpft sein können und einen Ring bilden können;
- R³: ist bei jedem Auftreten gleich oder verschieden H, D, F oder ein aliphatischer oder, aromatischer organischer Rest mit 1 bis 20 C-Atomen, in dem auch ein oder mehrere H-Atome durch D oder F ersetzt sein können; dabei können zwei oder mehr Substituenten R³ miteinander verknüpft sein und einen Ring bilden;
- n: ist bei jedem Auftreten gleich oder verschieden 0, 1, 2, 3, 4 oder 5;
- m: ist bei jedem Auftreten gleich oder verschieden 0, 1, 2 oder 3;
wobei die drei Gruppen, welche an das zentrale Stickstoffatom binden, als ganze gesehen, nicht alle identisch sind, wobei die summe der Indices n mindestens gleich 2 ist, und wobei die folgenden Verbindungen vom Anspruchsumfang ausgenommen sind:

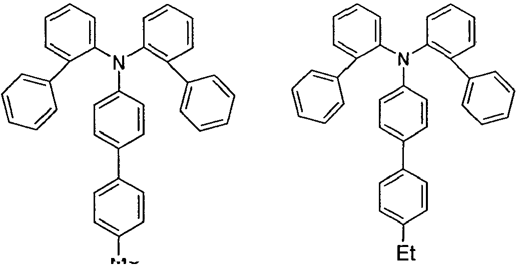

Die erfindungsgemäßen Verbindungen bzw. die erfindungsgemäßen organischen Elektrolumineszenzvorrichtungen zeichnen sich durch folgende überraschende Vorteile gegenüber dem Stand der Technik aus:
Die erfindungsgemäßen Verbindungen eignen sich sehr gut für den Einsatz in einer Lochtransportschicht oder einer Lochinjektionsschicht einer organischen Elektrolumineszenzvorrichtung, insbesondere aufgrund ihrer hohen Lochbeweglichkeit.

Die Verbindungen sind synthetisch einfach zugänglich und lassen sich kostengünstig herstellen.

Die Verbindungen bewirken bei Verwendung in OLEDs eine hohe Lebensdauer der Vorrichtungen, bevorzugt in Kombination mit einer hohen Effizienz.

Weiterhin zeichnen sich die Verbindungen durch eine geringe Kristallisationsneigung aus, insbesondere verglichen mit symmetrischen Verbindungen.

Im Folgenden sind allgemeine Begriffsdefinitionen aufgeführt, die im Rahmen der vorliegenden Anmeldung gelten.

Unter der Formulierung, dass die drei an das zentrale Stickstoffatom bindenden Gruppen, als ganze gesehen, nicht alle identisch sind, wird verstanden, dass die drei kompletten chemischen Gruppen, welche an das zentrale Stickstoffatom binden, nicht alle identisch sind. Dabei werden auch Substituenten berücksichtigt. Es wird nicht darauf abgestellt, ob der unmittelbar an das Stickstoffatom bindende Teil der Gruppe, beispielsweise die erste Phenylgruppe einer Terphenylgruppe gleich oder verschieden verglichen mit einem entsprechenden Teil der anderen Gruppen ist.

Bevorzugt sind zwei der drei kompletten chemischen Gruppen, welche an das zentrale Stickstoffatom binden, gleich. Gemäß einer alternativen bevorzugten Ausführungsform sind alle drei Gruppen verschieden.

Bevorzugt enthält die Verbindung der Formel (I) keine dreizählige Symmetrieachse durch das Stickstoffatom.

Unter einer Substitution mit -(Ar¹)ₙ wird verstanden, dass n gleiche oder verschiedene Gruppen Ar¹ an den entsprechenden Sechsring binden, jeweils in unterschiedlichen Positionen des Sechsrings. Entsprechendes gilt für eine Substitution mit -(Ar²)ₘ.

Eine Arylgruppe im Sinne dieser Erfindung enthält 6 bis 60 aromatische Ringatome; eine Heteroarylgruppe im Sinne dieser Erfindung enthält 5 bis 60 aromatische Ringatome, von denen mindestens eines ein Heteroatom darstellt. Die Heteroatome sind bevorzugt ausgewählt aus N, O und S. Dies stellt die grundlegende Definition dar. Werden in der Beschreibung der vorliegenden Erfindung andere Bevorzugungen angegeben, beispielsweise bezüglich der Zahl der aromatischen Ringatome oder der enthaltenen Heteroatome, so gelten diese.

Dabei wird unter einer Arylgruppe bzw. Heteroarylgruppe entweder ein einfacher aromatischer Cyclus, also Benzol, bzw. ein einfacher heteroaromatischer Cyclus, beispielsweise Pyridin, Pyrimidin oder Thiophen, oder ein kondensierter (annellierter) aromatischer bzw. heteroaromatischer Polycyclus, beispielsweise Naphthalin, Phenanthren, Chinolin oder Carbazol verstanden. Ein kondensierter (annellierter) aromatischer bzw. heteroaromatischer Polycyclus besteht im Sinne der vorliegenden Anmeldung aus zwei oder mehr miteinander kondensierten einfachen aromatischen bzw. heteroaromatischen Cyclen.

Unter einer Aryl- oder Heteroarylgruppe, die jeweils mit den oben genannten Resten substituiert sein kann und die über beliebige Positionen am Aromaten bzw. Heteroaromaten verknüpft sein kann, werden insbesondere Gruppen verstanden, welche abgeleitet sind von Benzol, Naphthalin, Anthracen, Phenanthren, Pyren, Dihydropyren, Chrysen, Perylen, Fluoranthen, Benzanthracen, Benzphenanthren, Tetracen, Pentacen, Benzpyren, Furan, Benzofuran, Isobenzofuran, Dibenzofuran, Thiophen, Benzothiophen, Isobenzothiophen, Dibenzothiophen, Pyrrol, Indol, Isoindol, Carbazol, Pyridin, Chinolin, Isochinolin, Acridin, Phenanthridin, Benzo-5,6-chinolin, Benzo-6,7-chinolin, Benzo-7,8-chinolin, Phenothiazin, Phenoxazin, Pyrazol, Indazol, Imidazol, Benzimidazol, Naphthimidazol, Phenanthrimidazol, Pyridimidazol, Pyrazinimidazol, Chinoxalinimidazol, Oxazol, Benzoxazol, Naphthoxazol, Anthroxazol, Phenanthroxazol, Isoxazol, 1,2-Thiazol, 1,3-Thiazol, Benzothiazol, Pyridazin, Benzopyridazin, Pyrimidin, Benzpyrimidin, Chinoxalin, Pyrazin, Phenazin, Naphthyridin, Azacarbazol, Benzocarbolin, Phenanthrolin, 1,2,3-Triazol, 1,2,4-Triazol, Benzotriazol, 1,2,3-Oxadiazol, 1,2,4-Oxadiazol, 1,2,5-Oxadiazol, 1,3,4-Oxadiazol, 1,2,3-Thiadiazol, 1,2,4-Thiadiazol, 1,2,5-Thiadiazol, 1,3,4-Thiadiazol, 1,3,5-Triazin, 1,2,4-Triazin, 1,2,3-Triazin, Tetrazol, 1,2,4,5-Tetrazin, 1,2,3,4-Tetrazin, 1,2,3,5-Tetrazin, Purin, Pteridin, Indolizin und Benzothiadiazol.

Ein aromatisches Ringsystem im Sinne dieser Erfindung enthält 6 bis 60 C-Atome im Ringsystem. Ein heteroaromatisches Ringsystem im Sinne dieser Erfindung enthält 5 bis 60 aromatische Ringatome, von denen mindestens eines ein Heteroatom darstellt. Die Heteroatome sind bevorzugt ausgewählt aus N, O und/oder S. Unter einem aromatischen oder heteroaromatischen Ringsystem im Sinne dieser Erfindung soll ein System verstanden werden, das nicht notwendigerweise nur Aryl- oder Heteroarylgruppen enthält, sondern in dem auch mehrere Aryl- oder Heteroarylgruppen durch eine nicht-aromatische Einheit (bevorzugt weniger als 10 % der von H verschiedenen Atome), wie z. B. ein sp³-hybridisiertes C-, Si-, N- oder O-Atom, ein sp²-hybridisiertes C- oder N-Atom oder ein sp-hybridisiertes C-Atom, verbunden sein können. So sollen beispielsweise auch Systeme wie 9,9'-Spirobifluoren, 9,9'-Diarylfluoren, Triarylamin, Diarylether, Stilben, etc. als aromatische Ringsysteme im Sinne dieser Erfindung verstanden werden, und ebenso Systeme, in denen zwei oder mehrere Arylgruppen beispielsweise durch eine lineare oder cyclische Alkyl-, Alkenyl- oder Alkinylgruppe oder durch eine Silylgruppe verbunden sind. Weiterhin werden auch Systeme, in denen zwei oder mehr Aryl- oder Heteroarylgruppen über Einfachbindungen miteinander verknüpft sind, als aromatische oder heteroaromatische Ringsysteme im Sinne dieser Erfindung verstanden, wie beispielsweise Systeme wie Biphenyl, Terphenyl oder Diphenyltriazin.

Unter einem aromatischen oder heteroaromatischen Ringsystem mit 5 - 60 aromatischen Ringatomen, welches noch jeweils mit Resten wie oben definiert substituiert sein kann und welches über beliebige Positionen am Aromaten bzw. Heteroaromaten verknüpft sein kann, werden insbesondere Gruppen verstanden, die abgeleitet sind von Benzol, Naphthalin, Anthracen, Benzanthracen, Phenanthren, Benzphenanthren, Pyren, Chrysen, Perylen, Fluoranthen, Naphthacen, Pentacen, Benzpyren, Biphenyl, Biphenylen, Terphenyl, Terphenylen, Quaterphenyl, Fluoren, Spirobifluoren, Dihydrophenanthren, Dihydropyren, Tetrahydropyren, cis-oder trans-Indenofluoren, Truxen, Isotruxen, Spirotruxen, Spiroisotruxen, Furan, Benzofuran, Isobenzofuran, Dibenzofuran, Thiophen, Benzothiophen, Isobenzothiophen, Dibenzothiophen, Pyrrol, Indol, Isoindol, Carbazol, Indolocarbazol, Indenocarbazol, Pyridin, Chinolin, Isochinolin, Acridin, Phenanthridin, Benzo-5,6-chinolin, Benzo-6,7-chinolin, Benzo-7,8-chinolin, Phenothiazin, Phenoxazin, Pyrazol, Indazol, Imidazol, Benzimidazol, Naphthimidazol, Phenanthrimidazol, Pyridimidazol, Pyrazinimidazol, Chinoxalinimidazol, Oxazol, Benzoxazol, Naphthoxazol, Anthroxazol, Phenanthroxazol, Isoxazol, 1,2-Thiazol, 1,3-Thiazol, Benzothiazol, Pyridazin, Benzopyridazin, Pyrimidin, Benzpyrimidin, Chinoxalin, 1,5-Diazaanthracen, 2,7-Diazapyren, 2,3-Diazapyren, 1,6-Diazapyren, 1,8-Diazapyren, 4,5-Diazapyren, 4,5,9,10-Tetraazaperylen, Pyrazin, Phenazin, Phenoxazin, Phenothiazin, Fluorubin, Naphthyridin, Azacarbazol, Benzocarbolin, Phenanthrolin, 1,2,3-Triazol, 1,2,4-Triazol, Benzotriazol, 1,2,3-Oxadiazol, 1,2,4-Oxadiazol, 1,2,5-Oxadiazol, 1,3,4-Oxadiazol, 1,2,3-Thiadiazol, 1,2,4-Thiadiazol, 1,2,5-Thiadiazol, 1,3,4-Thiadiazol, 1,3,5-Triazin, 1,2,4-Triazin, 1,2,3-Triazin, Tetrazol, 1,2,4,5-Tetrazin, 1,2,3,4-Tetrazin, 1,2,3,5-Tetrazin, Purin, Pteridin, Indolizin und Benzothiadiazol oder Kombinationen dieser Gruppen.

Im Rahmen der vorliegenden Erfindung werden unter einer geradkettigen Alkylgruppe mit 1 bis 40 C-Atomen bzw. einer verzweigten oder cyclischen Alkylgruppe mit 3 bis 40 C-Atomen bzw. einer Alkenyl- oder Alkinylgruppe mit 2 bis 40 C-Atomen, in der auch einzelne H-Atome oder CH₂-Gruppen durch die oben bei der Definition der Reste genannten Gruppen substituiert sein können, bevorzugt die Reste Methyl, Ethyl, n-Propyl, i-Propyl, n-Butyl, i-Butyl, s-Butyl, t-Butyl, 2-Methylbutyl, n-Pentyl, s-Pentyl, Cyclopentyl, neoPentyl, n-Hexyl, Cyclohexyl, neo-Hexyl, n-Heptyl, Cycloheptyl, n-Octyl, Cyclooctyl, 2-Ethylhexyl, Trifluormethyl, Pentafluorethyl, 2,2,2-Trifluorethyl, Ethenyl, Propenyl, Butenyl, Pentenyl, Cyclopentenyl, Hexenyl, Cyclohexenyl, Heptenyl, Cycloheptenyl, Octenyl, Cyclooctenyl, Ethinyl, Propinyl, Butinyl, Pentinyl, Hexinyl oder Octinyl verstanden. Unter einer Alkoxy- oder Thioalkylgruppe mit 1 bis 40 C-Atomen werden bevorzugt Methoxy, Trifluormethoxy, Ethoxy, n-Propoxy, i-Propoxy, n-Butoxy, i-Butoxy, s-Butoxy, t-Butoxy, n-Pentoxy, s-Pentoxy, 2-Methylbutoxy, n-Hexoxy, Cyclohexyloxy, n-Heptoxy, Cycloheptyloxy, n-Octyloxy, Cyclooctyloxy, 2-Ethylhexyloxy, Pentafluorethoxy, 2,2,2-Trifluorethoxy, Methylthio, Ethylthio, n-Propylthio, i-Propylthio, n-Butylthio, i-Butylthio, s-Butylthio, t-Butylthio, n-Pentylthio, s-Pentylthio, n-Hexylthio, Cyclohexylthio, n-Heptylthio, Cycloheptylthio, n-Octylthio, Cyclooctylthio, 2-Ethylhexylthio, Trifluormethylthio, Pentafluorethylthio, 2,2,2-Trifluorethylthio, Ethenylthio, Propenylthio, Butenylthio, Pentenylthio, Cyclopentenylthio, Hexenylthio, Cyclohexenylthio, Heptenylthio, Cycloheptenylthio, Octenylthio, Cyclooctenylthio, Ethinylthio, Propinylthio, Butinylthio, Pentinylthio, Hexinylthio, Heptinylthio oder Octinylthio verstanden.

Unter der Formulierung, dass zwei oder mehr Reste miteinander einen Ring bilden können, soll im Rahmen der vorliegenden Anmeldung unter anderem verstanden werden, dass die beiden Reste miteinander durch eine chemische Bindung verknüpft sind. Dies wird durch das folgende Schema verdeutlicht:

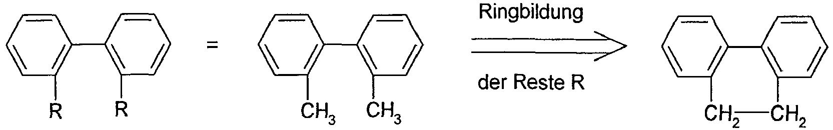

Weiterhin soll unter der oben genannten Formulierung aber auch verstanden werden, dass für den Fall, dass einer der beiden Reste Wasserstoff darstellt, der zweite Rest unter Bildung eines Rings an die Position, an die das Wasserstoffatom gebunden war, bindet. Dies soll durch das folgende Schema verdeutlicht werden:

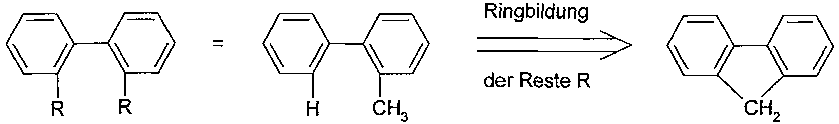

Für die erfindungsgemäßen Verbindungen sind die im Folgenden beschriebenen Ausführungsformen bevorzugt. Sie können unabhängig voneinander auftreten, bevorzugt treten sie jedoch kombiniert auf.

Bevorzugt umfasst die erfindungsgemäße Verbindungen keine Arylaminogruppe außer der in Formel (I) explizit abgebildeten Triarylaminogruppe.

Weiterhin bevorzugt umfasst die erfindungsgemäße Verbindung keine Carbazol-, Indol-, Pyrrol- oder Fluorenylgruppe.

Unter Carbazolderivaten werden im Sinne der vorliegenden Erfindung auch Carbazolderivate mit ankondensierten Gruppen, wie beispielsweise Indenocarbazole oder Indolocarbazole, sowie Carbazolderivate, in denen ein oder mehrere Kohlenstoffatome in den aromatischen Sechsringen durch Stickstoff ersetzt sind, verstanden. Analoges gilt für die oben genannten Pyrrol-, Indol- und Fluorenylgruppen.

Besonders bevorzugt umfasst die erfindungsgemäße Verbindung keine Heteroarylgruppe und kein heteroaromatisches Ringsystem.

Die Gruppe W ist bevorzugt gleich CH.

Die Gruppe Z ist bevorzugt gleich CR¹, wobei sie gleich C ist, wenn eine Gruppe Ar¹ oder Ar² an sie gebunden ist.

Bevorzugt sind nicht mehr als zwei benachbarte Gruppen Z gleich N. Weiterhin bevorzugt sind 0, 1, 2 oder 3 Gruppen Z in einem Ring gleich N, besonders bevorzugt 0, 1, oder 2, ganz besonders bevorzugt 0 oder 1.

Bevorzugt ist weiterhin Ar¹ bei jedem Auftreten gleich oder verschieden ein aromatisches Ringsystem mit 6 bis 18 aromatischen Ringatomen, bevorzugt mit 6 bis 12 aromatischen Ringatomen, wobei Ar¹ mit einem oder mehreren Resten R¹ substituiert sein kann. Besonders bevorzugt ist ein aromatisches Ringsystem mit der oben genannten Zahl an aromatischen Ringatomen. Noch stärker bevorzugt ist mit einem oder mehreren Resten R¹ substituiertes Phenyl oder Biphenyl.

Besonders bevorzugt ist Ar¹ eine Gruppe der Formel (Ar¹-1) 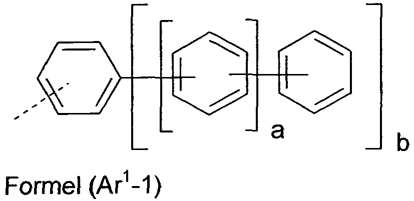
wobei an den freien Positionen jeweils eine Gruppe R¹ gebunden sein kann,und
wobei a und b jeweils gleich oder verschieden 0 oder 1 sein können, und
wobei die Gruppe über die gestrichelte Bindung an den Grundkörper der Formel (I) gebunden ist,
oder eine Gruppe der Formel (Ar¹-2) 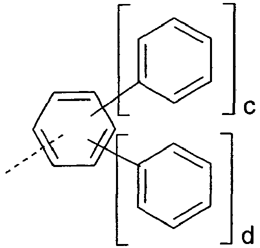 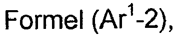
wobei an den freien Positionen jeweils eine Gruppe R¹ gebunden sein kann, und
wobei c und d jeweils gleich oder verschieden 0 oder 1 sein können, und
wobei die Gruppe über die gestrichelte Bindung an den Grundkörper der Formel (I) gebunden ist.

Ganz besonders bevorzugte Ausführungsformen der Gruppe Ar¹ entsprechen den folgenden Formeln (Ar¹-a) bis (Ar¹-d) 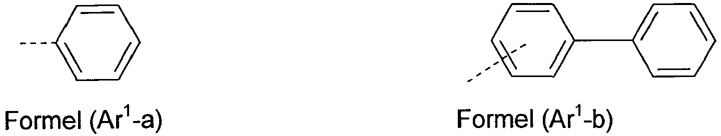 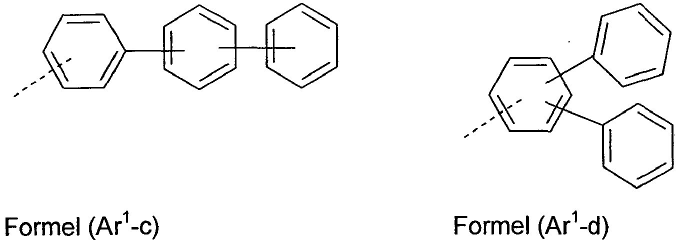 wobei an den freien Positionen jeweils ein Rest R¹ gebunden sein kann und die Gruppe über die gestrichelte Bindung an den Grundkörper der Formel (I) gebunden ist.

Bevorzugt ist weiterhin mindestens eine der Gruppen Ar¹ in der Verbindung gemäß Formel (I) an den Sechsring in meta-Position zur Bindung an das zentrale Stickstoffatom gebunden.

Bevorzugt ist weiterhin keine Gruppe Ar¹ am Sechsring in ortho-Position zur Bindung an das zentrale Stickstoffatom gebunden.

Bevorzugt ist weiterhin Ar² bei jedem Auftreten gleich oder verschieden ein aromatisches Ringsystem mit 6 bis 12 aromatischen Ringatomen, bevorzugt mit 6 aromatischen Ringatomen, wobei Ar² mit einem oder mehreren Resten R¹ substituiert sein kann. Besonders bevorzugt ist ein aromatisches Ringsystem mit der oben genannten Zahl an aromatischen Ringatomen. Noch stärker bevorzugt ist mit einem oder mehreren Resten R¹ substituiertes Phenyl.

Besonders bevorzugt ist Ar² eine Gruppe der Formel (A-3) 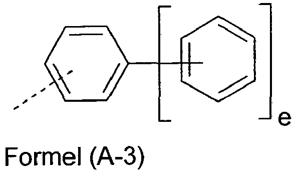
wobei an den freien Positionen jeweils eine Gruppe R¹ gebunden sein kann, und
wobei e jeweils gleich oder verschieden 0 oder 1 sein kann, und
wobei die Gruppe über die gestrichelte Bindung an den Grundkörper der Formel (I) gebunden ist.

Ganz besonders bevorzugte Ausführungsformen der Gruppe Ar² entsprechen den folgenden Formeln (Ar²-a) bis (Ar²-b) 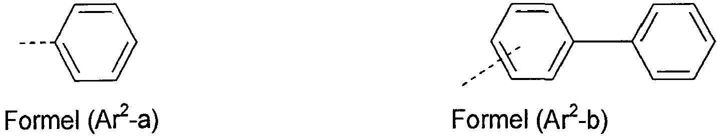 wobei an den freien Positionen jeweils ein Rest R¹ gebunden sein kann und die Gruppe über die gestrichelte Bindung an den Grundkörper der Formel (I) gebunden ist.

Bevorzugt ist weiterhin R¹ bei jedem Auftreten gleich oder verschieden H, D, F, CN, Si(R²)₃, eine geradkettige Alkyl- oder Alkoxygruppe mit 1 bis 10 C-Atomen oder eine verzweigte oder cyclische Alkyl- oder Alkoxygruppe mit 3 bis 10 C-Atomen, wobei die oben genannten Gruppen jeweils mit einem oder mehreren Resten R² substituiert sein können und wobei in den oben genannten Gruppen eine oder mehrere CH₂-Gruppen durch -C=C-, -R²C=CR²-, Si(R²)₂, C=O, C=NR², -NR²-, -O-, -S-, -C(=O)O- oder -C(=O)NR²- ersetzt sein können, oder ein aromatisches Ringsystem mit 6 bis 20 aromatischen Ringatomen, das jeweils mit einem oder mehreren Resten R² substituiert sein kann, wobei zwei oder mehr Reste R¹ jeweils miteinander verknüpft sein können und einen Ring bilden können.

Bevorzugt sind Reste R¹, welche an unterschiedliche aromatische Ringe gebunden sind, nicht miteinander unter Ringbildung verknüpft.

Bevorzugt ist weiterhin R² bei jedem Auftreten gleich oder verschieden H, D, F, CN, Si(R³)₃, eine geradkettige Alkyl- oder Alkoxygruppe mit 1 bis 10 C-Atomen oder eine verzweigte oder cyclische Alkyl- oder Alkoxygruppe mit 3 bis 10 C-Atomen, wobei die oben genannten Gruppen jeweils mit einem oder mehreren Resten R³ substituiert sein können und wobei in den oben genannten Gruppen eine oder mehrere CH₂-Gruppen durch -C=C-, -R³C=CR³-, Si(R³)₂, C=O, C=NR³, -NR³-, -O-, -S-, -C(=O)O- oder -C(=O)NR³- ersetzt sein können, oder ein aromatisches Ringsystem mit 6 bis 20 aromatischen Ringatomen, das jeweils mit einem oder mehreren Resten R³ substituiert sein kann, wobei zwei oder mehr Reste R² miteinander verknüpft sein können und einen Ring bilden können.

Bevorzugt ist n bei jedem Auftreten gleich oder verschieden 0, 1, 2 oder 3, besonders bevorzugt gleich 1 oder 2.

Weiterhin ist es bevorzugt, dass die Summe der Indices n mindestens ist. Ganz besonders bevorzugt ist sie mindestens 3.

Bevorzugt ist m gleich 0 oder 1, besonders bevorzugt gleich 0.

Bevorzugte Ausführungsformen der erfindungsgemäßen Verbindung entsprechen einer der Formeln (I-1) bis (I-12) 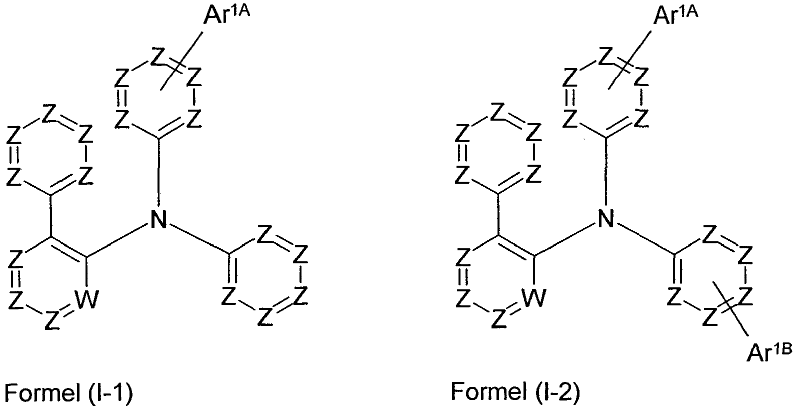 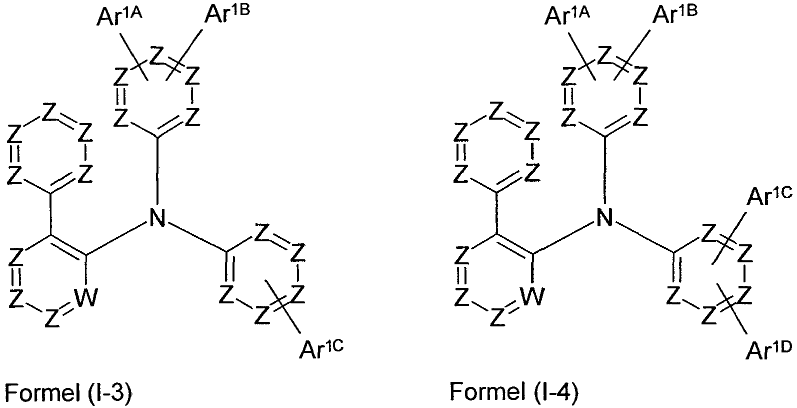 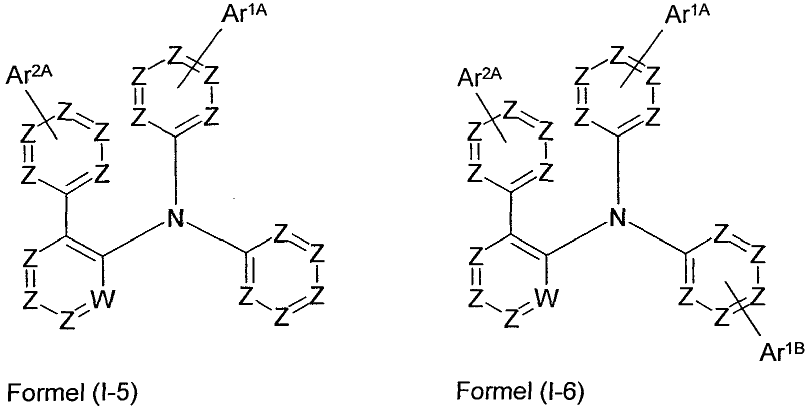 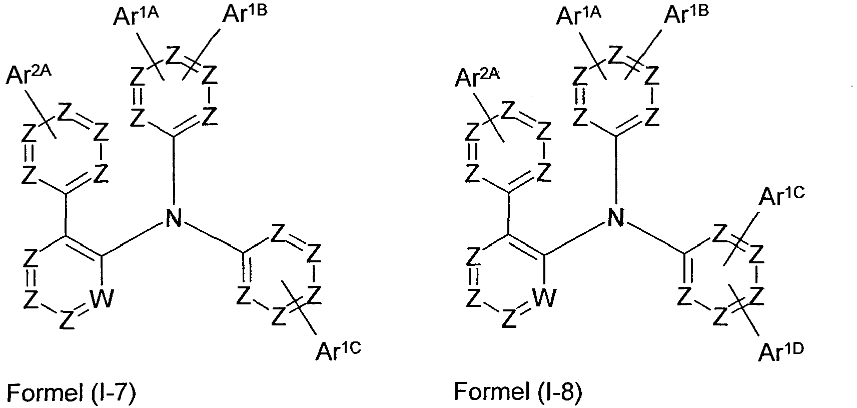 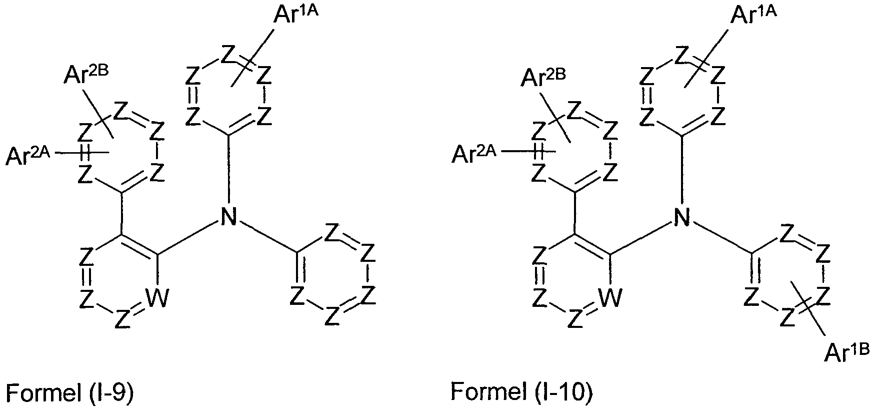 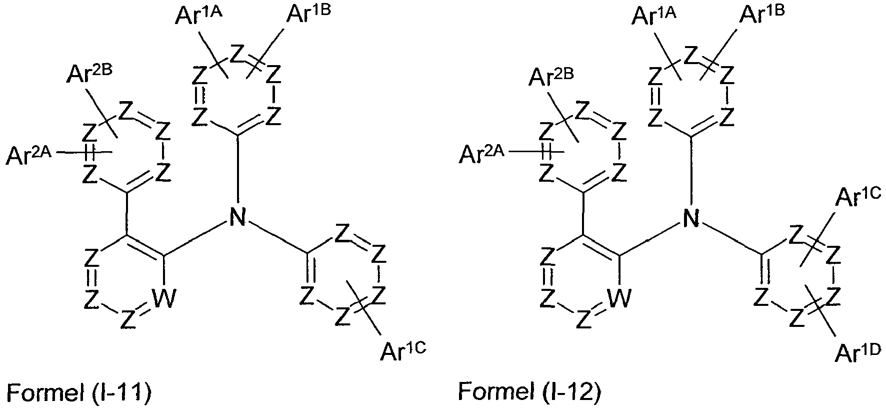 wobei die auftretenden Symbole wie oben definiert sind und Ar^{1A}, Ar^{1B}, Ar^{1C} und Ar^{1D} definiert ist wie Ar¹ und Ar^{2A} und Ar²⁶ definiert sind wie Ar².

Ar^{1A}, Ar^{1B}, Ar^{1C} und Ar^{1D} können gleich oder verschieden sein. Ar^{2A} und Ar²⁶ können gleich oder verschieden sein.

Bevorzugt ist Z in den Formeln (I-1) bis (I-12) gleich CR¹ bzw. gleich C, wenn eine Gruppe Ar¹ oder Ar² gebunden ist.

Weiterhin ist W in den Formeln (I-1) bis (I-12) bevorzugt gleich CH.

Weiterhin gelten für die Gruppen Ar¹, Ar² und R¹ bevorzugt die oben angegebenen bevorzugten Ausführungsformen.
Bevorzugt sind weiterhin Verbindungen gemäß den folgenden, von Formel (I-1), (I-2), (I-5) und (I-6) abgeleiteten Formeln: 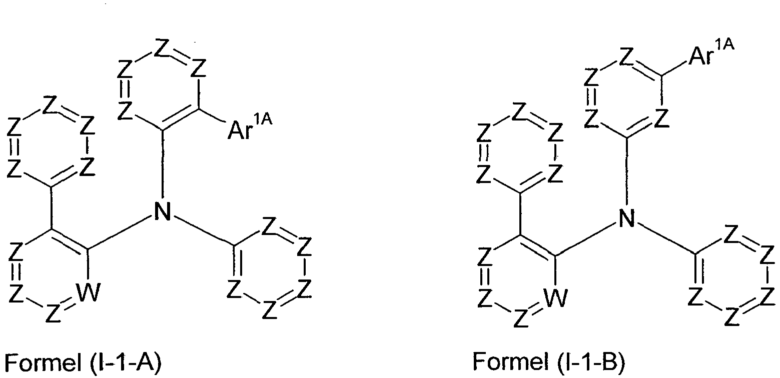 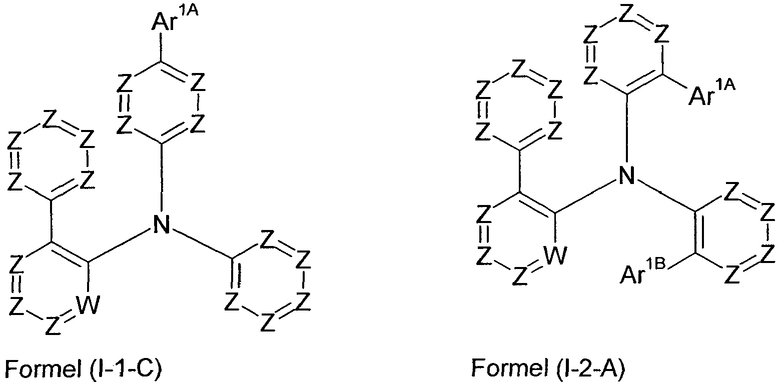 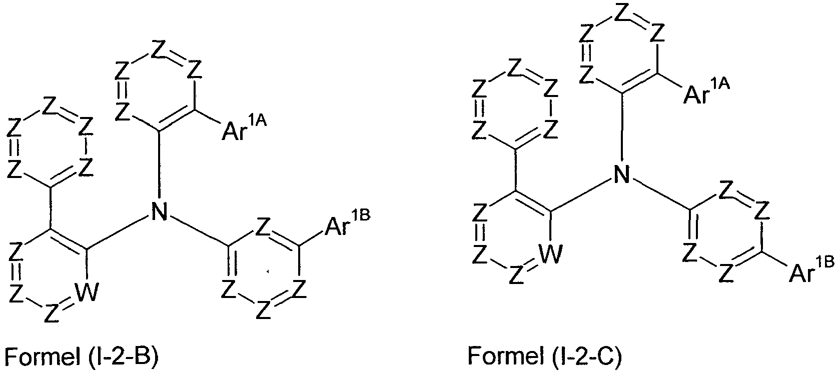 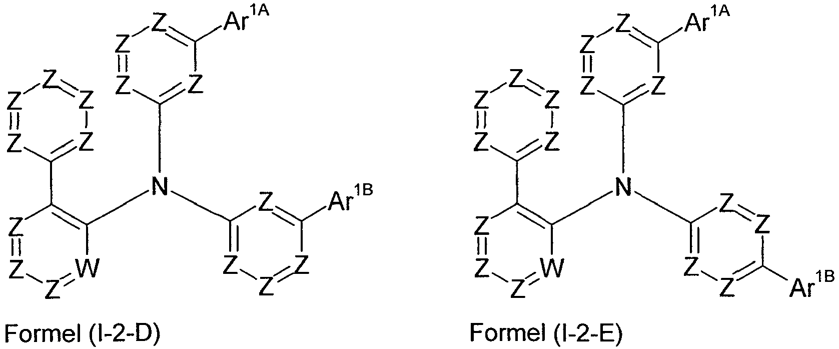 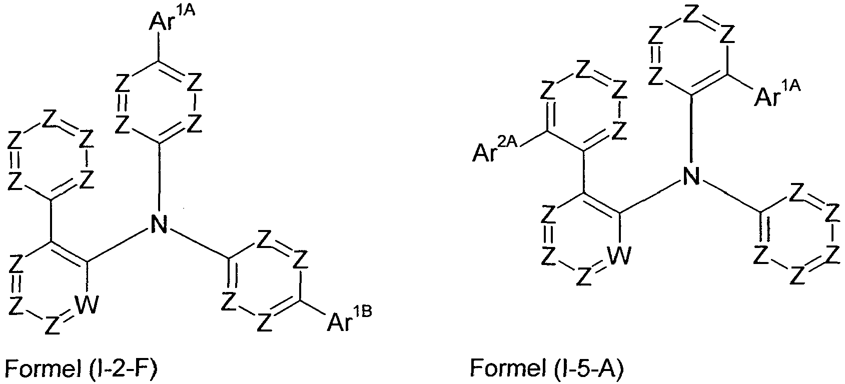 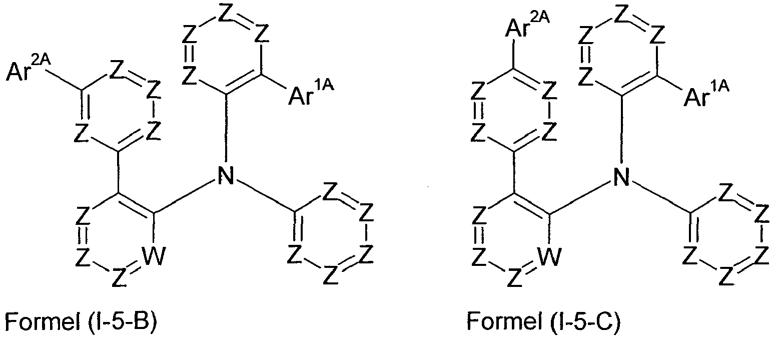 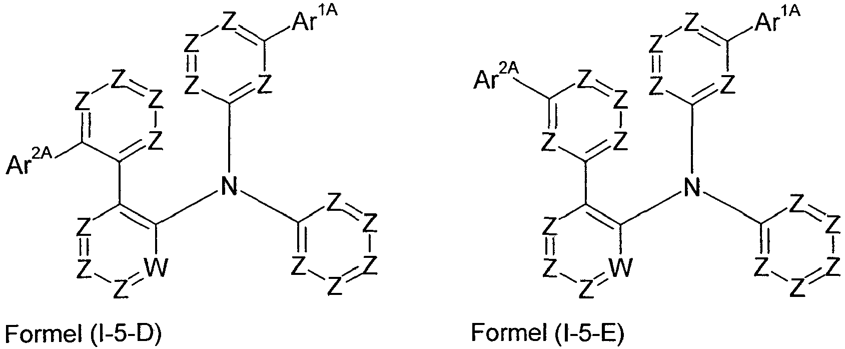 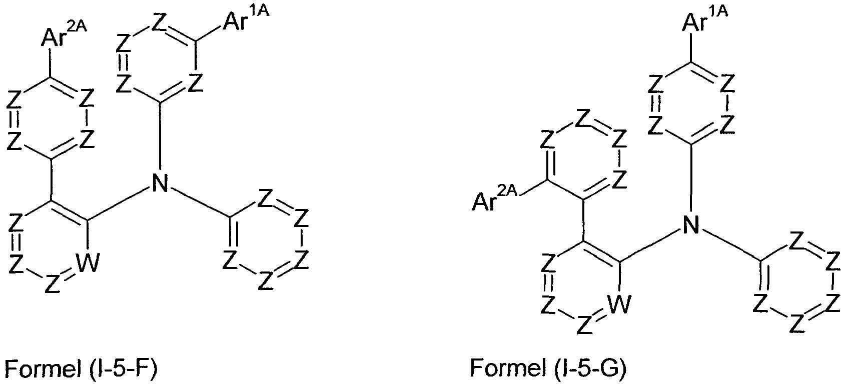 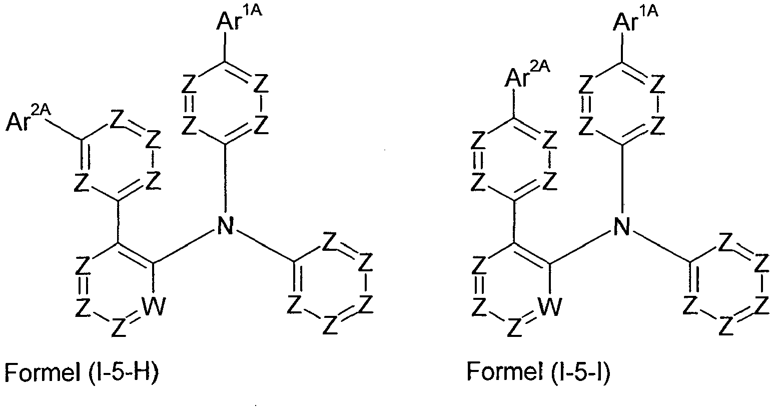 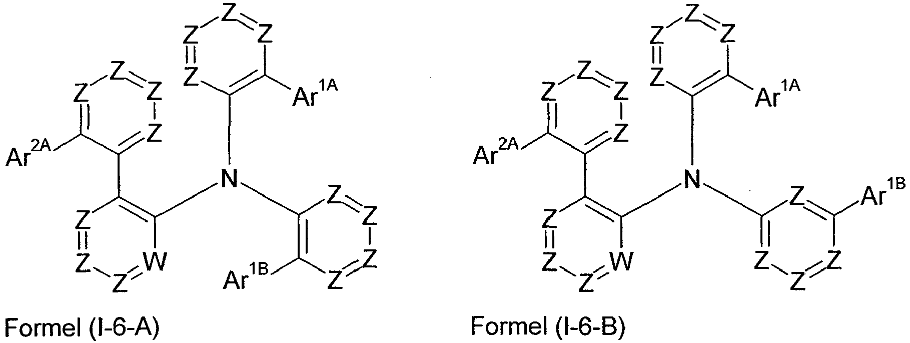 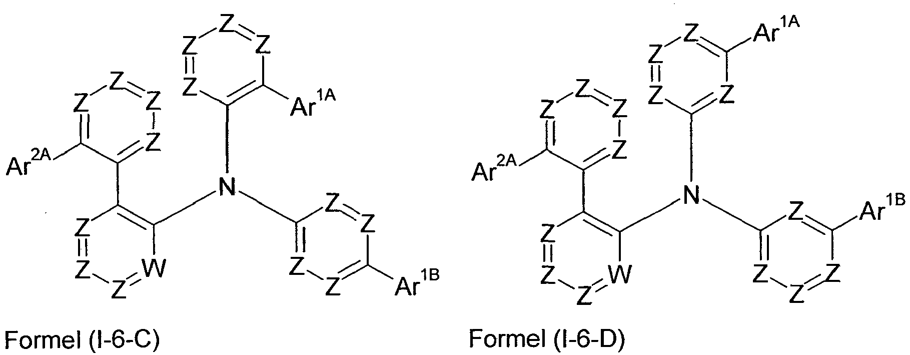 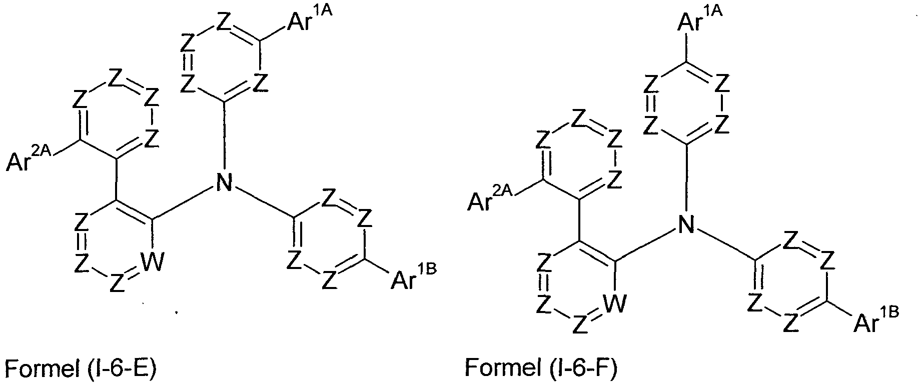 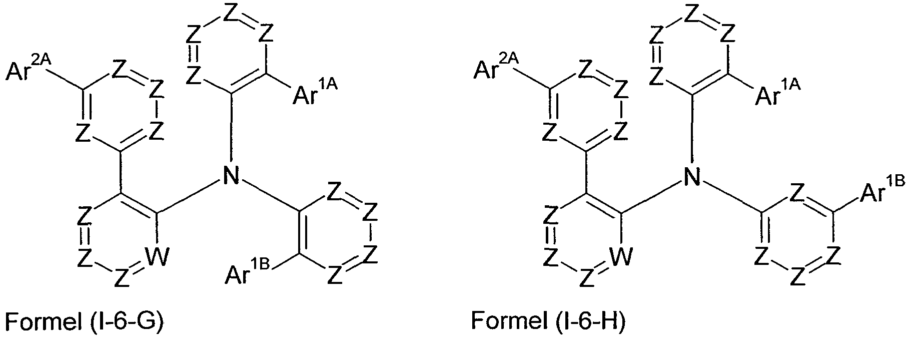 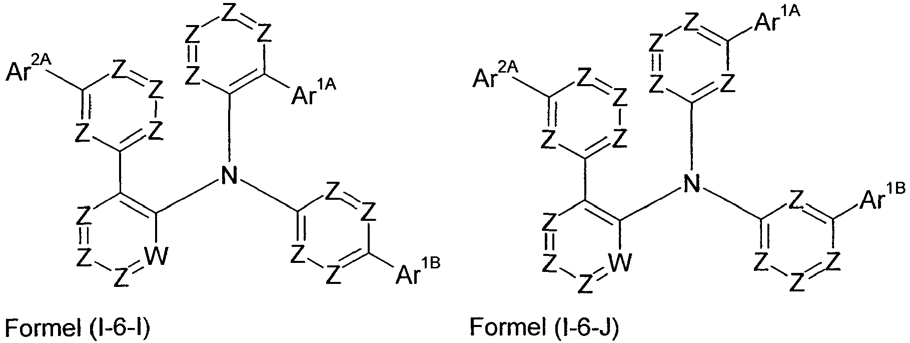 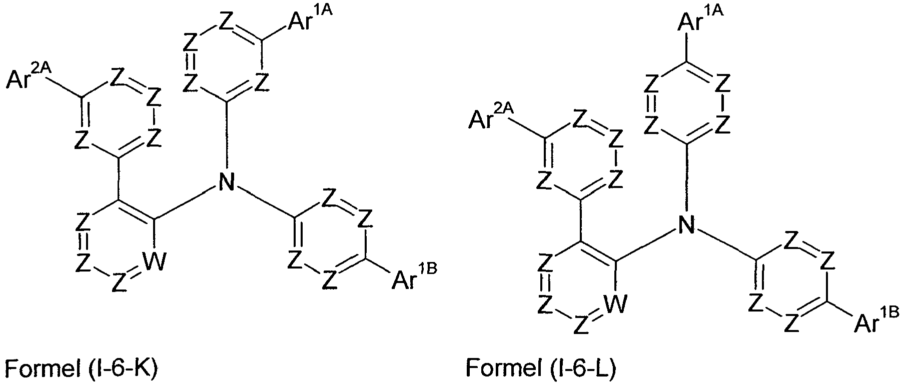 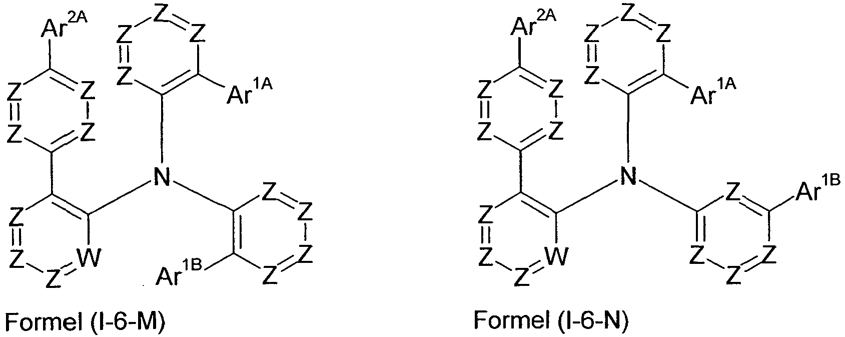 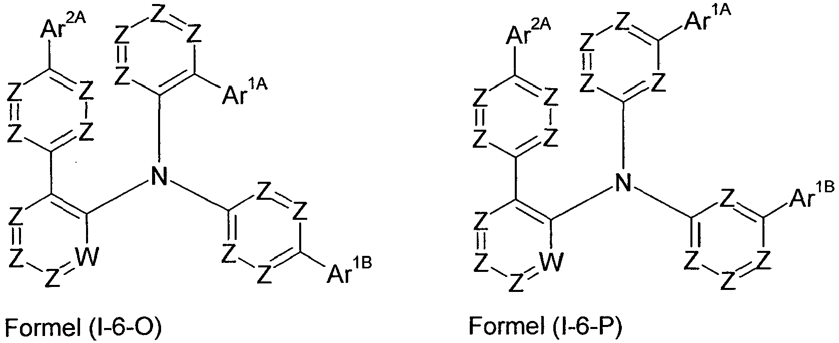 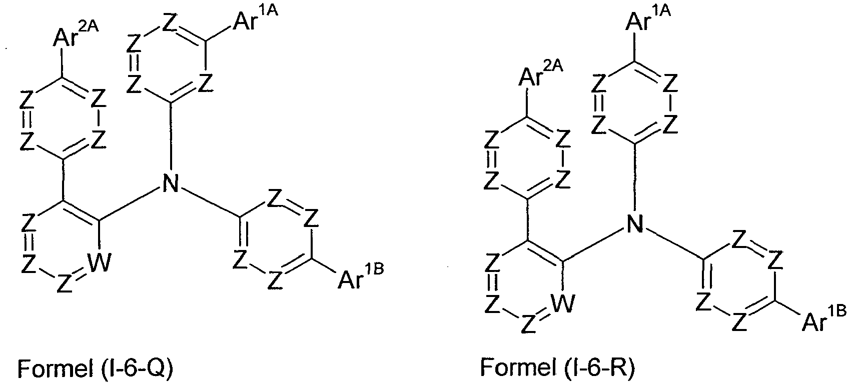 wobei die auftretenden Symbole wie oben definiert sind und Ar^{1A} und Ar^{1B} definiert sind wie Ar¹, und Ar^{2A} definiert ist wie Ar².

Ar^{1A} und Ar^{1B} können gleich oder verschieden sein.

Bevorzugt sind Ar^{1A} und Ar^{1B} Gruppen der Formeln (Ar¹-a) bis (Ar¹-d). Bevorzugt ist Ar^{2A} eine Gruppe der Formel (Ar²-a) oder (Ar²-b).

Bevorzugt ist Z in obenstehenden Formeln gleich CR¹ bzw. gleich C, wenn eine Gruppe Ar¹ oder Ar² gebunden ist.

Weiterhin ist W in den obenstehenden Formeln bevorzugt gleich CH.

Weiterhin gelten für die Gruppen Ar¹, Ar² und R¹ bevorzugt die oben angegebenen bevorzugten Ausführungsformen.

Ganz besonders bevorzugt sind die in der folgenden Tabelle wiedergegebenen Strukturen, wobei Z gleich CR¹ bzw. C ist und W gleich CH ist.

| | Grundstruktur | Ar^{1A} | Ar^{1B} | Ar^{1C} | Ar^{1D} | Ar^{2A} | Ar^{2B} |
|---|---|---|---|---|---|---|---|
| (I-1-1) | (I-1) | (Ar¹-a) | - | - | - | - | - |
| (I-1-2) | (I-1) | (Ar¹-b) | - | - | - | - | - |
| (I-1-3) | (I-1) | (Ar¹-c) | - | - | - | - | - |
| (I-1-4) | (I-1) | (Ar¹-d) | - | - | - | - | - |
| (I-2-1) | (I-2) | (Ar¹-a) | (Ar¹-a) | - | - | - | - |
| (I-2-2) | (I-2) | (Ar¹-a) | (Ar¹-b) | - | - | - | - |
| (I-2-3) | (I-2) | (Ar¹-a) | (Ar¹-c) | - | - | - | - |
| (I-2-4) | (I-2) | (Ar¹-a) | (Ar¹-d) | - | - | - | - |
| (I-2-5) | (I-2) | (Ar¹-b) | (Ar¹-b) | - | - | - | - |
| (I-2-6) | (I-2) | (Ar¹-b) | (Ar¹-c) | - | - | - | - |
| (I-2-7) | (I-2) | (Ar¹-b) | (Ar¹-d) | - | - | - | - |
| (I-2-8) | (I-2) | (Ar¹-c) | (Ar¹-c) | - | - | - | - |
| (I-2-9) | (I-2) | (Ar¹-c) | (Ar¹-d) | - | - | - | - |
| (I-2-10) | (I-2) | (Ar¹-d) | (Ar¹-d) | - | - | - | - |
| (I-3-1) | (I-3) | (Ar¹-a) | (Ar¹-a) | (Ar¹-a) | - | - | - |
| (I-3-2) | (I-3) | (Ar¹-a) | (Ar¹-b) | (Ar¹-a) | - | - | - |
| (I-3-3) | (I-3) | (Ar¹-a) | (Ar¹-c) | (Ar¹-a) | - | - | - |
| (I-3-4) | (I-3) | (Ar¹-a) | (Ar¹-d) | (Ar¹-a) | - | - | - |
| (I-3-5) | (I-3) | (Ar¹-b) | (Ar¹-b) | (Ar¹-a) | - | - | - |
| (I-3-6) | (I-3) | (Ar¹-b) | (Ar¹-c) | (Ar¹-a) | - | - | - |
| (I-3-7) | (I-3) | (Ar¹-b) | (Ar¹-d) | (Ar¹-a) | - | - | - |
| (I-3-8) | (I-3) | (Ar¹-c) | (Ar¹-c) | (Ar¹-a) | - | - | - |
| (I-3-9) | (I-3) | (Ar¹-c) | (Ar¹-d) | (Ar¹-a) | - | - | - |
| (I-3-10) | (I-3) | (Ar¹-d) | (Ar¹-d) | (Ar¹-a) | - | - | - |
| (I-3-11) | (I-3) | (Ar¹-a) | (Ar¹-a) | (Ar¹-b) | - | - | - |
| (I-3-12) | (I-3) | (Ar¹-a) | (Ar¹-b) | (Ar¹-b) | - | - | - |
| (I-3-13) | (I-3) | (Ar¹-a) | (Ar¹-c) | (Ar¹-b) | - | - | - |
| (I-3-14) | (I-3) | (Ar¹-a) | (Ar¹-d) | (Ar¹-b) | - | - | - |
| (I-3-15) | (I-3) | (Ar¹-b) | (Ar¹-b) | (Ar¹-b) | - | - | - |
| (I-3-16) | (I-3) | (Ar¹-b) | (Ar¹-c) | (Ar¹-b) | - | - | - |
| (I-3-17) | (I-3) | (Ar¹-b) | (Ar¹-d) | (Ar¹-b) | - | - | - |
| (I-3-18) | (I-3) | (Ar¹-c) | (Ar¹-c) | (Ar¹-b) | - | - | - |
| (I-3-19) | (I-3) | (Ar¹-c) | (Ar¹-d) | (Ar¹-b) | - | - | - |
| (I-3-20) | (I-3) | (Ar¹-d) | (Ar¹-d) | (Ar¹-b) | - | - | - |
| (I-3-21) | (I-3) | (Ar¹-a) | (Ar¹-a) | (Ar¹-c) | | - | - |
| (I-3-22) | (I-3) | (Ar¹-a) | (Ar¹-b) | (Ar¹-c) | - | - | - |
| (I-3-23) | (I-3) | (Ar¹-a) | (Ar¹-c) | (Ar¹-c) | - | - | - |
| (I-3-24) | (I-3) | (Ar¹-a) | (Ar¹-d) | (Ar¹-c) | - | - | - |
| (I-3-25) | (I-3) | (Ar¹-b) | (Ar¹-b) | (Ar¹-c) | - | - | - |
| (I-3-26) | (I-3) | (Ar¹-b) | (Ar¹-c) | (Ar¹-c) | - | - | - |
| (I-3-27) | (I-3) | (Ar¹-b) | (Ar¹-d) | (Ar¹-c) | - | - | - |
| (I-3-28) | (I-3) | (Ar¹-c) | (Ar¹-c) | (Ar¹-c) | - | - | - |
| (I-3-29) | (I-3) | (Ar¹-c) | (Ar¹-d) | (Ar¹-c) | - | - | - |
| (I-3-30) | (I-3) | (Ar¹-d) | (Ar¹-d) | (Ar¹-c) | - | - | - |
| (I-3-31) | (I-3) | (Ar¹-a) | (Ar¹-a) | (Ar¹-d) | - | - | - |
| (I-3-32) | (I-3) | (Ar¹-a) | (Ar¹-b) | (Ar¹-d) | - | - | - |
| (I-3-33) | (I-3) | (Ar¹-a) | (Ar¹-c) | (Ar¹-d) | - | - | - |
| (I-3-34) | (I-3) | (Ar¹-a) | (Ar¹-d) | (Ar¹-d) | - | - | - |
| (I-3-35) | (I-3) | (Ar¹-b) | (Ar¹-b) | (Ar¹-d) | - | - | - |
| (I-3-36) | (I-3) | (Ar¹-b) | (Ar¹-c) | (Ar¹-d) | - | - | - |
| (I-3-37) | (I-3) | (Ar¹-b) | (Ar¹-d) | (Ar¹-d) | - | - | - |
| (I-3-38) | (I-3) | (Ar¹-c) | (Ar¹-c) | (Ar¹-d) | - | - | - |
| (I-3-39) | (I-3) | (Ar¹-c) | (Ar¹-d) | (Ar¹-d) | - | - | - |
| (I-3-40) | (I-3) | (Ar¹-d) | (Ar¹-d) | (Ar¹-d) | - | - | - |
| (I-5-1) | (I-5) | (Ar¹-a) | - | - | - | (Ar²-a) | - |
| (I-5-2) | (I-5) | (Ar¹-a) | - | - | - | (Ar²-b) | - |
| (I-5-3) | (I-5) | (Ar¹-b) | - | - | - | (Ar²-a) | - |
| (I-5-4) | (I-5) | (Ar¹-b) | - | - | - | (Ar²-b) | - |
| (I-5-5) | (I-5) | (Ar¹-c) | - | - | - | (Ar²-a) | - |
| (I-5-6) | (I-5) | (Ar¹-c) | - | - | - | (Ar²-b) | - |
| (I-5-7) | (I-5) | (Ar¹-d) | - | - | - | (Ar²-a) | - |
| (I-5-8) | (I-5) | (Ar¹-d) | - | - | - | (Ar²-b) | - |
| (I-6-1) | (I-6) | (Ar¹-a) | (Ar¹-a) | - | - | (Ar²-a) | - |
| (I-6-2) | (I-6) | (Ar¹-a) | (Ar¹-b) | - | - | (Ar²-a) | - |
| (I-6-3) | (I-6) | (Ar¹-a) | (Ar¹-c) | - | - | (Ar²-a) | - |
| (I-6-4) | (I-6) | (Ar¹-a) | (Ar¹-d) | - | - | (Ar²-a) | - |
| (I-6-5) | (I-6) | (Ar¹-b) | (Ar¹-b) | - | - | (Ar²-a) | - |
| (I-6-6) | (I-6) | (Ar¹-b) | (Ar¹-c) | - | - | (Ar²-a) | - |
| (I-6-7) | (I-6) | (Ar¹-b) | (Ar¹-d) | - | - | (Ar²-a) | - |
| (I-6-8) | (I-6) | (Ar¹-c) | (Ar¹-c) | - | - | (Ar²-a) | - |
| (I-6-9) | (I-6) | (Ar¹-c) | (Ar¹-d) | - | - | (Ar²-a) | - |
| (I-6-10) | (I-6) | (Ar¹-d) | (Ar¹-d) | - | - | (Ar²-a) | - |
| (I-6-11) | (I-6) | (Ar¹-a) | (Ar¹-a) | - | - | (Ar²-b) | - |
| (I-6-12) | (I-6) | (Ar¹-a) | (Ar¹-b) | - | - | (Ar²-b) | - |
| (I-6-13) | (I-6) | (Ar¹-a) | (Ar¹-c) | - | - | (Ar²-b) | - |
| (I-6-14) | (I-6) | (Ar¹-a) | (Ar¹-d) | - | - | (Ar²-b) | - |
| (I-6-15) | (I-6) | (Ar¹-b) | (Ar¹-b) | - | - | (Ar²-b) | - |
| (I-6-16) | (I-6) | (Ar¹-b) | (Ar¹-c) | - | - | (Ar²-b) | - |
| (I-6-17) | (I-6) | (Ar¹-b) | (Ar¹-d) | - | - | (Ar²-b) | - |
| (I-6-18) | (I-6) | (Ar¹-c) | (Ar¹-c) | - | - | (Ar²-b) | - |
| (I-6-19) | (I-6) | (Ar¹-c) | (Ar¹-d) | - | - | (Ar²-b) | - |
| (I-6-20) | (I-6) | (Ar¹-d) | (Ar¹-d) | - | - | (Ar²-b) | - |
| (I-7-1) | (I-7) | (Ar¹-a) | (Ar¹-a) | (Ar¹-a) | - | (Ar²-a) | - |
| (I-7-2) | (I-7) | (Ar¹-a) | (Ar¹-b) | (Ar¹-a) | - | (Ar²-a) | - |
| (I-7-3) | (I-7) | (Ar¹-a) | (Ar¹-c) | (Ar¹-a) | - | (Ar²-a) | - |
| (I-7-4) | (I-7) | (Ar¹-a) | (Ar¹-d) | (Ar¹-a) | - | (Ar²-a) | - |
| (I-7-5) | (I-7) | (Ar¹-b) | (Ar¹-b) | (Ar¹-a) | - | (Ar²-a) | - |
| (I-7-6) | (I-7) | (Ar¹-b) | (Ar¹-c) | (Ar¹-a) | - | (Ar²-a) | - |
| (I-7-7) | (I-7) | (Ar¹-b) | (Ar¹-d) | (Ar¹-a) | - | (Ar²-a) | - |
| (I-7-8) | (I-7) | (Ar¹-c) | (Ar¹-c) | (Ar¹-a) | - | (Ar²-a) | - |
| (I-7-9) | (I-7) | (Ar¹-c) | (Ar¹-d) | (Ar¹-a) | - | (Ar²-a) | - |
| (I-7-10) | (I-7) | (Ar¹-d) | (Ar¹-d) | (Ar¹-a) | - | (Ar²-a) | - |
| (I-7-11) | (I-7) | (Ar¹-a) | (Ar¹-a) | (Ar¹-b) | - | (Ar²-a) | - |
| (I-7-12) | (I-7) | (Ar¹-a) | (Ar¹-b) | (Ar¹-b) | - | (Ar²-a) | - |
| (I-7-13) | (I-7) | (Ar¹-a) | (Ar¹-c) | (Ar¹-b) | - | (Ar²-a) | - |
| (I-7-14) | (I-7) | (Ar¹-a) | (Ar¹-d) | (Ar¹-b) | - | (Ar²-a) | - |
| (I-7-15) | (I-7) | (Ar¹-b) | (Ar¹-b) | (Ar¹-b) | - | (Ar²-a) | - |
| (I-7-16) | (I-7) | (Ar¹-b) | (Ar¹-c) | (Ar¹-b) | - | (Ar²-a) | - |
| (I-7-17) | (I-7) | (Ar¹-b) | (Ar¹-d) | (Ar¹-b) | - | (Ar²-a) | - |
| (I-7-18) | (I-7) | (Ar¹-c) | (Ar¹-c) | (Ar¹-b) | - | (Ar²-a) | - |
| (I-7-19) | (I-7) | (Ar¹-c) | (Ar¹-d) | (Ar¹-b) | - | (Ar²-a) | - |
| (I-7-20) | (I-7) | (Ar¹-d) | (Ar¹-d) | (Ar¹-b) | - | (Ar²-a) | - |
| (I-7-21) | (I-7) | (Ar¹-a) | (Ar¹-a) | (Ar¹-c) | - | (Ar²-a) | - |
| (I-7-22) | (I-7) | (Ar¹-a) | (Ar¹-b) | (Ar¹-c) | - | (Ar²-a) | - |
| (I-7-23) | (I-7) | (Ar¹-a) | (Ar¹-c) | (Ar¹-c) | - | (Ar²-a) | - |
| (I-7-24) | (I-7) | (Ar¹-a) | (Ar¹-d) | (Ar¹-c) | - | (Ar²-a) | - |
| (I-7-25) | (I-7) | (Ar¹-b) | (Ar¹-b) | (Ar¹-c) | - | (Ar²-a) | - |
| (I-7-26) | (I-7) | (Ar¹-b) | (Ar¹-c) | (Ar¹-c) | - | (Ar²-a) | - |
| (I-7-27) | (I-7) | (Ar¹-b) | (Ar¹-d) | (Ar¹-c) | - | (Ar²-a) | - |
| (I-7-28) | (I-7) | (Ar¹-c) | (Ar¹-c) | (Ar¹-c) | - | (Ar²-a) | - |
| (I-7-29) | (I-7) | (Ar¹-c) | (Ar¹-d) | (Ar¹-c) | - | (Ar²-a) | - |
| (I-7-30) | (I-7) | (Ar¹-d) | (Ar¹-d) | (Ar¹-c) | - | (Ar²-a) | - |
| (I-7-31) | (I-7) | (Ar¹-a) | (Ar¹-a) | (Ar¹-d) | - | (Ar²-a) | - |
| (I-7-32) | (I-7) | (Ar¹-a) | (Ar¹-b) | (Ar¹-d) | - | (Ar²-a) | - |
| (I-7-33) | (I-7) | (Ar¹-a) | (Ar¹-c) | (Ar¹-d) | - | (Ar²-a) | - |
| (I-7-34) | (I-7) | (Ar¹-a) | (Ar¹-d) | (Ar¹-d) | - | (Ar²-a) | - |
| (I-7-35) | (I-7) | (Ar¹-b) | (Ar¹-b) | (Ar¹-d) | - | (Ar²-a) | - |
| (I-7-36) | (I-7) | (Ar¹-b) | (Ar¹-c) | (Ar¹-d) | - | (Ar²-a) | - |
| (I-7-37) | (I-7) | (Ar¹-b) | (Ar¹-d) | (Ar¹-d) | - | (Ar²-a) | - |
| (I-7-38) | (I-7) | (Ar¹-c) | (Ar¹-c) | (Ar¹-d) | - | (Ar²-a) | - |
| (I-7-39) | (I-7) | (Ar¹-c) | (Ar¹-d) | (Ar¹-d) | - | (Ar²-a) | - |
| (I-7-40) | (I-7) | (Ar¹-d) | (Ar¹-d) | (Ar¹-d) | - | (Ar²-a) | - |
| (I-7-41) | (I-7) | (Ar¹-a) | (Ar¹-a) | (Ar¹-a) | - | (Ar²-b) | - |
| (I-7-42) | (I-7) | (Ar¹-a) | (Ar¹-b) | (Ar¹-a) | - | (Ar²-b) | |
| (I-7-43 | (I-7) | (Ar¹-a) | (Ar¹-c) | (Ar¹-a) | - | (Ar²-b) | - |
| (I-7-44) | (I-7) | (Ar¹-a) | (Ar¹-d) | (Ar¹-a) | - | (Ar²-b) | - |
| (I-7-45) | (I-7) | (Ar¹-b) | (Ar¹-b) | (Ar¹-a) | - | Ar²-b) | - |
| (I-7-46) | (I-7) | (Ar¹-b) | (Ar¹-c) | (Ar¹-a) | - | (Ar²-b) | - |
| (I-7-47) | (I-7) | (Ar¹-b) | (Ar¹-d) | (Ar¹-a) | - | (Ar²-b) | - |
| (I-7-48) | (I-7) | (Ar¹-c) | (Ar¹-c) | (Ar¹-a) | - | (Ar²-b) | - |
| (I-7-49) | (I-7) | (Ar¹-c) | (Ar¹-d) | (Ar¹-a) | - | (Ar²-b) | - |
| (I-7-50) | (I-7) | (Ar¹-d) | (Ar¹-d) | (Ar¹-a) | - | (Ar²-b) | - |
| (I-7-51) | (I-7) | (Ar¹-a) | (Ar¹-a) | (Ar¹-b) | - | (Ar²-b) | - |
| (I-7-52) | (I-7) | (Ar¹-a) | (Ar¹-b) | (Ar¹-b) | - | (Ar²-b) | - |
| (I-7-53) | (I-7) | (Ar¹-a) | (Ar¹-c) | (Ar¹-b) | - | (Ar²-b) | - |
| (I-7-54) | (I-7) | (Ar¹-a) | (Ar¹-d) | (Ar¹-b) | - | (Ar²-b) | - |
| (I-7-55) | (I-7) | (Ar¹-b) | (Ar¹-b) | (Ar¹-b) | - | (Ar²-b) | - |
| (I-7-56) | (I-7) | (Ar¹-b) | (Ar¹-c) | (Ar¹-b) | - | (Ar²-b) | - |
| (I-7-57) | (I-7) | (Ar¹-b) | (Ar¹-d) | (Ar¹-b) | - | (Ar²-b) | - |
| (I-7-58) | (I-7) | (Ar¹-c) | (Ar¹-c) | (Ar¹-b) | - | (Ar²-b) | - |
| (I-7-59) | (I-7) | (Ar¹-c) | (Ar¹-d) | (Ar¹-b) | - | (Ar²-b) | - |
| (I-7-60) | (I-7) | (Ar¹-d) | (Ar¹-d) | (Ar¹-b) | - | (Ar²-b) | - |
| (I-7-61) | (I-7) | (Ar¹-a) | (Ar¹-a) | (Ar¹-c) | - | (Ar²-b) | - |
| (I-7-62) | (I-7) | (Ar¹-a) | (Ar¹-b) | (Ar¹-c) | - | (Ar²-b) | - |
| (I-7-63) | (I-7) | (Ar¹-a) | (Ar¹-c) | (Ar¹-c) | - | (Ar²-b) | - |
| (I-7-64) | (I-7) | (Ar¹-a) | (Ar¹-d) | (Ar¹-c) | - | (Ar²-b) | - |
| (I-7-65) | (I-7) | (Ar¹-b) | (Ar¹-b) | (Ar¹-c) | - | (Ar²-b) | - |
| (I-7-66) | (I-7) | (Ar¹-b) | (Ar¹-c) | (Ar¹-c) | - | (Ar²-b) | - |
| (I-7-67) | (I-7) | (Ar¹-b) | (Ar¹-d) | (Ar¹-c) | - | (Ar²-b) | - |
| (I-7-68) | (I-7) | (Ar¹-c) | (Ar¹-c) | (Ar¹-c) | - | (Ar²-b) | - |
| (I-7-69) | (I-7) | (Ar¹-c) | (Ar¹-d) | (Ar¹-c) | - | (Ar²-b) | - |
| (I-7-70) | (I-7) | (Ar¹-d) | (Ar¹-d) | (Ar¹-c) | - | (Ar²-b) | - |
| (I-7-71) | (I-7) | (Ar¹-a) | (Ar¹-a) | (Ar¹-d) | - | (Ar²-b) | - |
| (I-7-72) | (I-7) | (Ar¹-a) | (Ar¹-b) | (Ar¹-d) | - | (Ar²-b) | - |
| (I-7-73) | (I-7) | (Ar¹-a) | (Ar¹-c) | (Ar¹-d) | - | (Ar²-b) | - |
| (I-7-74) | (I-7) | (Ar¹-a) | (Ar¹-d) | (Ar¹-d) | - | (Ar²-b | - |
| (I-7-75) | (I-7) | (Ar¹-b) | (Ar¹-b) | (Ar¹-d) | - | (Ar²-b) | - |
| (I-7-76) | (I-7) | (Ar¹-b) | (Ar¹-c) | (Ar¹-d) | - | (Ar²-b) | - |
| (I-7-77) | (I-7) | (Ar¹-b) | (Ar¹-d) | (Ar¹-d) | - | (Ar²-b) | - |
| (I-7-78) | (I-7) | (Ar¹-c) | (Ar¹-c) | (Ar¹-d) | - | (Ar²-b) | - |
| (I-7-79) | (I-7) | (Ar¹-c) | (Ar¹-d) | (Ar¹-d) | - | (Ar²-b) | - |
| (I-7-80) | (I-7) | (Ar¹-d) | (Ar¹-d) | (Ar¹-d) | - | (Ar²-b) | - |

Die in der Tabelle aufgelisteten Strukturen können mit beliebigen Substituenten, wie oben definiert, substituiert sein. Bevorzugt sind sie unsubstituiert.

Beispiele für erfindungsgemäße Verbindungen sind in der untenstehenden Tabelle abgebildet. Nicht anspruchsgemäße Verbindungen sind mit # gekennzeichnet.

| | |
|---|---|
| 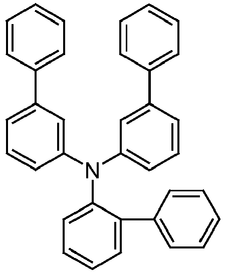 | 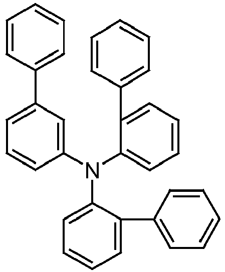 |
| 1 | 2 |
| 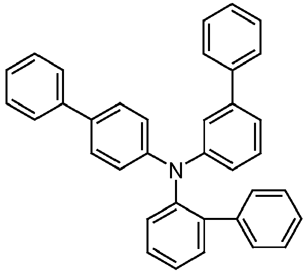 | 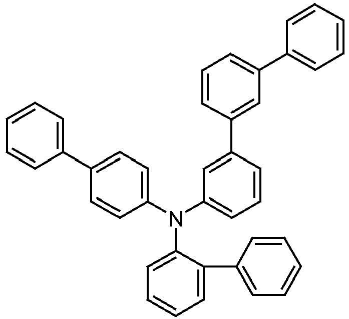 |
| 3 | 4 |
| 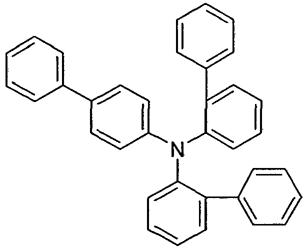 | 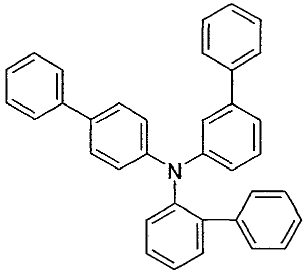 |
| 5 | 6 |
| 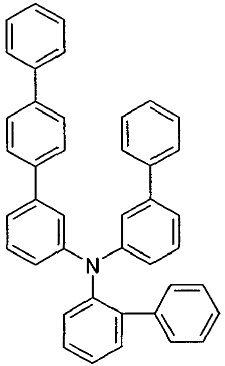 | 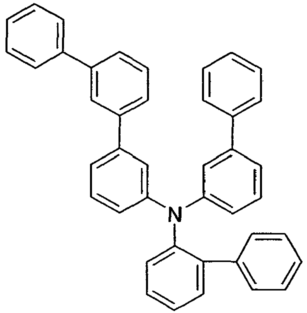 |
| 7 | 8 |
| 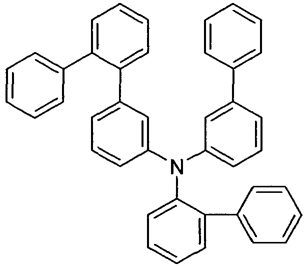 | 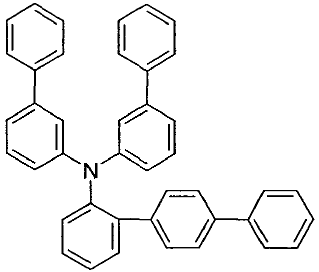 |
| 9 | 10 |
| 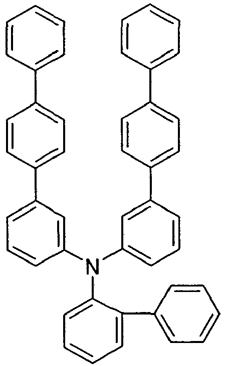 | 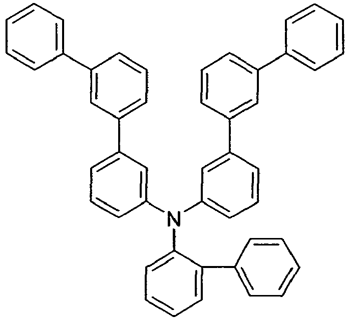 |
| 11 | 12 |
| 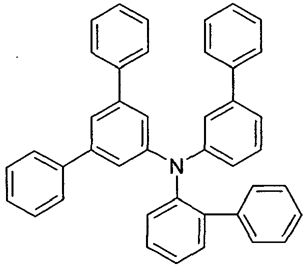 | 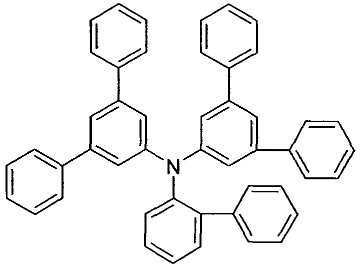 |
| 13 | 14 |
| 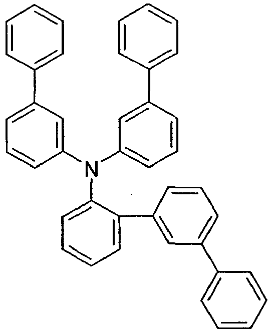 | 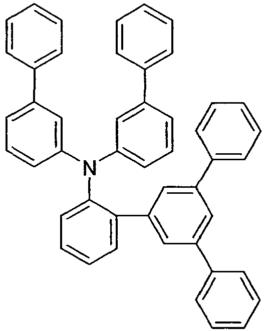 |
| 14 | 16 |
| 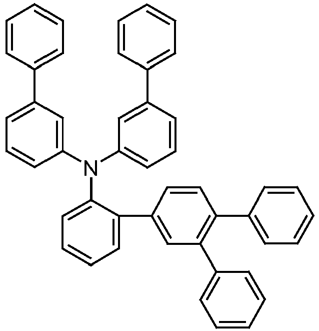 | 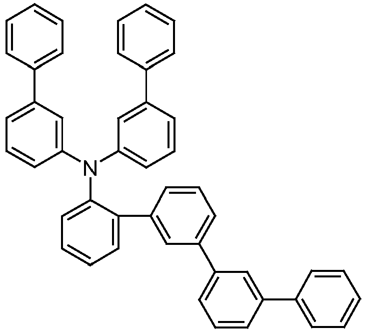 |
| 17 | 18 |
| 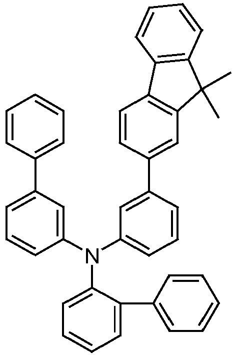 | 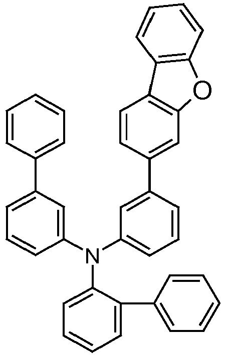 |
| 19 | 20 # |
| 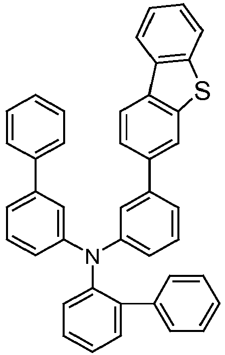 | 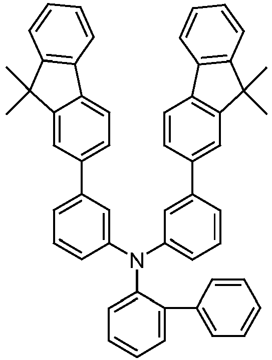 |
| 21 # | 22 |
| 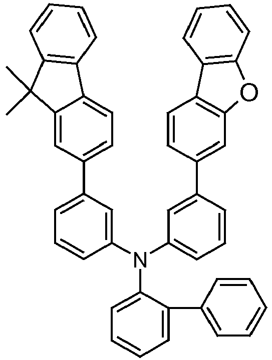 | 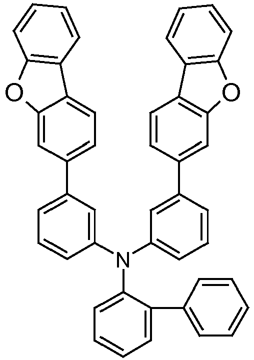 |
| 23 # | 24 # |
| 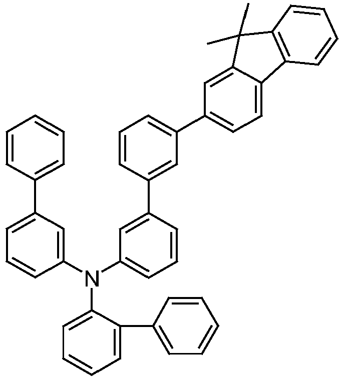 | 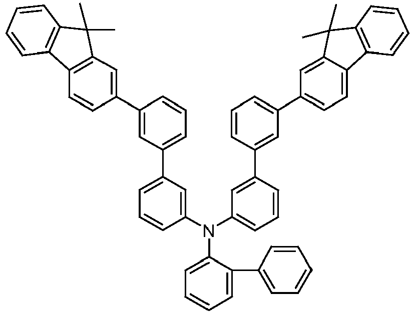 |
| 25 | 26 |
| 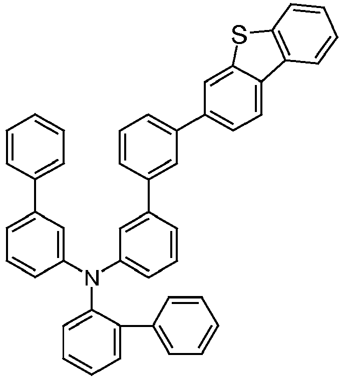 | 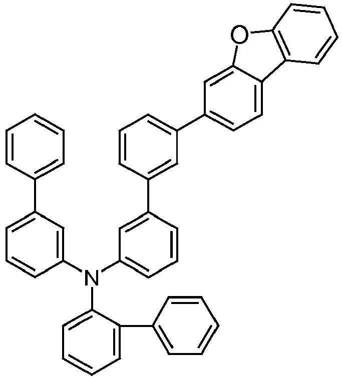 |
| 27 # | 28 # |
| 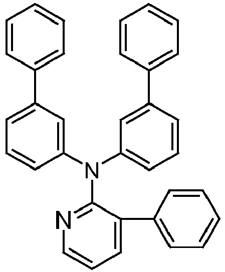 | 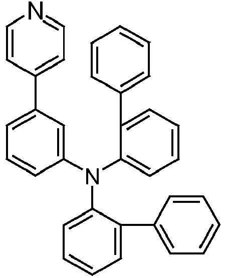 |
| 29 | 30 # |
| 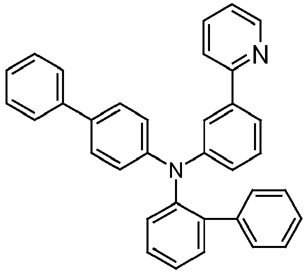 | 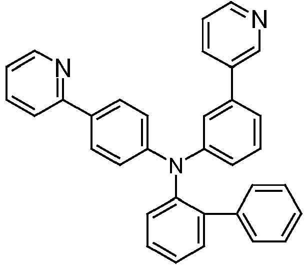 |
| 31 # | 32 # |

Die Synthese der erfindungsgemäßen Verbindungen kann gemäß dem Fachmann allgemein bekannten Verfahren der organischen präparativen Chemie erfolgen. Beispiele für bevorzugt eingesetzte Reaktionen sind Halogenierungen sowie übergangsmetallkatalysierte Kupplungsreaktionen, bevorzugt Suzuki-Kupplungen und Buchwald-Kupplungen.

Zwei bevorzugte Wege zur Herstellung der erfindungsgemäßen Verbindungen sind im Folgenden dargestellt.

Zum Einen ist es möglich, zur Herstellung der erfindungsgemäßen Verbindungen von einem primären aromatischen Amin auszugehen, welches mit zwei Arylgruppen in einer Kupplungsreaktion verknüpft wird (Schema 1).

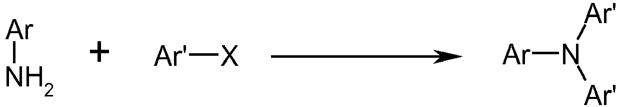

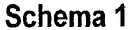

Ar steht hierbei für ein aromatisches oder heteroaromatisches Ringsystem, Ar' steht für ein aromatisches oder heteroaromatisches Ringsystem und X steht für eine beliebige Abgangsgruppe, bevorzugt ein Halogenid wie Cl, Br oder I, ein Alkyl- oder Arylsulfonat oder Diazonium.

Die Kupplungsreaktion ist dabei bevorzugt eine übergangsmetallkatalysierte Kupplungsreaktion, beispielsweise eine Hartwig-Buchwald-Kupplung. Es kann auch eine Ullmann-Reaktion verwendet werden.

Alternativ ist es möglich, zur Herstellung der erfindungsgemäßen Verbindungen von einem sekundären aromatischen Amin auszugehen, welches zwei Arylgruppen trägt und welches in einer Kupplungsreaktion mit einer Arylgruppe verknüpft wird (Schema 2).

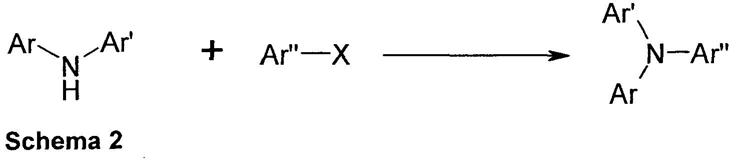

Die auftretenden Symbole sind dabei wie in Schema 1 definiert, wobei Ar" ein aromatisches oder heteroaromatisches Ringsystem darstellt. Es wird bevorzugt eine Kupplungsreaktion wie für Schema 1 angegeben verwendet.

An die gezeigten Reaktionsschritte können sich weitere Synthesestufen, beispielsweise zur Einführung von Substituenten oder zur Modifikation des Grundgerüsts, anschließen.

Der Fachmann kann im Rahmen seines allgemeinen Fachwissens, unter anderem auch in Abhängigkeit von der kommerziellen oder synthetischen Verfügbarkeit der Ausgangsverbindungen, das für den jeweils vorliegenden Fall geeignete Verfahren aus den beiden oben beschriebenen Verfahren auswählen.

Gegenstand der vorliegenden Erfindung ist somit weiterhin ein Verfahren zur Herstellung einer Verbindung gemäß Formel (I), dadurch gekennzeichnet, dass eine Arylverbindung enthaltend eine Aminogruppe und eine Arylverbindung enthaltend eine Abgangsgruppe in einer übergangsmetallkatalysierten Kupplungsreaktion miteinander verknüpft werden.

Die Abgangsgruppe ist dabei bevorzugt gewählt aus Cl, Br, I, Methylsulfonat, Trifluormethylsulfonat, Phenylsulfonat, Tolylsulfonat und Diazonium.

Die oben beschriebenen erfindungsgemäßen Verbindungen, insbesondere Verbindungen, welche mit reaktiven Abgangsgruppen, wie Brom, Iod, Chlor, Boronsäure oder Boronsäureester, substituiert sind, können als Monomere zur Erzeugung entsprechender Oligomere, Dendrimere oder Polymere Verwendung finden. Geeignete reaktive Abgangsgruppen sind beispielsweise Brom, Iod, Chlor, Boronsäuren, Boronsäureester, Amine, Alkenyl- oder Alkinylgruppen mit endständiger C-C-Doppelbindung bzw. C-C-Dreifachbindung, Oxirane, Oxetane, Gruppen, die eine Cycloaddition, beispielsweise eine 1,3-dipolare Cycloaddition, eingehen, wie beispielsweise Diene oder Azide, Carbonsäurederivate, Alkohole und Silane.

Weiterer Gegenstand der Erfindung sind daher Oligomere, Polymere oder Dendrimere enthaltend eine oder mehrere Verbindungen gemäß Formel (I), wobei die Bindung(en) zum Polymer, Oligomer oder Dendrimer an beliebigen, in Formel (I) mit R¹ substituierten Positionen lokalisiert sein können. Je nach Verknüpfung der Verbindung gemäß Formel (I) ist die Verbindung Bestandteil einer Seitenkette des Oligomers oder Polymers oder Bestandteil der Hauptkette. Unter einem Oligomer im Sinne dieser Erfindung wird eine Verbindung verstanden, welche aus mindestens drei Monomereinheiten aufgebaut ist. Unter einem Polymer im Sinne der Erfindung wird eine Verbindung verstanden, die aus mindestens zehn Monomereinheiten aufgebaut ist. Die erfindungsgemäßen Polymere, Oligomere oder Dendrimere können konjugiert, teilkonjugiert oder nichtkonjugiert sein. Die erfindungsgemäßen Oligomere oder Polymere können linear, verzweigt oder dendritisch sein. In den linear verknüpften Strukturen können die Einheiten gemäß Formel (I) direkt miteinander verknüpft sein oder sie können über eine bivalente Gruppe, beispielsweise über eine substituierte oder unsubstituierte Alkylengruppe, über ein Heteroatom oder über eine bivalente aromatische oder heteroaromatische Gruppe miteinander verknüpft sein. In verzweigten und dendritischen Strukturen können beispielsweise drei oder mehrere Einheiten gemäß Formel (I) über eine trivalente oder höhervalente Gruppe, beispielsweise über eine trivalente oder höhervalente aromatische oder heteroaromatische Gruppe, zu einem verzweigten bzw. dendritischen Oligomer oder Polymer verknüpft sein.

Für die Wiederholeinheiten gemäß Formel (I) in Oligomeren, Dendrimeren und Polymeren gelten dieselben Bevorzugungen wie oben für Verbindungen gemäß Formel (I) beschrieben.

Zur Herstellung der Oligomere oder Polymere werden die erfindungsgemäßen Monomere homopolymerisiert oder mit weiteren Monomeren copolymerisiert. Geeignete und bevorzugte Comonomere sind gewählt aus Fluorenen (z. B. gemäß EP 842208 oder WO 2000/22026), Spirobifluorenen (z. B. gemäß EP 707020, EP 894107 oder WO 2006/061181), Paraphenylenen (z. B. gemäß WO 1992/18552), Carbazolen (z. B. gemäß WO 2004/070772 oder WO 2004/113468), Thiophenen (z. B. gemäß EP 1028136), Dihydrophenanthrenen (z. B. gemäß WO 2005/014689 oder WO 2007/006383), cis- und trans-Indenofluorenen (z. B. gemäß WO 2004/041901 oder WO 2004/113412), Ketonen (z. B. gemäß WO 2005/040302), Phenanthrenen (z. B. gemäß WO 2005/104264 oder WO 2007/017066) oder auch mehreren dieser Einheiten. Die Polymere, Oligomere und Dendrimere enthalten üblicherweise noch weitere Einheiten, beispielsweise emittierende (fluoreszierende oder phosphoreszierende) Einheiten, wie z. B. Vinyltriarylamine (z. B. gemäß WO 2007/068325) oder phosphoreszierende Metallkomplexe (z. B. gemäß WO 2006/003000), und/oder Ladungstransporteinheiten, insbesondere solche basierend auf Triarylaminen.

Die erfindungsgemäßen Polymere, Oligomere und Dendrimere weisen vorteilhafte Eigenschaften, insbesondere hohe Lebensdauern, hohe Effizienzen und gute Farbkoordinaten auf.

Die erfindungsgemäßen Polymere und Oligomere werden in der Regel durch Polymerisation von einer oder mehreren Monomersorten hergestellt, von denen mindestens ein Monomer im Polymer zu Wiederholungseinheiten der Formel (I) führt. Geeignete Polymerisationsreaktionen sind dem Fachmann bekannt und in der Literatur beschrieben. Besonders geeignete und bevorzugte Polymerisationsreaktionen, die zu C-C- bzw. C-N-Verknüpfungen führen, sind folgende:
(A) SUZUKI-Polymerisation;
(B) YAMAMOTO-Polymerisation;
(C) STILLE-Polymerisation; und
(D) HARTWIG-BUCHWALD-Polymerisation.

Wie die Polymerisation nach diesen Methoden durchgeführt werden kann und wie die Polymere dann vom Reaktionsmedium abgetrennt und aufgereinigt werden können, ist dem Fachmann bekannt und in der Literatur, beispielsweise in WO 2003/048225, WO 2004/037887 und WO 2004/037887, im Detail beschrieben.

Gegenstand der vorliegenden Erfindung ist somit auch ein Verfahren zur Herstellung der erfindungsgemäßen Polymere, Oligomere und Dendrimere, das dadurch gekennzeichnet ist, dass sie durch Polymerisation gemäß SUZUKI, Polymerisation gemäß YAMAMOTO, Polymerisation gemäß STILLE oder Polymerisation gemäß HARTWIG-BUCHWALD hergestellt werden. Die erfindungsgemäßen Dendrimere können gemäß dem Fachmann bekannten Verfahren oder in Analogie dazu hergestellt werden. Geeignete Verfahren sind in der Literatur beschrieben, wie z. B. in Frechet, Jean M. J.; Hawker, Craig J., "Hyperbranched polyphenylene and hyperbranched polyesters: new soluble, three-dimensional, reactive polymers", Reactive & Functional Polymers (1995), 26(1-3), 127-36; Janssen, H. M.; Meijer, E. W., "The synthesis and characterization of dendritic molecules", Materials Science and Technology (1999), 20 (Synthesis of Polymers), 403-458; Tomalia, Donald A., "Dendrimer molecules", Scientific American (1995), 272(5), 62-6; WO 2002/067343 A1 und WO 2005/026144 A1.

Für die Verarbeitung der erfindungsgemäßen Verbindungen aus flüssiger Phase, beispielsweise durch Spin-Coating oder durch Druckverfahren, sind Formulierungen der erfindungsgemäßen Verbindungen erforderlich. Diese Formulierungen können beispielsweise Lösungen, Dispersionen oder Miniemulsionen sein. Es kann bevorzugt sein, hierfür Mischungen aus zwei oder mehr Lösemitteln zu verwenden. Geeignete und bevorzugte Lösemittel sind beispielsweise Toluol, Anisol, o-, m- oder p-Xylol, Methylbenzoat, Dimethylanisol, Mesitylen, Tetralin, Veratrol, THF, Methyl-THF, THP, Chlorbenzol, Dioxan oder Mischungen dieser Lösemittel. Es ist bevorzugt, dass die Formulierung enthaltend die erfindungsgemäße Mischung ein oder mehrere Polymere, Oligomere oder Dendrimere enthält. Die Polymere, Oligomere oder Dendrimere sind bevorzugt in einer Konzentration von 1-80 Gew.-%, besonders bevorzugt 5-65 Gew.-% und ganz besonders bevorzugt 10-50 Gew.-% in der Formulierung enthalten. Sie dienen insbesondere zum Einstellen der Eigenschaften der Formulierung, beispielsweise der Viskosität. Bevorzugt ist die Verwendung von Polyarylaminen, Polystyrolen, Polyacrylaten und Polyestern, insbesondere die Verwendung der in der WO 2011/076325 offenbarten Polymere.

Gegenstand der Erfindung ist daher weiterhin eine Formulierung, insbesondere eine Lösung, Dispersion oder Miniemulsion, enthaltend mindestens eine Verbindung gemäß Formel (I) oder mindestens ein Polymer, Oligomer oder Dendrimer enthaltend mindestens eine Einheit gemäß Formel (I) sowie mindestens ein Lösungsmittel, bevorzugt ein organisches Lösungsmittel. Wie solche Lösungen hergestellt werden können, ist dem Fachmann bekannt und beispielsweise in WO 2002/072714, WO 2003/019694 und der darin zitierten Literatur beschrieben.

Die erfindungsgemäßen Verbindungen gemäß Formel (I) eignen sich für den Einsatz in elektronischen Vorrichtungen, insbesondere in organischen Elektrolumineszenzvorrichtungen (OLEDs). Abhängig von der Substitution werden die Verbindungen in unterschiedlichen Funktionen und Schichten eingesetzt.

Weitere Gegenstände der Erfindung sind daher die Verwendung der Verbindungen gemäß Formel (I) in elektronischen Vorrichtungen sowie elektronische Vorrichtungen selbst, welche eine oder mehrere Verbindungen gemäß Formel (I) enthalten. Dabei sind die elektronischen Vorrichtungen bevorzugt ausgewählt aus der Gruppe bestehend aus organischen integrierten Schaltungen (OICs), organischen Feld-Effekt-Transistoren (OFETs), organischen Dünnfilmtransistoren (OTFTs), organischen lichtemittierenden Transistoren (OLETs), organischen Solarzellen (OSCs), organischen optischen Detektoren, organischen Photorezeptoren, organischen Feld-Quench-Devices (OFQDs), organischen lichtemittierenden elektrochemischen Zellen (OLECs), organischen Laserdioden (O-Laser) und besonders bevorzugt organischen Elektrolumineszenzvorrichtungen (OLEDs).

Gegenstand der Erfindung sind, wie bereits oben ausgeführt, elektronische Vorrichtungen, enthaltend mindestens eine Verbindung gemäß Formel (I). Dabei sind die elektronischen Vorrichtungen bevorzugt ausgewählt aus den oben genannten Vorrichtungen. Besonders bevorzugt sind organische Elektrolumineszenzvorrichtungen, enthaltend Anode, Kathode und mindestens eine emittierende Schicht, dadurch gekennzeichnet, dass mindestens eine organische Schicht, die eine emittierende Schicht, eine Lochtransportschicht oder eine andere Schicht sein kann, mindestens eine Verbindung gemäß Formel (I) enthält.

Außer Kathode, Anode und der emittierenden Schicht kann die organische Elektrolumineszenzvorrichtung noch weitere Schichten enthalten. Diese sind beispielsweise gewählt aus jeweils einer oder mehreren Lochinjektionsschichten, Lochtransportschichten, Lochblockierschichten, Elektronentransportschichten, Elektroneninjektionsschichten, Elektronenblockierschichten, Excitonenblockierschichten, Zwischenschichten (Interlayers), Ladungserzeugungsschichten (Charge-Generation Layers) (IDMC 2003, Taiwan; Session 21 OLED (5), T. Matsumoto, T. Nakada, J. Endo, K. Mori, N. Kawamura, A. Yokoi, J. Kido, Multiphoton Organic EL Device Having Charge Generation Layer) und/oder organischen oder anorganischen p/n-Übergängen. Es sei aber darauf hingewiesen, dass nicht notwendigerweise jede dieser Schichten vorhanden sein muss und die Wahl der Schichten immer von den verwendeten Verbindungen abhängt und insbesondere auch von der Tatsache, ob es sich um eine fluoreszierende oder phosphoreszierende Elektrolumineszenzvorrichtung handelt.

Die erfindungsgemäße organische Elektrolumineszenzvorrichtung kann mehrere emittierende Schichten enthalten. Besonders bevorzugt weisen diese Emissionsschichten in diesem Fall insgesamt mehrere Emissionsmaxima zwischen 380 nm und 750 nm auf, so dass insgesamt weiße Emission resultiert, d. h. in den emittierenden Schichten werden verschiedene emittierende Verbindungen verwendet, die fluoreszieren oder phosphoreszieren können und die blaues oder gelbes oder orangefarbenes oder rotes Licht emittieren. Insbesondere bevorzugt sind Dreischichtsysteme, also Systeme mit drei emittierenden Schichten, wobei die drei Schichten blaue, grüne und orange oder rote Emission zeigen (für den prinzipiellen Aufbau siehe z. B. WO 2005/011013). Die erfindungsgemäßen Verbindungen können in solchen Vorrichtungen in einer Lochtransportschicht, einer emittierenden Schicht und/oder in einer anderen Schicht vorhanden sein. Es soll angemerkt werden, dass sich für die Erzeugung von weißem Licht anstelle mehrerer farbig emittierender Emitterverbindungen auch eine einzeln verwendete Emitterverbindung eignen kann, welche in einem breiten Wellenlängenbereich emittiert.

Es ist erfindungsgemäß bevorzugt, wenn die Verbindung gemäß Formel (I) in einer elektronischen Vorrichtung enthaltend einen oder mehrere phosphoreszierende Dotanden eingesetzt wird. Dabei kann die Verbindung in unterschiedlichen Schichten, bevorzugt in einer Lochtransportschicht, einer Lochinjektionsschicht oder in einer emittierenden Schicht, verwendet werden. Die Verbindung gemäß Formel (I) kann aber auch erfindungsgemäß in einer elektronischen Vorrichtung enthaltend einen oder mehrere fluoreszierende Dotanden eingesetzt werden.

Vom Begriff phosphoreszierende Dotanden sind typischerweise Verbindungen umfasst, bei denen die Lichtemission durch einen spinverbotenen Übergang erfolgt, beispielsweise einen Übergang aus einem angeregten Triplettzustand oder einem Zustand mit einer höheren Spinquantenzahl, beispielsweise einem Quintett-Zustand.

Als phosphoreszierende Dotanden (= Triplettemitter) eignen sich insbesondere Verbindungen, die bei geeigneter Anregung Licht, vorzugsweise im sichtbaren Bereich, emittieren und außerdem mindestens ein Atom der Ordnungszahl größer 20, bevorzugt größer 38 und kleiner 84, besonders bevorzugt größer 56 und kleiner 80 enthalten. Bevorzugt werden als Phosphoreszenzemitter Verbindungen, die Kupfer, Molybdän, Wolfram, Rhenium, Ruthenium, Osmium, Rhodium, Iridium, Palladium, Platin, Silber, Gold oder Europium enthalten, verwendet, insbesondere Verbindungen, die Iridium, Platin oder Kupfer enthalten.

Dabei werden im Sinne der vorliegenden Erfindung alle lumineszierenden Iridium-, Platin- oder Kupferkomplexe als phosphoreszierende Verbindungen angesehen.

Beispiele der oben beschriebenen Emitter können den Anmeldungen WO 00/70655, WO 01/41512, WO 02/02714, WO 02/15645, EP 1191613, EP 1191612, EP 1191614, WO 05/033244, WO 05/019373 und US 2005/0258742 entnommen werden. Generell eignen sich alle phosphoreszierenden Komplexe, wie sie gemäß dem Stand der Technik für phosphoreszierende OLEDs verwendet werden und wie sie dem Fachmann auf dem Gebiet der organischen Elektrolumineszenzvorrichtungen bekannt sind. Auch kann der Fachmann ohne erfinderisches Zutun weitere phosphoreszierende Komplexe in Kombination mit den Verbindungen gemäß Formel (I) in organischen Elektrolumineszenzvorrichtungen einsetzen.

Explizite Beispiele für geeignete phosphoreszierende Emitterverbindungen können weiterhin der folgenden Tabelle entnommen werden.

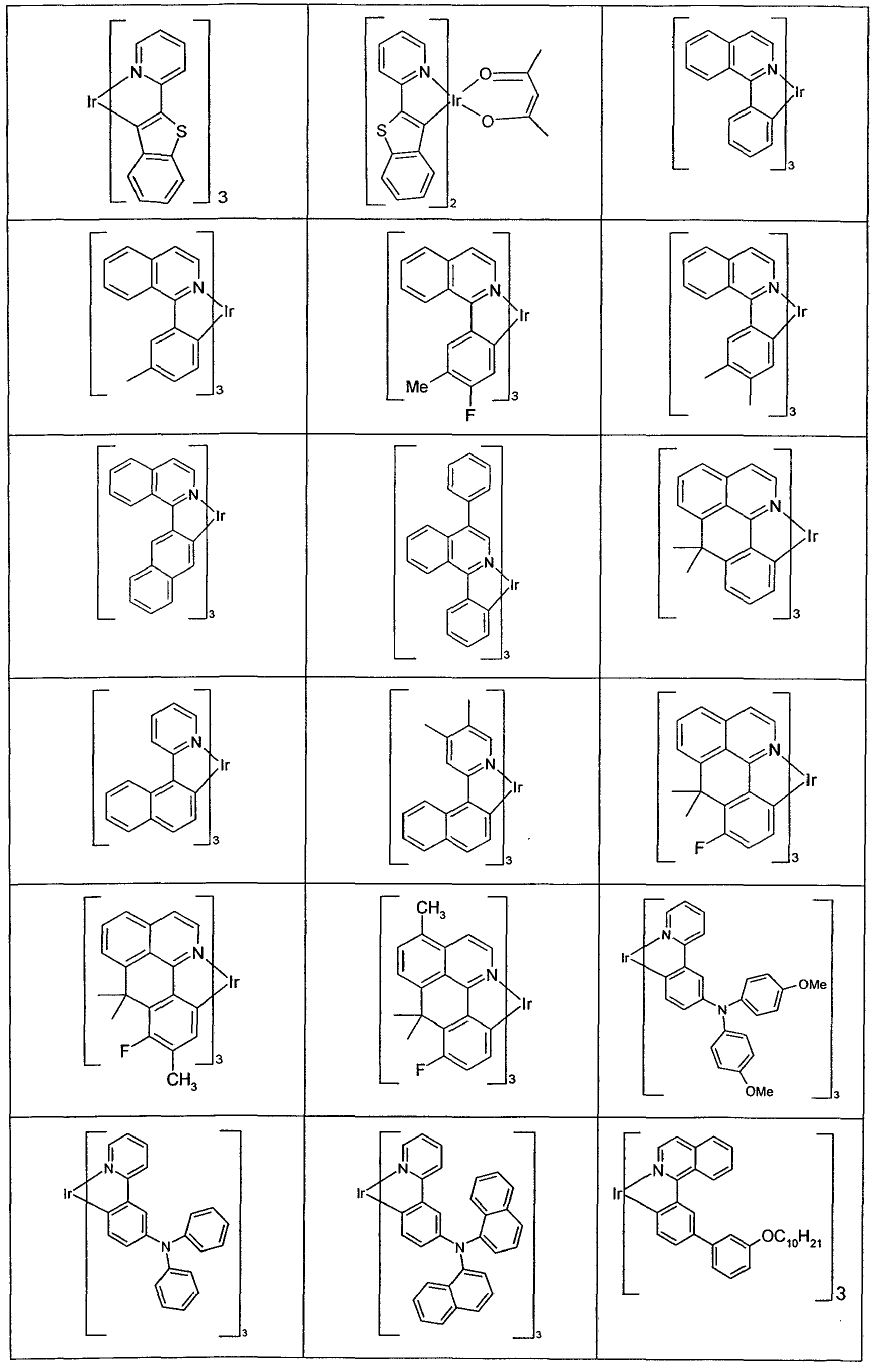

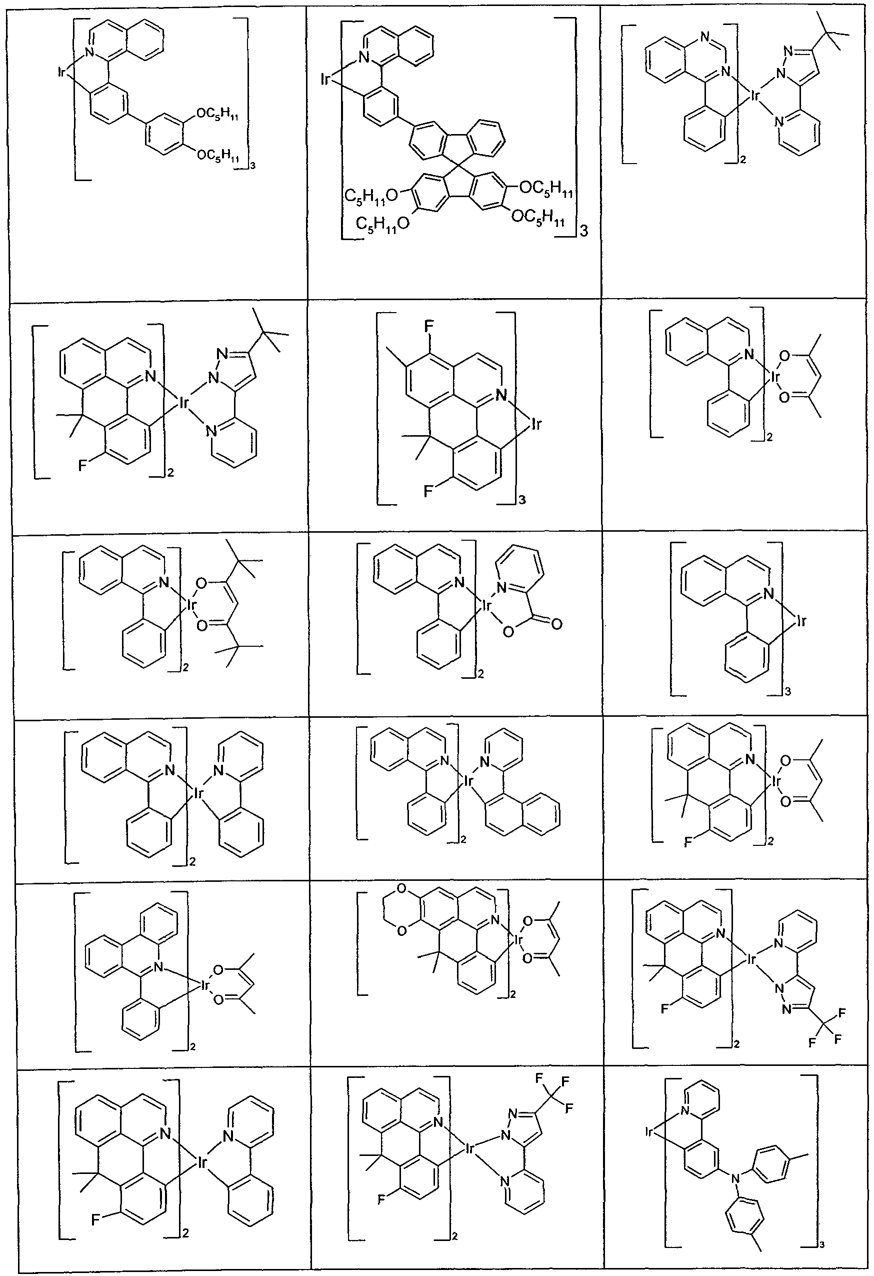

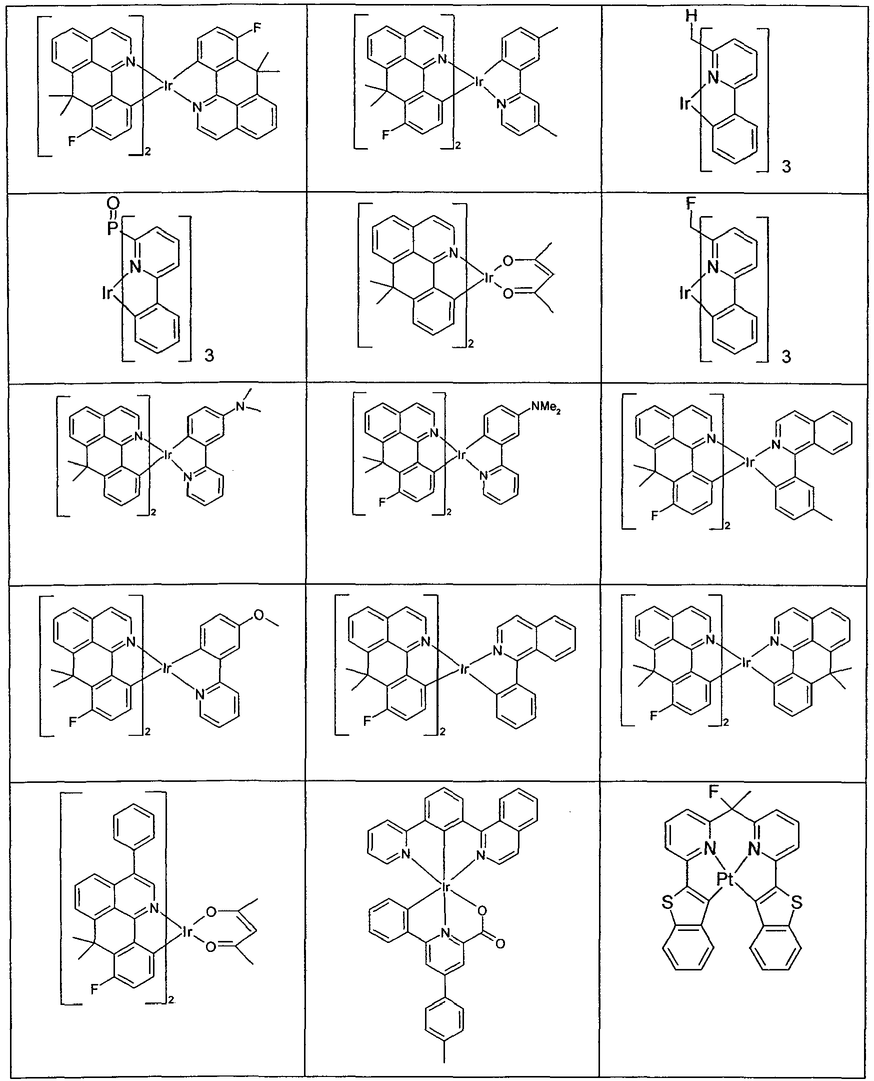

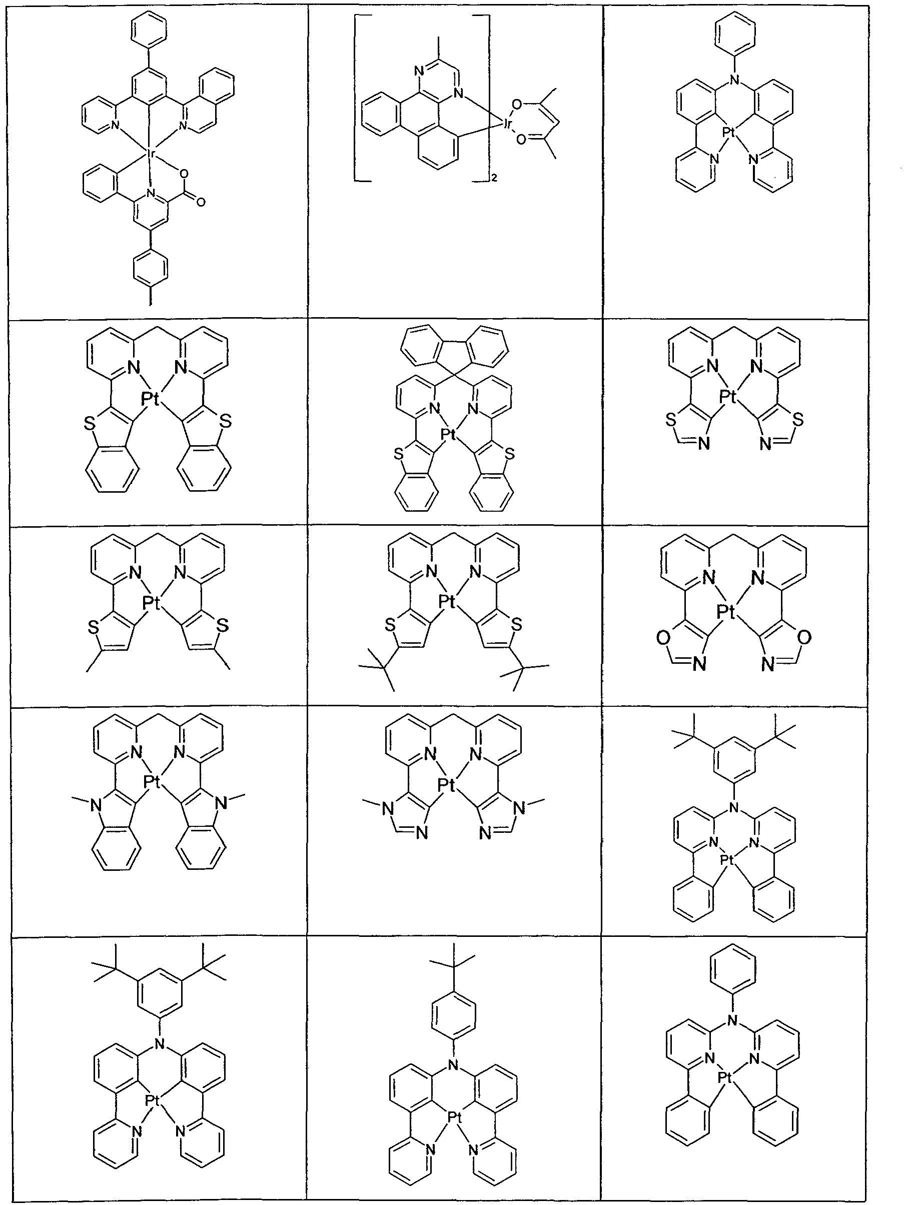

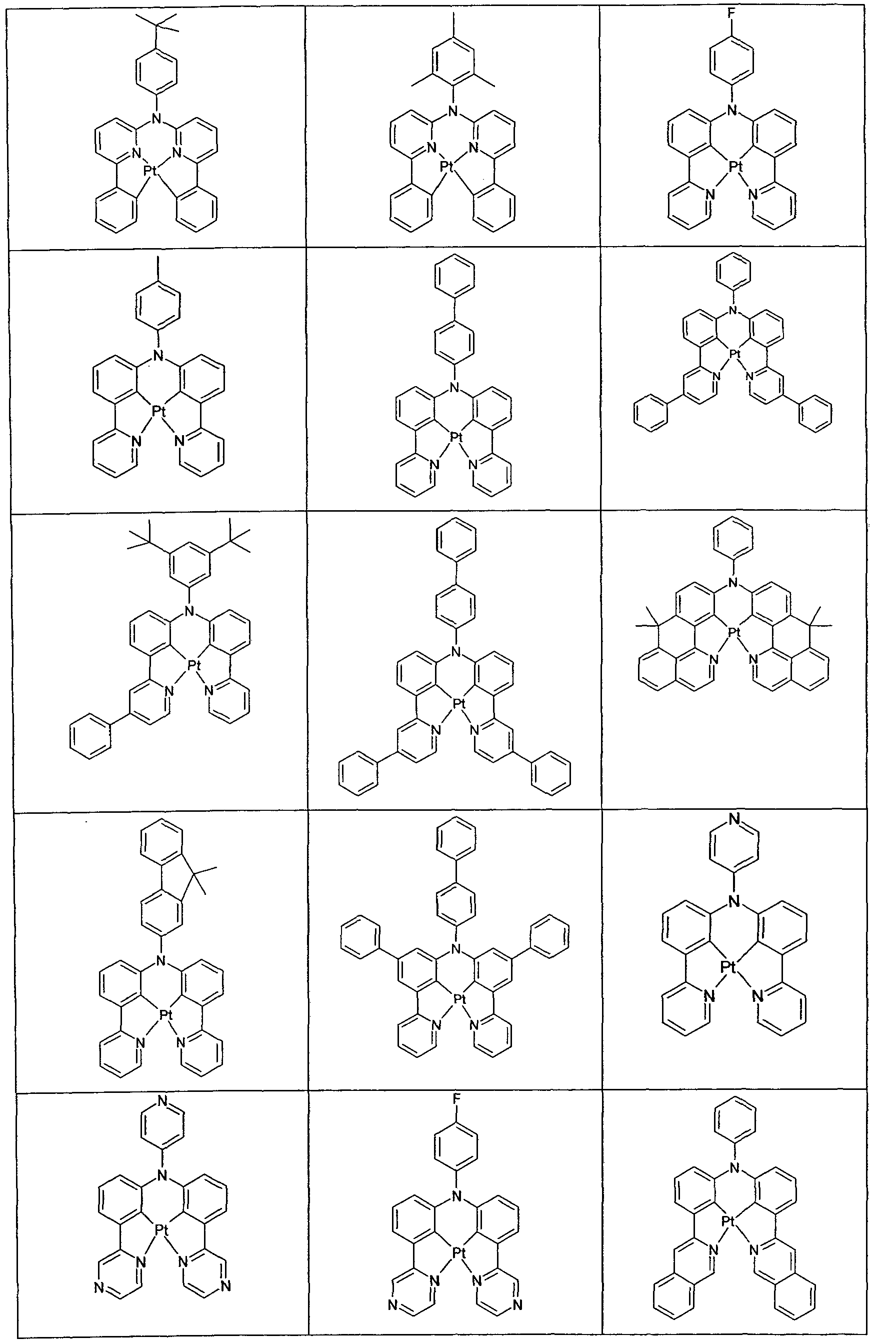

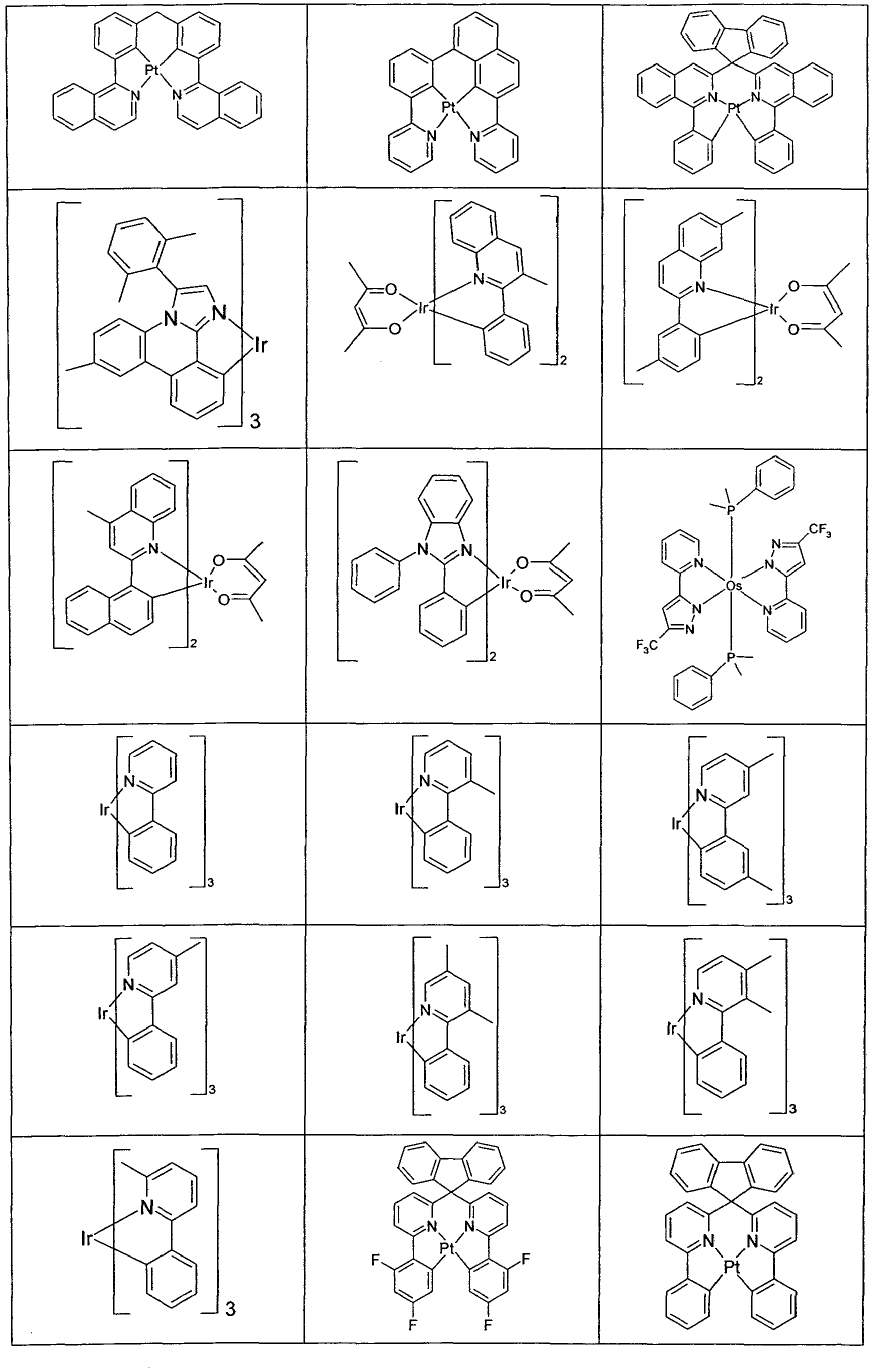

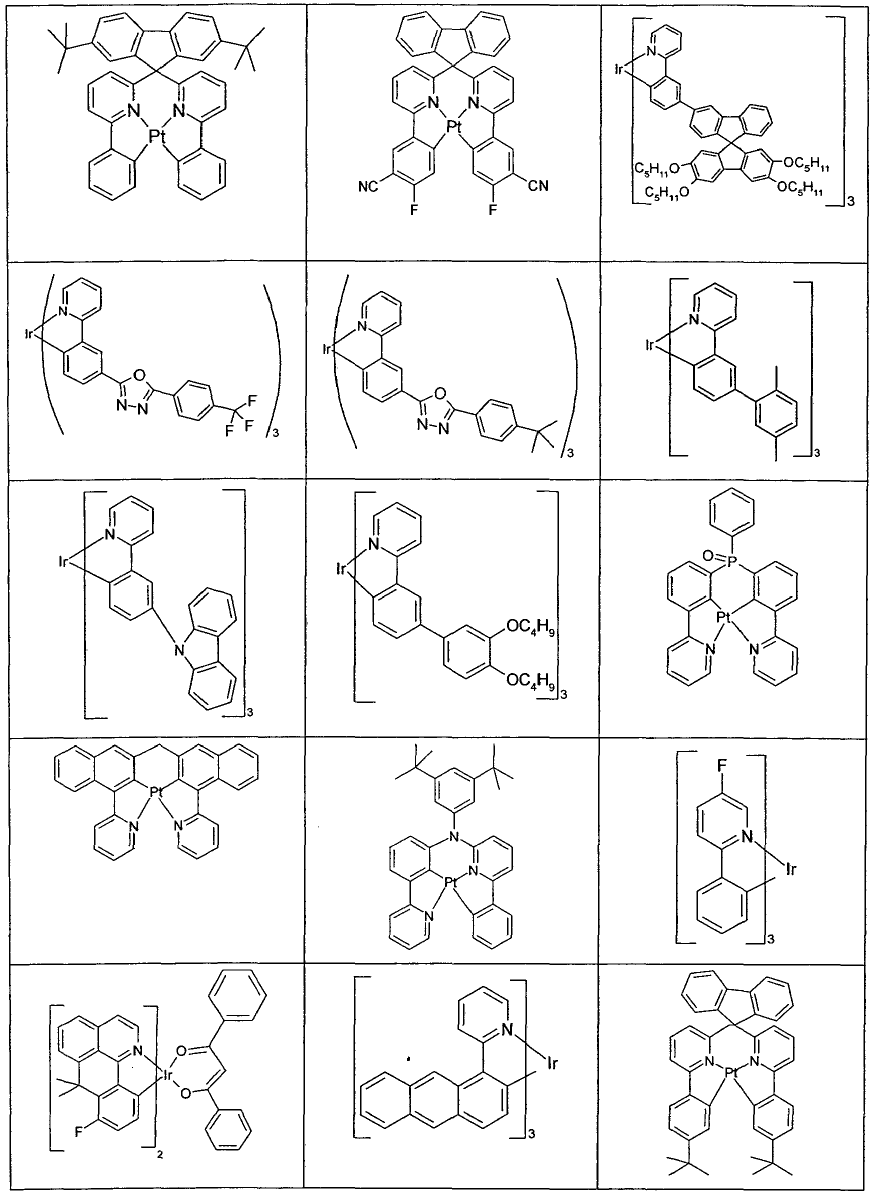

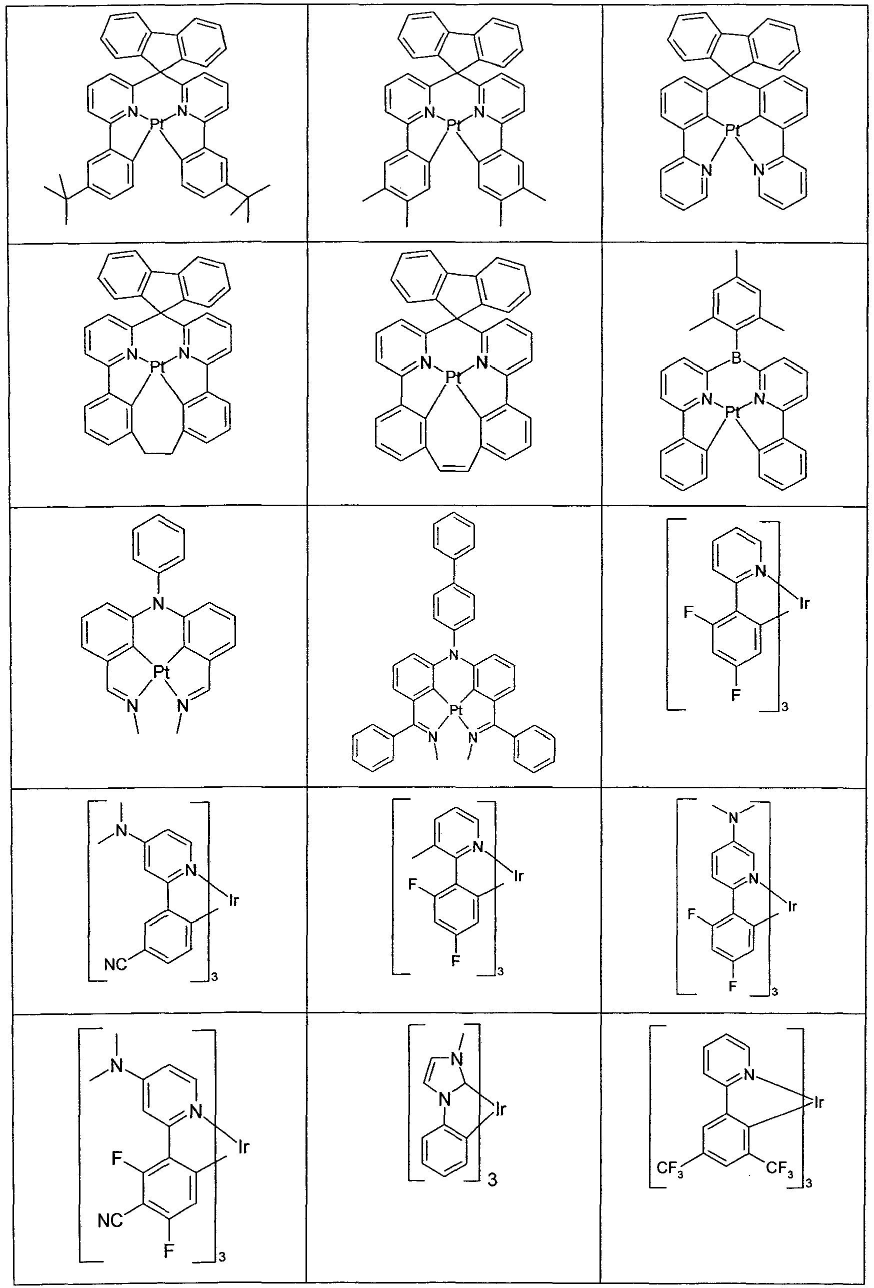

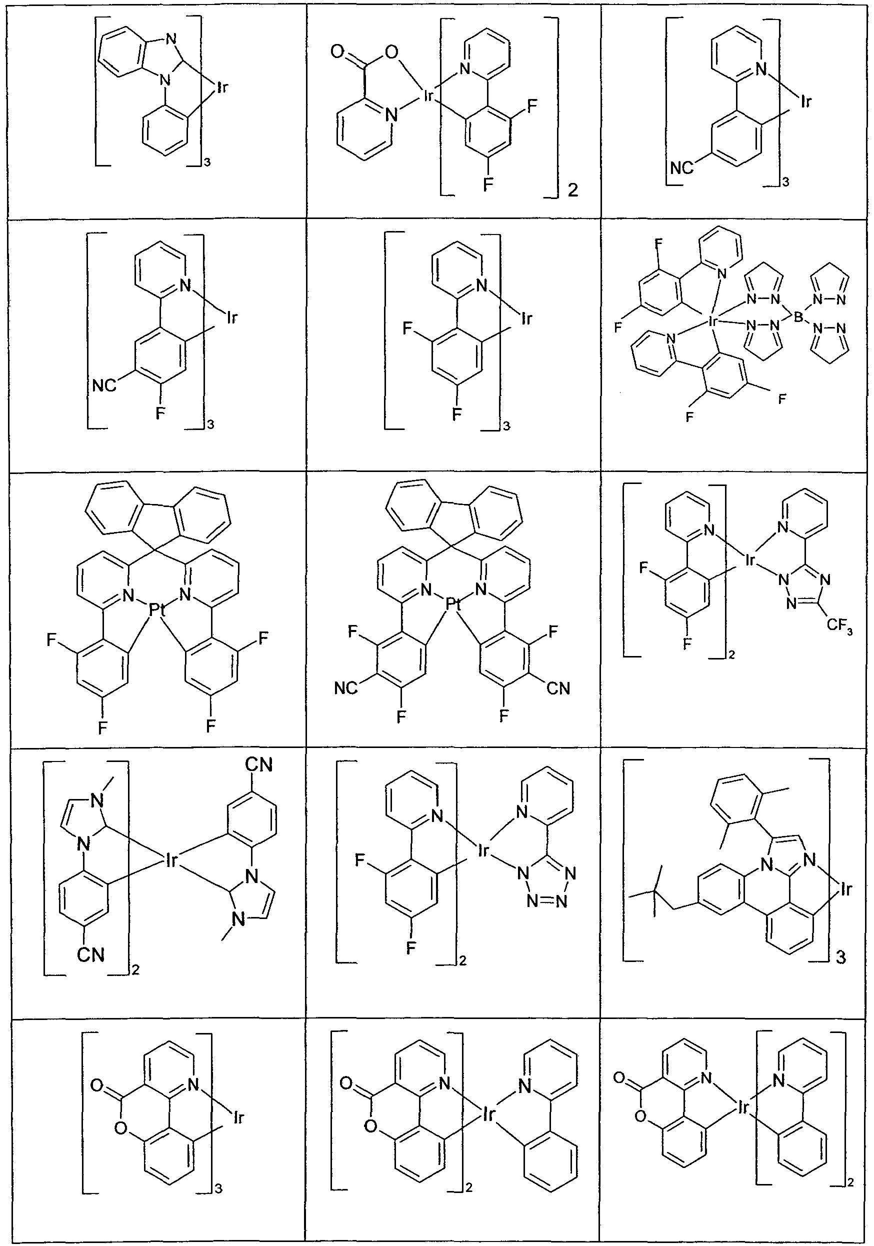

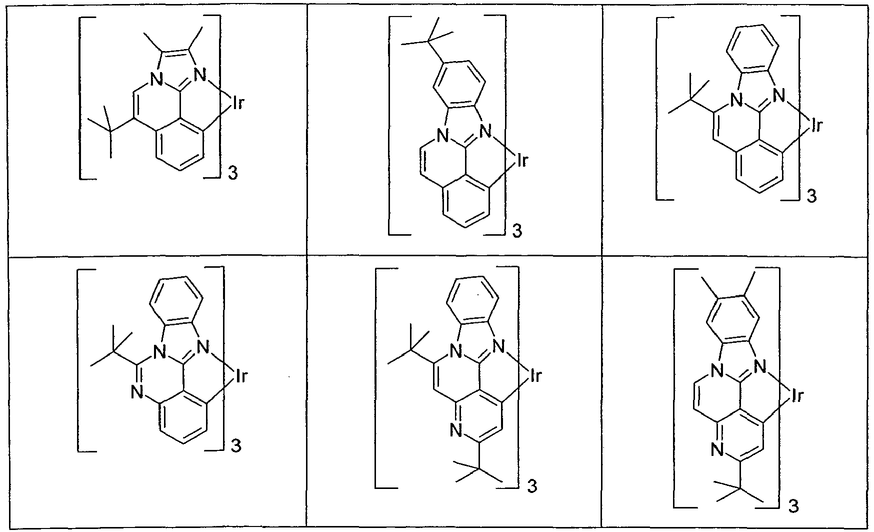

In einer bevorzugten Ausführungsform der Erfindung werden die Verbindungen gemäß Formel (I) als Lochtransportmaterial eingesetzt. Die Verbindungen werden entsprechend bevorzugt in einer Lochtransportschicht, einer Lochinjektionsschicht und/oder einer Elektronenblockierschicht eingesetzt.

Eine Lochinjektionsschicht im Sinne dieser Erfindung ist eine Schicht, die direkt an die Anode angrenzt. Eine Lochtransportschicht im Sinne dieser Erfindung ist eine Schicht, die zwischen der Lochinjektionsschicht und der Emissionsschicht liegt. Die Lochtransportschicht kann direkt an die Emissionschicht angrenzen. Eine Elektronenblockierschicht im Sinne dieser Erfindung ist eine Schicht, die anodenseitig direkt an die Emissionsschicht angrenzt. Insbesondere wird unter einer Elektronenblockierschicht eine Schicht verstanden, welche anodenseitig direkt an die Emissionsschicht angrenzt, wobei zusätzlich anodenseitig zur Elektronenblockierschicht eine oder mehrere Lochtransportschichten vorhanden sind. Bevorzugt enthalten die Lochinjektionsschicht, Lochtransportschicht und Elektronenblockierschicht jeweils Materialien mit lochtransportierenden Eigenschaften. Für die Verwendung in der Elektronenblockierschicht ist es insbesondere bevorzugt, dass die Verbindung ein hochliegendes LUMO aufweist.

In einer weiteren Ausführungsform der vorliegenden Erfindung werden die Verbindungen der Formel (I) als Matrixmaterial in Kombination mit einem oder mehreren fluoreszierenden oder phosphoreszierenden Dotanden eingesetzt.

Unter einem Dotanden wird in einem System enthaltend ein Matrixmaterial und einen Dotanden diejenige Komponente verstanden, deren Anteil in der Mischung der kleinere ist. Entsprechend wird unter einem Matrixmaterial in einem System enthaltend ein Matrixmaterial und einen Dotanden diejenige Komponente verstanden, deren Anteil in der Mischung der größere ist.

Der Anteil des Matrixmaterials in der emittierenden Schicht beträgt in diesem Fall zwischen 50.0 und 99.9 Vol.-%, bevorzugt zwischen 80.0 und 99.5 Vol.-% und besonders bevorzugt für fluoreszierende emittierende Schichten zwischen 92.0 und 99.5 Vol.-% sowie für phosphoreszierende emittierende Schichten zwischen 85.0 und 97.0 Vol.-%.

Entsprechend beträgt der Anteil des Dotanden zwischen 0.1 und 50.0 Vol.-%, bevorzugt zwischen 0.5 und 20.0 Vol.-% und besonders bevorzugt für fluoreszierende emittierende Schichten zwischen 0.5 und 8.0 Vol.-% sowie für phosphoreszierende emittierende Schichten zwischen 3.0 und 15.0 Vol.-%.

Im Folgenden werden die in den erfindungsgemäßen Vorrichtungen in den betreffenden Funktionen bevorzugt eingesetzten Materialien aufgeführt.

Bevorzugte fluoreszierende Dotanden sind ausgewählt aus der Klasse der Arylamine. Unter einem Arylamin bzw. einem aromatischen Amin im Sinne dieser Erfindung wird eine Verbindung verstanden, die drei substituierte oder unsubstituierte aromatische oder heteroaromatische Ringsysteme direkt an den Stickstoff gebunden enthält. Bevorzugt ist mindestens eines dieser aromatischen oder heteroaromatischen Ringsysteme ein kondensiertes Ringsystem, besonders bevorzugt mit mindestens 14 aromatischen Ringatomen. Bevorzugte Beispiele hierfür sind aromatische Anthracenamine, aromatische Anthracendiamine, aromatische Pyrenamine, aromatische Pyrendiamine, aromatische Chrysenamine oder aromatische Chrysendiamine. Unter einem aromatischen Anthracenamin wird eine Verbindung verstanden, in der eine Diarylaminogruppe direkt an eine Anthracengruppe gebunden ist, vorzugsweise in 9-Position. Unter einem aromatischen Anthracendiamin wird eine Verbindung verstanden, in der zwei Diarylaminogruppen direkt an eine Anthracengruppe gebunden sind, vorzugsweise in 9,10-Position. Aromatische Pyrenamine, Pyrendiamine, Chrysenamine und Chrysendiamine sind analog dazu definiert, wobei die Diarylaminogruppen am Pyren bevorzugt in 1-Position bzw. in 1,6-Position gebunden sind.

Als Matrixmaterialien, bevorzugt für fluoreszierende Dotanden, kommen neben den erfindungsgemäßen Verbindungen Materialien verschiedener Stoffklassen in Frage. Bevorzugte Matrixmaterialien sind ausgewählt aus den Klassen der Oligoarylene (z. B. 2,2',7,7'-Tetraphenylspirobifluoren gemäß EP 676461 oder Dinaphthylanthracen), insbesondere der Oligoarylene enthaltend kondensierte aromatische Gruppen, der Oligoarylenvinylene (z. B. DPVBi oder Spiro-DPVBi gemäß EP 676461), der polypodalen Metallkomplexe (z. B. gemäß WO 2004/081017), der lochleitenden Verbindungen (z. B. gemäß WO 2004/058911), der elektronenleitenden Verbindungen, insbesondere Ketone, Phosphinoxide, Sulfoxide, etc. (z. B. gemäß WO 2005/084081 und WO 2005/084082), der Atropisomere (z. B. gemäß WO 2006/048268), der Boronsäurederivate (z. B. gemäß WO 2006/117052) oder der Benzanthracene (z. B. gemäß WO 2008/145239). Besonders bevorzugte Matrixmaterialien sind ausgewählt aus den Klassen der Oligoarylene, enthaltend Naphthalin, Anthracen, Benzanthracen und/oder Pyren oder Atropisomere dieser Verbindungen, der Oligoarylenvinylene, der Ketone, der Phosphinoxide und der Sulfoxide. Ganz besonders bevorzugte Matrixmaterialien sind ausgewählt aus den Klassen der Oligoarylene, enthaltend Anthracen, Benzanthracen, Benzphenanthren und/oder Pyren oder Atropisomere dieser Verbindungen. Unter einem Oligoarylen im Sinne dieser Erfindung soll eine Verbindung verstanden werden, in der mindestens drei Aryl- bzw. Arylengruppen aneinander gebunden sind.

Bevorzugte Matrixmaterialien für phosphoreszierende Dotanden sind neben den erfindungsgemäßen Verbindungen aromatische Amine, insbesondere Triarylamine, z. B. gemäß US 2005/0069729, Carbazolderivate (z. B. CBP, N,N-Biscarbazolylbiphenyl) oder Verbindungen gemäß WO 2005/039246, US 2005/0069729, JP 2004/288381, EP 1205527 oder WO 2008/086851, verbrückte Carbazolderivate, z. B. gemäß WO 2011/088877 und WO 2011/128017, Indenocarbazolderivate, z. B. gemäß WO 2010/136109 und WO 2011/000455, Azacarbazolderivate, z. B. gemäß EP 1617710, EP 1617711, EP 1731584, JP 2005/347160, Indolocarbazolderivate, z. B. gemäß WO 2007/063754 oder WO 2008/056746, Ketone, z. B. gemäß WO 2004/093207 oder WO 2010/006680, Phosphinoxide, Sulfoxide und Sulfone, z. B. gemäß WO 2005/003253, Oligophenylene, bipolare Matrixmaterialien, z. B. gemäß WO 2007/137725, Silane, z. B. gemäß WO 2005/111172, Azaborole oder Boronester, z. B. gemäß WO 2006/117052, Triazinderivate, z. B. gemäß WO 2010/015306, WO 2007/063754 oder WO 2008/056746, Zinkkomplexe, z. B. gemäß EP 652273 oder WO 2009/062578, Aluminiumkomplexe, z. B. BAIq, Diazasilol- und Tetraazasilol-Derivate, z. B. gemäß WO 2010/054729, Diazaphosphol-Derivate, z. B. gemäß WO 2010/054730 und Aluminiumkomplexe, z. B. BAIQ.

Geeignete Ladungstransportmaterialien, wie sie in der Lochinjektions- bzw. Lochtransportschicht oder in der Elektronentransportschicht der erfindungsgemäßen organischen Elektrolumineszenzvorrichtung verwendet werden können, sind neben den erfindungsgemäßen Verbindungen beispielsweise die in Y. Shirota et al., Chem. Rev. 2007, 107(4), 953-1010 offenbarten Verbindungen oder andere Materialien, wie sie gemäß dem Stand der Technik in diesen Schichten eingesetzt werden.

Als Kathode der organischen Elektrolumineszenzvorrichtung sind Metalle mit geringer Austrittsarbeit, Metalllegierungen oder mehrlagige Strukturen aus verschiedenen Metallen bevorzugt, wie beispielsweise Erdalkalimetalle, Alkalimetalle, Hauptgruppenmetalle oder Lanthanoide (z. B. Ca, Ba, Mg, Al, In, Mg, Yb, Sm, etc.). Weiterhin eignen sich Legierungen aus einem Alkali- oder Erdalkalimetall und Silber, beispielsweise eine Legierung aus Magnesium und Silber. Bei mehrlagigen Strukturen können auch zusätzlich zu den genannten Metallen weitere Metalle verwendet werden, die eine relativ hohe Austrittsarbeit aufweisen, wie z. B. Ag oder Al, wobei dann in der Regel Kombinationen der Metalle, wie beispielsweise Ca/Ag, Mg/Ag oder Ba/Ag verwendet werden. Es kann auch bevorzugt sein, zwischen einer metallischen Kathode und dem organischen Halbleiter eine dünne Zwischenschicht eines Materials mit einer hohen Dielektrizitätskonstante einzubringen. Hierfür kommen beispielsweise Alkalimetall- oder Erdalkalimetallfluoride, aber auch die entsprechenden Oxide oder Carbonate in Frage (z. B. LiF, Li₂O, BaF₂, MgO, NaF, CsF, Cs₂CO₃, etc.). Weiterhin kann dafür Lithiumchinolinat (LiQ) verwendet werden. Die Schichtdicke dieser Schicht beträgt bevorzugt zwischen 0.5 und 5 nm.

Als Anode sind Materialien mit hoher Austrittsarbeit bevorzugt. Bevorzugt weist die Anode eine Austrittsarbeit größer 4.5 eV vs. Vakuum auf. Hierfür sind einerseits Metalle mit hohem Redoxpotential geeignet, wie beispielsweise Ag, Pt oder Au. Es können andererseits auch Metall/Metalloxid-Elektroden (z. B. Al/Ni/NiOₓ, Al/PtOₓ) bevorzugt sein. Für einige Anwendungen muss mindestens eine der Elektroden transparent oder teiltransparent sein, um entweder die Bestrahlung des organischen Materials (organische Solarzelle) oder die Auskopplung von Licht (OLED, O-LASER) zu ermöglichen. Bevorzugte Anodenmaterialien sind hier leitfähige gemischte Metalloxide. Besonders bevorzugt sind Indium-ZinnOxid (ITO) oder Indium-Zink Oxid (IZO). Bevorzugt sind weiterhin leitfähige, dotierte organische Materialien, insbesondere leitfähige dotierte Polymere.

Die Vorrichtung wird entsprechend (je nach Anwendung) strukturiert, kontaktiert und schließlich versiegelt, da sich die Lebensdauer der erfindungsgemäßen Vorrichtungen bei Anwesenheit von Wasser und/oder Luft verkürzt.

In einer bevorzugten Ausführungsform ist die erfindungsgemäße organische Elektrolumineszenzvorrichtung dadurch gekennzeichnet, dass eine oder mehrere Schichten mit einem Sublimationsverfahren beschichtet werden. Dabei werden die Materialien in Vakuum-Sublimationsanlagen bei einem Anfangsdruck kleiner 10⁻⁵ mbar, bevorzugt kleiner 10⁻⁶ mbar aufgedampft. Dabei ist es jedoch auch möglich, dass der Anfangsdruck noch geringer ist, beispielsweise kleiner 10⁻⁷ mbar.

Bevorzugt ist ebenfalls eine organische Elektrolumineszenzvorrichtung, dadurch gekennzeichnet, dass eine oder mehrere Schichten mit dem OVPD (Organic Vapour Phase Deposition) Verfahren oder mit Hilfe einer Trägergassublimation beschichtet werden. Dabei werden die Materialien bei einem Druck zwischen 10⁻⁵ mbar und 1 bar aufgebracht. Ein Spezialfall dieses Verfahrens ist das OVJP (Organic Vapour Jet Printing) Verfahren, bei dem die Materialien direkt durch eine Düse aufgebracht und so strukturiert werden (z. B. M. S. Arnold et al., Appl. Phys. Lett. 2008, 92, 053301).

Weiterhin bevorzugt ist eine organische Elektrolumineszenzvorrichtung, dadurch gekennzeichnet, dass eine oder mehrere Schichten aus Lösung, wie z. B. durch Spincoating, oder mit einem beliebigen Druckverfahren, wie z. B. Siebdruck, Flexodruck, Nozzle Printing oder Offsetdruck, besonders bevorzugt aber LITI (Light Induced Thermal Imaging, Thermotransferdruck) oder Ink-Jet Druck (Tintenstrahldruck), hergestellt werden. Hierfür sind lösliche Verbindungen gemäß Formel (I) nötig. Hohe Löslichkeit lässt sich durch geeignete Substitution der Verbindungen erreichen.

Weiterhin bevorzugt ist es, dass zur Herstellung einer erfindungsgemäßen organischen Elektrolumineszenzvorrichtung eine oder mehrere Schichten aus Lösung und eine oder mehrere Schichten durch ein Sublimationsverfahren aufgetragen werden.

Erfindungsgemäß können die elektronischen Vorrichtungen enthaltend eine oder mehrere Verbindungen gemäß Formel (I) in Displays, als Lichtquellen in Beleuchtungsanwendungen sowie als Lichtquellen in medizinischen und/oder kosmetischen Anwendungen (z.B. Lichttherapie) eingesetzt werden.

Die Erfindung wird durch die nachfolgenden Anwendungsbeispiele näher erläutert, wobei die Erfindung nicht auf den Umfang der Beispiele beschränkt ist.

### Ausführungsbeispiele

### A) Synthesebeispiele

Die nachfolgenden Synthesen wurden, sofern nicht anders angegeben, unter einer Schutzgasatmosphäre in getrockneten Lösungsmitteln durchgeführt. Die Edukte können z.B. von ALDRICH bzw. ABCR bezogen werden. Angaben in eckigen Klammern betreffen die CAS-Nummern.

### Beispiel 1: Bis-biphenyl-4-yl-biphenyl-2-yl-amin

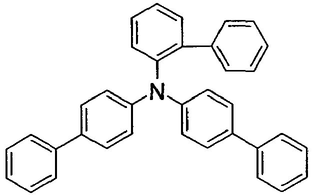

Ein Gemisch aus 94.3 g (404 mmol) 2-Brombiphenyl [2052-07-5], 130.9 g (404 mmol) Bis-biphenyl-4-ylamin [102113-98-4], 48.1 g (500 mmol) Natrium-tert-buylat, 6.7 g (12 mmol) DPPF, 1.8 g (8 mmol) Palladium(II)acetat und 1500 ml Mesitylen wird 55 h unter Rückfluss erhitzt. Nach Erkalten wird mit 1000 ml Wasser versetzt, 30 min. gerührt, die wässrige Phase wird abgetrennt, die org. Phase wird dreimal mit je 500 ml Wasser gewaschen und dann im Vakuum eingeengt. Der so erhaltene graue Feststoff wird einer Heißextraktion über Alox, basisch, Aktivitätsstufe 1 unterzogen, anschließend fünfmal aus DMF (ca. 2 ml/g) umkristallisiert und dann zweimal fraktioniert sublimiert (p ca. 10⁻⁵ mbar, T ca. 260 - 270 °C). Ausbeute: 53.5 g (113 mmol), 28 %; Reinheit > 99.9 % n. HPLC.

Analog werden aus den entsprechenden sec.-Aminen und Bromiden folgende erfindungsgemäße Verbindungen hergestellt:

| **Bsp.** | **sec.-Amin** | **Bromid** | **tert.-Amin** | **Ausbeute** |
|---|---|---|---|---|
| 2 | 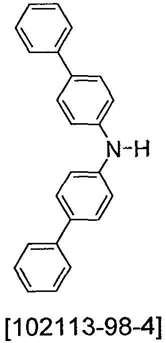 | 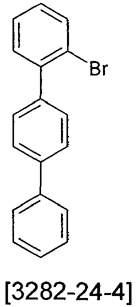 | 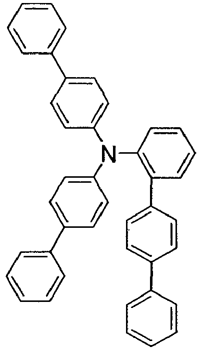 | 34 % |
| 3 | 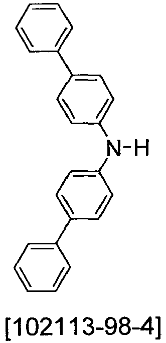 | 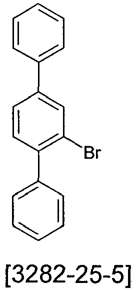 | 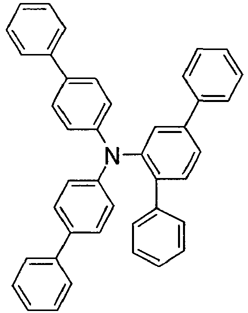 | 29 % |
| 4 | 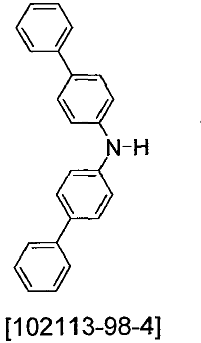 | 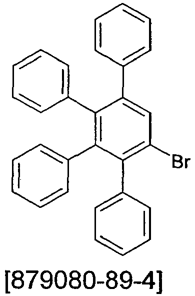 | 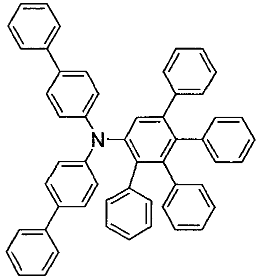 | 23 % |
| 5 | 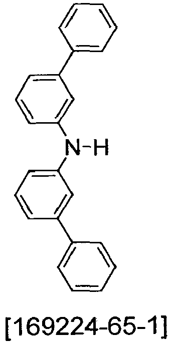 | 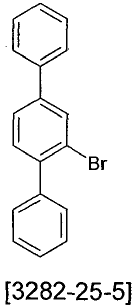 | 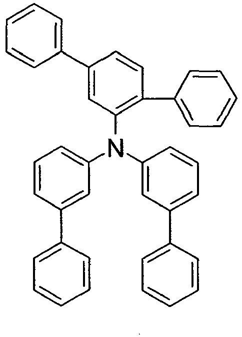 | 26 % |
| 6 | 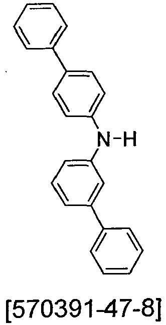 | 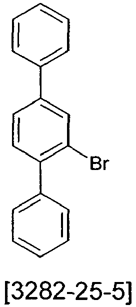 | 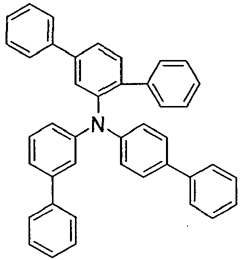 | 20 % |
| 7 | 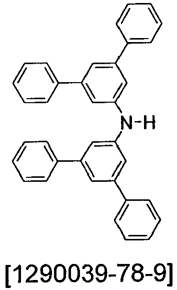 | 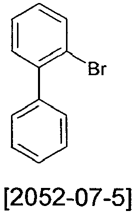 | 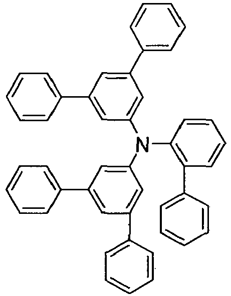 | 36 % |
| 8 | 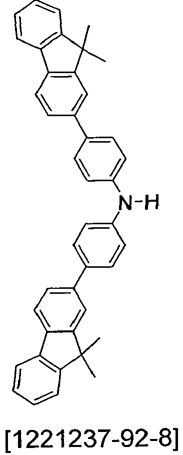 | 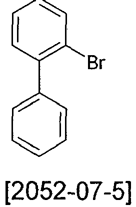 | 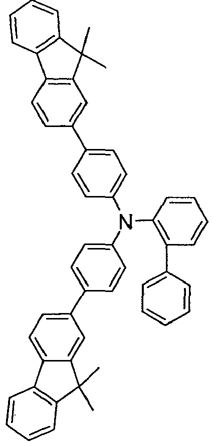 | 30 % |
| 9 | 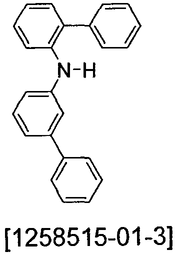 | 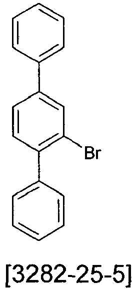 | 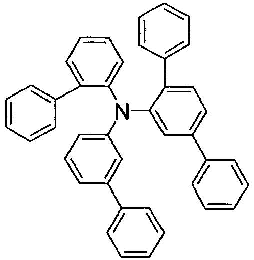 | 27 % |

### Beispiel 10: Biphenyl-2-yl-bis-[1,1'3,3']terphenyl-4-yl-amin

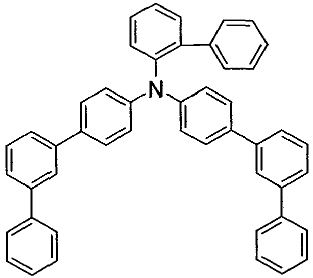

Ein Gemisch aus 33.8 g (200 mmol) 2-Aminobiphenyl [2052-07-5], 129.9 g (420 mmol) 4-Brom-[1,1'3,3']terphenyl [54590-37-3], 48.1 g (500 mmol) Natrium-tert-butylat, 6.7 g (12 mmol) DPPF, 1.8 g (8 mmol) Palladium(II)acetat und 1500 ml Mesitylen wird 48 h unter Rückfluss erhitzt. Nach Erkalten wird mit 1000 ml Wasser versetzt, 30 min. gerührt, die wässrige Phase wird abgetrennt, die org. Phase wird dreimal mit je 500 ml Wasser gewaschen und dann im Vakuum eingeengt. Der so erhaltene graue Feststoff wird einer Heißextraktion über Alox, basisch, Aktivitätsstufe 1, unterzogen, anschließend fünfmal aus DMF (ca. 2 ml/g) umkristallisiert und dann zweimal fraktioniert sublimiert (p ca. 10⁻⁵ mbar, T ca. 300 - 310 °C). Ausbeute: 48.0 g (77 mmol), 38.3 %; Reinheit > 99.9 % n. HPLC.

### B) Device-Beispiele

Die Herstellung von erfindungsgemäßen OLEDs sowie OLEDs nach dem Stand der Technik erfolgt nach einem allgemeinen Verfahren gemäß WO 04/058911, das auf die hier beschriebenen Gegebenheiten (Schichtdickenvariation, Materialien) angepasst wird.

In den folgenden Beispielen V1 bis V6 und E1 bis E12 (siehe Tabellen 1 bis 4) werden die Daten verschiedener OLEDs vorgestellt (V: Beispiele gemäß dem Stand der Technik; E: erfindungsgemäße Beispiele).

Glasplättchen, die mit strukturiertem ITO (Indium Zinn Oxid) der Dicke 150 nm beschichtet sind, werden zur verbesserten Prozessierung mit 20 nm PEDOT beschichtet (Poly(3,4-ethylendioxy-2,5-thiophen), aus Wasser aufgeschleudert; bezogen von H. C. Starck, Goslar, Deutschland). Diese beschichteten Glasplättchen bilden die Substrate, auf welche die OLEDs aufgebracht werden. Die OLEDs haben prinzipiell folgenden Schichtaufbau: Substrat / Lochinjektionsschicht (HIL) / Lochtransportschichten (HTL) / Zwischenschicht (IL)/optionale Lochtransportschicht (HTL2), Elektronenblockerschicht (EBL)/Emissionsschicht (EML) / Elektronentransportschicht (ETL) und abschließend eine Kathode. Die Kathode wird durch eine 100 nm dicke Aluminiumschicht gebildet. Der genaue Aufbau der OLEDs ist Tabelle 1 und 2 zu entnehmen. Die zur Herstellung der OLEDs benötigten Materialien sind in Tabelle 5 gezeigt.

Alle Materialien werden in einer Vakuumkammer thermisch aufgedampft. Dabei besteht die Emissionsschicht immer aus mindestens einem Matrixmaterial (Hostmaterial, Wirtsmaterial) und einem emittierenden Dotierstoff (Dotand, Emitter), der dem Matrixmaterial bzw. den Matrixmaterialien durch Coverdampfung in einem bestimmten Volumenanteil beigemischt wird. Eine Angabe wie H1 :SEB1 (95%:5%) bedeutet hierbei, dass das Material H1 in einem Volumenanteil von 95% und SEB1 in einem Anteil von 5% in der Schicht vorliegt. Analog kann auch die Elektronentransportschicht aus einer Mischung von zwei Materialien bestehen.

Die OLEDs werden standardmäßig charakterisiert. Hierfür werden die Elektrolumineszenzspektren, die Stromeffizienz (gemessen in cd/A), die Leistungseffizienz (gemessen in lm/W) und die externe Quanteneffizienz (EQE, gemessen in Prozent) in Abhängigkeit der Leuchtdichte, berechnet aus Strom-Spannungs-Leuchtdichte-Kennlinien (IUL-Kennlinien) unter Annahme einer lambertschen Abstrahlcharakteristik sowie die Lebensdauer bestimmt. Die Elektrolumineszenzspektren werden bei einer Leuchtdichte von 1000 cd/m² bestimmt und daraus die CIE 1931 x und y Farbkoordinaten berechnet. Die Angabe U @ 1000 cd/m² in Tabelle 2 und 4 bezeichnet die Spannung, die für eine Leuchtdichte von 1000 cd/m² benötigt wird. EQE @ 1000 cd/m² schließlich bezeichnet die externe Quanteneffizienz bei einer Betriebsleuchtdichte von 1000 cd/m². LD80 @ 6000 cd/m2 ist die Lebensdauer bis das OLED bei einer Helligkeit von 6000 cd/m² auf 80 % der Anfangsintensität, also auf 4800 cd/m² abgefallen ist. Die Daten, die für die verschiedenen OLEDs erhalten wurden, sind in Tabelle 2 und 4 zusammengefasst angegeben.

Insbesondere eignen sich die erfindungsgemäßen Verbindungen als Lochtransportmaterial, beispielsweise in einer Lochtransportschicht oder in einer Elektronenblockierschicht, in OLEDs. Sie eignen sich als Einzelschicht, aber auch als Mixed- Komponente als HTM, EBM (Elektronenblockiermaterial) oder innerhalb der emittierenden Schicht.

Verglichen mit Vorrichtungen enthaltend NPB (V1 und V4) zeigen alle Proben mit den erfindungsgemäßen Verbindungen höhere Effizienzen kombiniert mit gleichen oder verbesserten Lebensdauern.

Im Vergleich zum Referenzmaterial HTMV1 (V2 und V5) haben die erfindungsgemäßen Verbindungen ähnliche Effizienzen und deutlich bessere Lebensdauern im blauen und bessere Lebensdauern in grün emittierenden Vorrichtungen.

Im Vergleich zum Referenzmaterial HTMV2 (V3 und V6) haben die erfindungsgemäßen Verbindungen ähnliche Effizienzen und deutlich bessere Lebensdauern in grün emittierenden Vorrichtungen und bessere Lebensdauern in blau emittierenden Vorrichtungen.

| **Tabelle 1: Aufbau der OLEDs** | | | | | | | |
|---|---|---|---|---|---|---|---|
| ***Bsp.*** | ***IL*** | ***HTL*** | ***IL*** | ***HTL2*** | ***EBL*** | ***EML*** | ***ETL*** |
| | *Dicke* / *nm* | *Dicke* / *nm* | *Dicke* / *nm* | *Dicke* / *nm* | *Dicke* / *nm* | *Dicke* / *nm* | *Dicke* / *nm* |
| *V1* | *HIL1* | *HIL2* | *HIL1* | | *NPB* | *H1(95%):SEB1(5%)* | *ETM1(50%):LiQ(50%)* |
| | *5 nm* | *140 nm* | *5 nm* | | *20 nm* | *20 nm* | *30 nm* |
| *V2* | *HIL1* | *HIL2* | *HIL1* | *NPB* | *HTMV1* | *H1(95%):SEB1(5%)* | *ETM1(50%):LiQ(50%)* |
| | *5 nm* | *130 nm* | *5 nm* | *10 nm* | *20 nm* | *20 nm* | *30 nm* |
| *V3* | *HIL1* | *HIL2* | *HIL1* | *NPB* | *HTMV2* | *H1(95%):SEB1(5%)* | *ETM1(50%):LiQ(50%)* |
| | *5 nm* | *130 nm* | *5 nm* | *10 nm* | *20 nm* | *20 nm* | *30 nm* |
| *E1* | *HIL1* | *HIL2* | *HIL1* | *NPB* | *HTM1* | *H1(95%):SEB1(5%)* | *ETM1(50%):LiQ(50%)* |
| | *5 nm* | *130 nm* | *5 nm* | *10 nm* | *20 nm* | *20 nm* | *30 nm* |
| *E2* | *HIL1* | *HIL2* | *HIL1* | *NPB* | *HTM2* | *H1(95%):SEB1(5%)* | *ETM1(50%):LiQ(50%)* |
| | *5 nm* | *130 nm* | *5nm* | *10 nm* | *20 nm* | *20 nm* | *30 nm* |
| *E3* | *HIL1* | *HIL2* | *HIL1* | *NPB* | *HTM3* | *H1(95%):SEB1(5%)* | *ETM1(50%):LiQ(50%)* |
| | *5 nm* | *130 nm* | *5 nm* | *10 nm* | *20 nm* | *20 nm* | *30 nm* |
| *E4* | *HIL1* | *HIL2* | *HIL1* | *NPB* | *HTM4* | *H1(95%):SEB1(5%)* | *ETM1(50%):LiQ(50%)* |
| | *5 nm* | *130 nm* | *5 nm* | *10 nm* | *20 nm* | *20 nm* | *30 nm* |
| *E5* | *HIL1* | *HIL2* | *HIL1* | *NPB* | *HTM5* | *H1(95%):SEB1(5%)* | *ETM1(50%):LiQ(50%)* |
| | *5 nm* | *130 nm* | *5 nm* | *10 nm* | *20 nm* | *20 nm* | *30 nm* |
| *E6* | *HIL1* | *HIL2* | *HIL 1* | *NPB* | *HTM6* | *H1(95%):SEB1(5%)* | *ETM1(50%):LiQ(50%)* |
| | *5 nm* | *130 nm* | *5 nm* | *10 nm* | *20 nm* | *20 nm* | *30 nm* |

| **Tabelle 2: Daten der OLEDs** | | | | | |
|---|---|---|---|---|---|
| ***Bsp.*** | ***U*@ *1000 cd*/*m2*** | ***EQE* @ *1000 cd*/*m2*** | ***LD80* @ *6000 cd*/*m²*** | ***CIE*** | |
| | *V* | *%* | *[h]* | *x* | *y* |
| *V1* | *4.7* | *4.8* | *70* | 0.14 | 0.17 |
| *V2* | *4.2* | *7.7* | *100* | 0.14 | 0.16 |
| *V3* | *4.6* | *7.6* | *115* | 0.14 | 0.16 |
| *E1* | *4.5* | *7.5* | *120* | 0.14 | 0.15 |
| *E2* | *4.6* | *7.6* | *125* | 0.14 | 0.16 |
| *E3* | *4.5* | *7.5* | *120* | 0.14 | 0.16 |
| *E4* | *4.6* | *7.6* | *130* | 0.14 | 0.15 |
| *E5* | *4.5* | *7.5* | *120* | 0.14 | 0.16 |
| *E6* | *4.4* | *7.4* | *120* | 0.14 | 0.16 |

| **Tabelle 3: Aufbau der OLEDs** | | | | | | |
|---|---|---|---|---|---|---|
| ***Bsp.*** | ***HTL*** | ***IL*** | ***HTL2*** | ***EBL*** | ***EML*** | ***ETL*** |
| | *Dicke*/*nm* | *Dicke*/*nm* | *Dicke*/*nm* | *Dicke*/*nm* | *Dicke*/*nm* | *Dicke*/*nm* |
| *V4* | *HIL2* | *HIL1* | | *NPB* | *H2(88%):Irpy(12%)* | *ETM1(50%):LiQ(50%)* |
| | *70 nm* | *5 nm* | | *90 nm* | *30 nm* | *40 nm* |
| *V5* | *HIL2* | *HIL1* | *NPB* | *HTMV1* | *H2(88%):Irpy(12%)* | *ETM1(50%):LiQ(50%)* |
| | *70 nm* | *5 nm* | *10 nm* | *80 nm* | *30 nm* | *40 nm* |
| *V6* | *HIL2* | *HIL1* | *NPB* | *HTMV2* | *H2(88%):Irpy(12%)* | *ETM1(50%):LiQ(50%)* |
| | *70 nm* | *5 nm* | *10 nm* | *80 nm* | *30 nm* | *40 nm* |
| *E7* | *HIL2* | *HIL1* | *NPB* | *HTM1* | *H2(88%):Irpy(12%)* | *ETM1(50%):LiQ(50%)* |
| | *70 nm* | *5 nm* | *10 nm* | *80 nm* | *30 nm* | *40 nm* |
| *E8* | *HIL2* | *HIL1* | *NPB* | *HTM2* | *H2(88%):Irpy(12%)* | *ETM1(50%):LiQ(50%)* |
| | *70 nm* | *5 nm* | *10 nm* | *80 nm* | *30 nm* | *40 nm* |
| *E9* | *HIL2* | *HIL1* | *NPB* | *HTM3* | *H2(88%):Irpy(12%)* | *ETM1(50%):LiQ(50%)* |
| | *70 nm* | *5 nm* | *10 nm* | *80 nm* | *30 nm* | *40 nm* |
| *E10* | *HIL2* | *HIL1* | *NPB* | *HTM2* | *H2(88%):Irpy(12%)* | *ETM1(50%):LiQ(50%)* |
| | *70 nm* | *5 nm* | *10 nm* | *80 nm* | *30 nm* | *40 nm* |
| *E11* | *HIL2* | *HIL1* | *NPB* | *HTM3* | *H2(88%):Irpy(12%)* | *ETM1(50%):LiQ(50%)* |
| | *70 nm* | *5 nm* | *10 nm* | *80 nm* | *30 nm* | *40 nm* |
| *E12* | *HIL2* | *HIL1* | *NPB* | *HTM2* | *H2(88%):Irpy(12%)* | *ETM1(50%):LiQ(50%)* |
| | *70 nm* | *5 nm* | *10 nm* | *80 nm* | *30 nm* | *40 nm* |

| **Tabelle 4: Daten der OLEDs** | | | | | |
|---|---|---|---|---|---|
| ***Bsp.*** | ***U @ 1000 cd*/*m2*** | ***EQE @ 1000 cd*/*m2*** | ***LD80 @ 8000 cd*/*m2*** | ***CIE*** | |
| | *V* | *%* | *[h]* | *x* | *y* |
| *V4* | *3.6* | *14.4* | *85* | 0.32 | 0.63 |
| *V5* | *3.6* | *17.8* | *120* | 0.35 | 0.62 |
| *V6* | *3.6* | *17.8* | *115* | 0.34 | 0.62 |
| *E7* | *3.5* | *17.7* | *145* | 0.34 | 0.63 |
| *E8* | *3.5* | *17.3* | *150* | 0.36 | 0.62 |
| *E9* | *3.6* | *17.5* | *140* | 0.35 | 0.62 |
| *E10* | *3.6* | *17.8* | *125* | 0.34 | 0.63 |
| *E11* | *3.6* | *17.7* | *145* | 0.35 | 0.63 |
| *E12* | *3.5* | *17.3* | *145* | 0.35 | 0.62 |

| Tabelle 5: Strukturen der verwendeten Materialien | | |
|---|---|---|
| 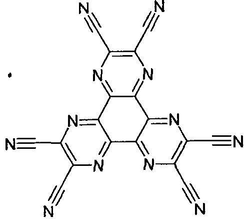 | 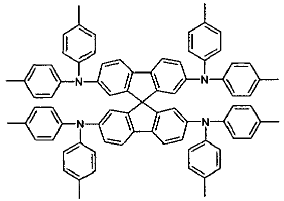 | 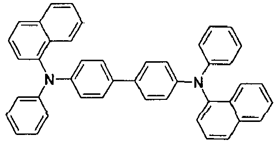 |
| HIL1 | HIL2 | NPB |
| 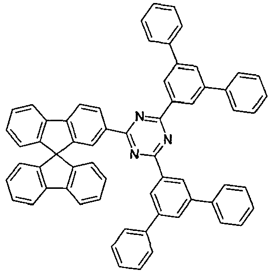 | 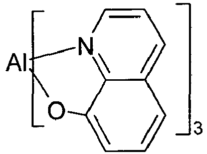 | 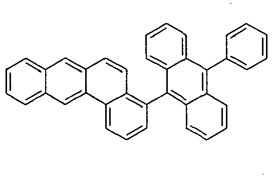 |
| ETM1 | AlQ3 | H1 |
| 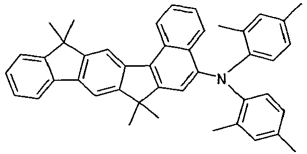 | 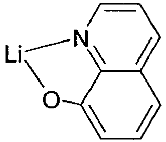 | 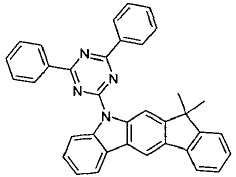 |
| SEB1 | LiQ | H2 |
| 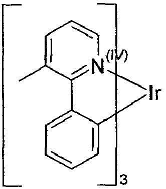 | 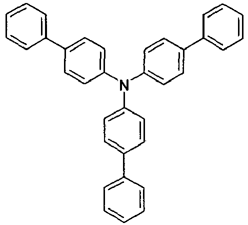 | 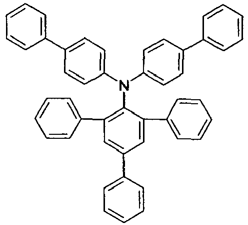 |
| Irpy | HTMV1 | HTMV2 |
| 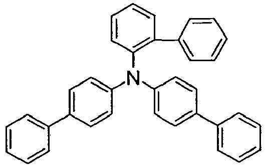 | 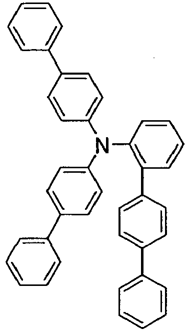 | 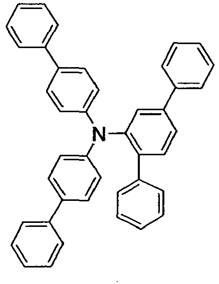 |
| HTM1 | HTM2 | HTM3 |
| 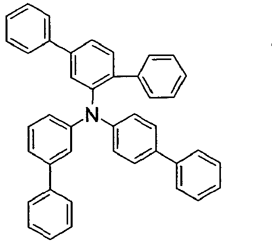 | 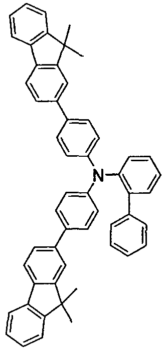 | 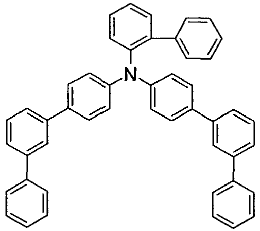 |
| HTM4 | HTM5 | HTM6 |

## Patentansprüche

1. Verbindung der Formel (I) 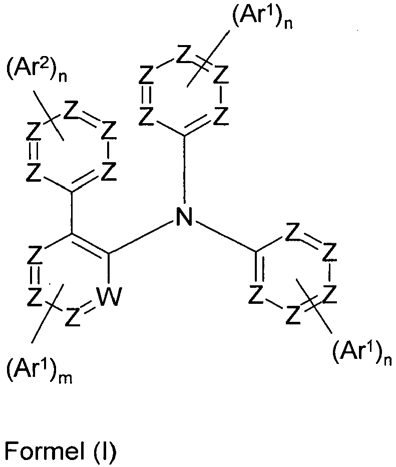 wobei für die auftretenden Symbole und Indices gilt:
Z ist bei jedem Auftreten gleich oder verschieden CR¹ oder N, wobei Z gleich C ist, wenn eine Gruppe Ar¹ oder Ar² gebunden ist;
W ist gleich CH oder N;
Ar¹ ist bei jedem Auftreten gleich oder verschieden ein aromatisches Ringsystem mit 6 bis 30 aromatischen Ringatomen, welches mit einem oder mehreren Resten R¹ substituiert sein kann;
Ar² ist bei jedem Auftreten gleich oder verschieden ein aromatisches Ringsystem mit 6 bis 24 aromatischen Ringatomen, welches mit einem oder mehreren Resten R¹ substituiert sein kann;
R¹ ist bei jedem Auftreten gleich oder verschieden H, D, F, Cl, Br, I, C(=O)R², CN, Si(R²)₃, NO₂, P(=O)(R²)₂, S(=O)R², S(=O)₂R², eine geradkettige Alkyl-, Alkoxy- oder Thio-alkylgruppe mit 1 bis 20 C-Atomen oder eine verzweigte oder cyclische Alkyl-, Alkoxy- oder Thioalkylgruppe mit 3 bis 20 C-Atomen oder eine Alkenyl- oder Alkinylgruppe mit 2 bis 20 C-Atomen, wobei die oben genannten Gruppen jeweils mit einem oder mehreren Resten R² substituiert sein können und wobei eine oder mehrere CH₂-Gruppen in den oben genannten Gruppen durch -R²C=CR²-, -C≡C-, Si(R²)₂, C=O, C=S, C=NR², -C(=O)O-, -C(=O)NR²-, NR², P(=O)(R²), -O-,-S-, SO oder SO₂ ersetzt sein können und wobei ein oder mehrere H-Atome in den oben genannten Gruppen durch D, F, Cl, Br, I, CN oder NO₂ ersetzt sein können, oder ein aromatisches Ringsystem mit 6 bis 30 aromatischen Ring-atomen, das jeweils durch einen oder mehrere Reste R² substituiert sein kann;
R² ist bei jedem Auftreten gleich oder verschieden H, D, F, Cl, Br, I, C(=O)R³, CN, Si(R³)₃, NO₂, P(=O)(R³)₂, S(=O)R³, S(=O)₂R³, eine geradkettige Alkyl-, Alkoxy- oder Thio-alkylgruppe mit 1 bis 20 C-Atomen oder eine verzweigte oder cyclische Alkyl-, Alkoxy- oder Thioalkylgruppe mit 3 bis 20 C-Atomen oder eine Alkenyl- oder Alkinylgruppe mit 2 bis 20 C-Atomen, wobei die oben genannten Gruppen jeweils mit einem oder mehreren Resten R³ substituiert sein können und wobei eine oder mehrere CH₂-Gruppen in den oben genannten Gruppen durch -R³C=CR³-, -C≡C-, Si(R³)₂, C=O, C=S, C=NR³, -C(=O)O-, -C(=O)NR³-, NR³, P(=O)(R³), -O-, -S-, SO oder SO₂ ersetzt sein können und wobei ein oder mehrere H-Atome in den oben genannten Gruppen durch D, F, Cl, Br, I, CN oder NO₂ ersetzt sein können, oder ein aromatisches Ringsystem mit 6 bis 30 aromatischen Ring-atomen, das jeweils durch einen oder mehrere Reste R³ substituiert sein kann;
R³ ist bei jedem Auftreten gleich oder verschieden H, D, F oder ein aliphatischer oder aromatischer organischer Rest mit 1 bis 20 C-Atomen, in dem auch ein oder mehrere H-Atome durch D oder F ersetzt sein können;
n ist bei jedem Auftreten gleich oder verschieden 0, 1, 2, 3, 4 oder 5;
m ist bei jedem Auftreten gleich oder verschieden 0, 1, 2 oder 3;
wobei die drei Gruppen, welche an das zentrale Stickstoffatom binden, als ganze gesehen, nicht alle identisch sind,
wobei die Summe der Indices n mindestens gleich 2 ist, und
wobei die folgenden Verbindungen vom Anspruchsumfang ausgenommen sind:

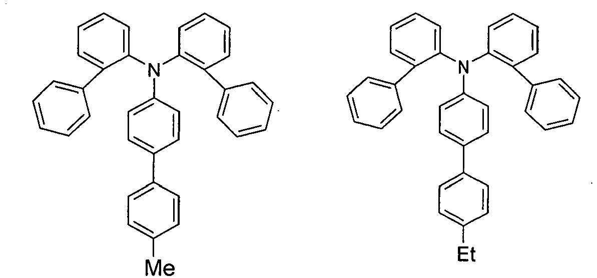

2. Verbindung nach Anspruch 1, **dadurch gekennzeichnet, dass** sie außer der in Formel (I) abgebildeten Triarylaminogruppe keine weitere Arylaminogruppe umfasst.

3. Verbindung nach Anspruch 1 oder 2, **dadurch gekennzeichnet, dass** sie keine Heteroarylgruppe und kein heteroaromatisches Ringsystem umfasst.

4. Verbindung nach einem oder mehreren der Ansprüche 1 bis 3, **dadurch gekennzeichnet, dass** die Gruppe W gleich CH ist.

5. Verbindung nach einem oder mehreren der Ansprüche 1 bis 4, **dadurch gekennzeichnet, dass** 0, 1, 2 oder 3 Gruppen Z in einem Ring gleich N sind, wobei nicht mehr als zwei benachbarte Gruppen Z gleich N sind.

6. Verbindung nach einem oder mehreren der Ansprüche 1 bis 5, **dadurch gekennzeichnet, dass** Ar¹ bei jedem Auftreten gleich oder verschieden ein aromatisches Ringsystem mit 6 bis 18 aromatischen Ringatomen ist, das mit einem oder mehreren Resten R¹ substituiert sein kann.

7. Verbindung nach einem oder mehreren der Ansprüche 1 bis 6, **dadurch gekennzeichnet, dass** mindestens eine der Gruppen Ar¹ in der Verbindung gemäß Formel (I) an den Sechsring in meta-Position zur Bindung an das zentrale Stickstoffatom gebunden ist.

8. Verbindung nach nach einem oder mehreren der Ansprüche 1 bis 7, **dadurch gekennzeichnet, dass** Ar² bei jedem Auftreten gleich oder verschieden ein aromatisches Ringsystem mit 6 bis 12 aromatischen Ringatomen ist, das mit einem oder mehreren Resten R¹ substituiert sein kann.

9. Verbindung nach nach einem oder mehreren der Ansprüche 1 bis 8, **dadurch gekennzeichnet, dass** n gleich 1 oder 2 ist.

10. Verbindung nach nach einem oder mehreren der Ansprüche 1 bis 9, **dadurch gekennzeichnet, dass** m gleich 0 oder 1 ist, bevorzugt 0.

11. Verbindung nach einem oder mehreren der Ansprüche 1 bis 10, **dadurch gekennzeichnet, dass** keine Gruppe Ar¹ am Sechsring in ortho-Position zur Bindung an das zentrale Stickstoffatom gebunden ist.

12. Verfahren zur Herstellung einer Verbindung nach einem oder mehreren der Ansprüche 1 bis 11, **dadurch gekennzeichnet, dass** eine Arylverbindung enthaltend eine Aminogruppe und eine Arylverbindung enthaltend eine Abgangsgruppe in einer übergangsmetallkatalysierten Kupplungsreaktion miteinander verknüpft werden.

13. Oligomer, Polymer oder Dendrimer enthaltend eine oder mehrere Verbindungen nach einem oder mehreren der Ansprüche 1 bis 11, wobei die Bindungen zum Oligomer, Polymer oder Dendrimer an beliebigen, in Formel (I) mit R¹ substituierten Positionen lokalisiert sein können.

14. Formulierung enthaltend mindestens eine Verbindung nach einem oder mehreren der Ansprüche 1 bis 11 oder mindestens ein Polymer, Oligomer oder Dendrimer gemäß Anspruch 13 und mindestens ein Lösungsmittel.

15. Elektronische Vorrichtung, ausgewählt aus organischen integrierten Schaltungen (O-ICs), organischen Feld-Effekt-Transistoren (O-FETs), organischen Dünnfilmtransistoren (O-TFTs), organischen lichtemittierenden Transistoren (O-LETs), organischen Solarzellen (O-SCs), organischen optischen Detektoren, organischen Photorezeptoren, organischen Feld-Quench-Devices (O-FQDs), lichtemittierenden elektrochemischen Zellen (LECs), organischen Laserdioden (O-Laser) und organischen Elektrolumineszenzvorrichtungen (OLEDs), **dadurch gekennzeichnet, dass** sie mindestens eine Verbindung nach einem oder mehreren der Ansprüche 1 bis 11 oder mindestens ein Polymer, Oligomer oder Dendrimer nach Anspruch 13 enthält.

16. Elektronische Vorrichtung nach Anspruch 15, ausgewählt aus organischen Elektrolumineszenzvorrichtungen, **dadurch gekennzeichnet, dass** die Verbindung nach einem oder mehreren der Ansprüche 1 bis 11 in einer oder mehreren der folgenden Schichten eingesetzt wird:
- in einer Lochtransportschicht, einer Lochinjektionsschicht oder einer Elektronenblockierschicht als Lochtransportmaterial,
- in einer emittierenden Schicht als Matrixmaterial in Kombination mit einem oder mehreren fluoreszierenden oder phosphoreszierenden Dotanden.

## Claims

1. Compound of the formula (I) 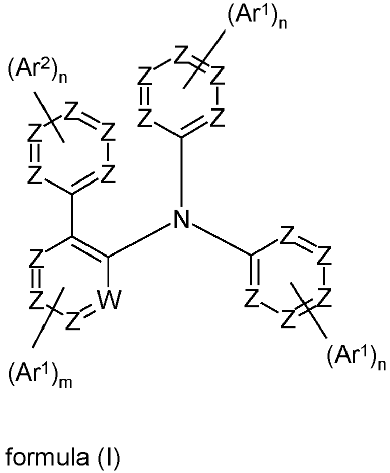 where the following applies to the symbols and indices occurring:
Z is on each occurrence, identically or differently, CR¹ or N, where Z is equal to C if a group Ar¹ or Ar² is bonded;
W is equal to CH or N;
Ar¹ is on each occurrence, identically or differently, an aromatic ring system having 6 to 30 aromatic ring atoms, which may be substituted by one or more radicals R¹;
Ar² is on each occurrence, identically or differently, an aromatic ring system having 6 to 24 aromatic ring atoms, which may be substituted by one or more radicals R¹;
R¹ is on each occurrence, identically or differently, H, D, F, Cl, Br, I, C(=O)R², CN, Si(R²)₃, NO₂, P(=O)(R²)₂, S(=O)R², S(=O)₂R², a straight-chain alkyl, alkoxy or thioalkyl group having 1 to 20 C atoms or a branched or cyclic alkyl, alkoxy or thioalkyl group having 3 to 20 C atoms or an alkenyl or alkynyl group having 2 to 20 C atoms, where the above-mentioned groups may in each case be substituted by one or more radicals R² and where one or more CH₂ groups in the above-mentioned groups may be replaced by -R²C=CR²-, -C≡C-, Si(R²)₂, C=O, C=S, C=NR², -C(=O)O-, -C(=O)NR²-, NR², P(=O)(R²), -O-, -S-, SO or SO₂ and where one or more H atoms in the above-mentioned groups may be replaced by D, F, Cl, Br, I, CN or NO₂, or an aromatic ring system having 6 to 30 aromatic ring atoms, which may in each case be substituted by one or more radicals R²;
R² is on each occurrence, identically or differently, H, D, F, Cl, Br, I, C(=O)R³, CN, Si(R³)₃, NO₂, P(=O)(R³)₂, S(=O)R³, S(=O)₂R³, a straight-chain alkyl, alkoxy or thioalkyl group having 1 to 20 C atoms or a branched or cyclic alkyl, alkoxy or thioalkyl group having 3 to 20 C atoms or an alkenyl or alkynyl group having 2 to 20 C atoms, where the above-mentioned groups may in each case be substituted by one or more radicals R³ and where one or more CH₂ groups in the above-mentioned groups may be replaced by -R³C=CR³-, -C≡C-, Si(R³)₂, C=O, C=S, C=NR³, -C(=O)O-, -C(=O)NR³-, NR³, P(=O)(R³), -O-, -S-, SO or SO₂ and where one or more H atoms in the above-mentioned groups may be replaced by D, F, Cl, Br, I, CN or NO₂, or an aromatic ring system having 6 to 30 aromatic ring atoms, which may in each case be substituted by one or more radicals R³;
R³ is on each occurrence, identically or differently, H, D, F or an aliphatic or aromatic organic radical having 1 to 20 C atoms, in which, in addition, one or more H atoms may be replaced by D or F;
n is on each occurrence, identically or differently, 0, 1, 2, 3, 4 or 5;
m is on each occurrence, identically or differently, 0, 1, 2 or 3;
where the three groups which are bonded to the central nitrogen atom, regarded as a whole, are not all identical,
where the sum of the indices n is at least equal to 2, and
where the following compounds are excluded from the claim scope:

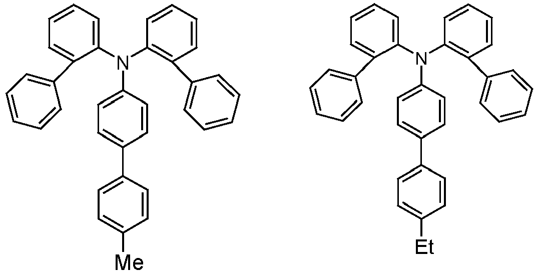

2. Compound according to Claim 1, **characterised in that** it contains no further arylamino group apart from the triarylamino group depicted in formula (I).

3. Compound according to Claim 1 or 2, **characterised in that** it contains no heteroaryl group and no heteroaromatic ring system.

4. Compound according to one or more of Claims 1 to 3, **characterised in that** the group W is equal to CH.

5. Compound according to one or more of Claims 1 to 4, **characterised in that** 0, 1, 2 or 3 groups Z in a ring are equal to N, where not more than two adjacent groups Z are equal to N.

6. Compound according to one or more of Claims 1 to 5, **characterised in that** Ar¹ is on each occurrence, identically or differently, an aromatic ring system having 6 to 18 aromatic ring atoms, which may be substituted by one or more radicals R¹.

7. Compound according to one or more of Claims 1 to 6, **characterised in that** at least one of the groups Ar¹ in the compound of the formula (I) is bonded to the six-membered ring in the meta position to the bond to the central nitrogen atom.

8. Compound according to one or more of Claims 1 to 7, **characterised in that** Ar² is on each occurrence, identically or differently, an aromatic ring system having 6 to 12 aromatic ring atoms, which may be substituted by one or more radicals R¹.

9. Compound according to one or more of Claims 1 to 8, **characterised in that** n is equal to 1 or 2.

10. Compound according to one or more of Claims 1 to 9, **characterised in that** m is equal to 0 or 1, preferably 0.

11. Compound according to one or more of Claims 1 to 10, **characterised in that** no group Ar¹ is bonded on the six-membered ring in the ortho position to the bond to the central nitrogen atom.

12. Process for the preparation of a compound according to one or more of Claims 1 to 11, **characterised in that** an aryl compound containing an amino group and an aryl compound containing a leaving group are linked to one another in a transition-metal-catalysed coupling reaction.

13. Oligomer, polymer or dendrimer containing one or more compounds according to one or more of Claims 1 to 11, where the bonds to the oligomer, polymer or dendrimer may be localised at any desired positions in formula (I) that are substituted by R¹.

14. Formulation comprising at least one compound according to one or more of Claims 1 to 11 or at least one polymer, oligomer or dendrimer according to Claim 13 and at least one solvent.

15. Electronic device selected from organic integrated circuits (O-ICs), organic field-effect transistors (O-FETs), organic thin-film transistors (O-TFTs), organic light-emitting transistors (O-LETs), organic solar cells (O-SCs), organic optical detectors, organic photoreceptors, organic field-quench devices (O-FQDs), light-emitting electrochemical cells (LECs), organic laser diodes (O-lasers) and organic electroluminescent devices (OLEDs), **characterised in that** it contains at least one compound according to one or more of Claims 1 to 11 or at least one polymer, oligomer or dendrimer according to Claim 13.

16. Electronic device according to Claim 15, selected from organic electroluminescent devices, **characterised in that** the compound according to one or more of Claims 1 to 11 is employed in one or more of the following layers:
- in a hole-transport layer, a hole-injection layer or an electron-blocking layer as hole-transport material,
- in an emitting layer as matrix material in combination with one or more fluorescent or phosphorescent dopants.

## Revendications

1. Composé de la formule (I) 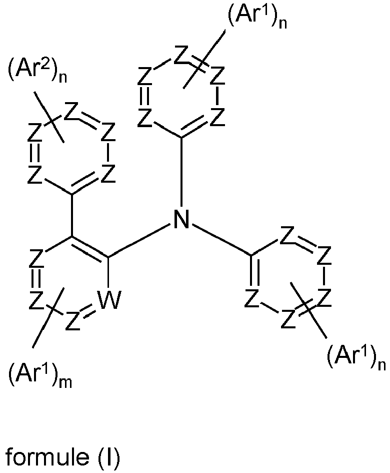 dans laquelle ce qui suit s'applique aux symboles et aux indices qui sont rencontrés :
Z est pour chaque occurrence, de manière identique ou différente, CR¹ ou N, où Z est égal à C si un groupe Ar¹ ou Ar² est lié ;
W est égal à CH ou N ;
Ar¹ est pour chaque occurrence, de manière identique ou différente, un système de cycle aromatique qui comporte de 6 à 30 atomes de cycle aromatique, lequel peut être substitué par un radical ou par plusieurs radicaux R¹ ;
Ar² est pour chaque occurrence, de manière identique ou différente, un système de cycle aromatique qui comporte de 6 à 24 atomes de cycle aromatique, lequel peut être substitué par un radical ou par plusieurs radicaux R¹ ;
R¹ est pour chaque occurrence, de manière identique ou différente, H, D, F, Cl, Br, I, C(=O)R², CN, Si(R²)₃, NO₂, P(=O)(R²)₂, S(=O)R², S(=O)₂R², un groupe alkyle, alcoxy ou thioalkyle en chaîne droite qui comporte de 1 à 20 atome(s) de C ou un groupe alkyle, alcoxy ou thioalkyle ramifié ou cyclique qui comporte de 3 à 20 atomes de C ou un groupe alkényle ou alkynyle qui comporte de 2 à 20 atomes de C, où les groupes qui ont été mentionnés ci-avant peuvent dans chaque cas être substitués par un radical ou par plusieurs radicaux R² et où un ou plusieurs groupe(s) CH₂ dans les groupes qui ont été mentionnés ci-avant peut/peuvent être remplacé(s) par -R²C=CR²-, -C≡C-, Si(R²)₂, C=O, C=S, C=NR², -C(=O)O-, -C(=O)NR²-, NR², P(=O)(R²), -O-, -S-, SO ou SO₂ et où un ou plusieurs atome(s) de H dans les groupes qui ont été mentionnés ci-avant peut/peuvent être remplacé(s) par D, F, Cl, Br, I, CN ou NO₂, ou un système de cycle aromatique qui comporte de 6 à 30 atomes de cycle aromatique, lequel peut dans chaque cas être substitué par un radical ou par plusieurs radicaux R² ;
R² est pour chaque occurrence, de manière identique ou différente, H, D, F, Cl, Br, I, C(=O)R³, CN, Si(R³)₃, NO₂, P(=O)(R³)₂, S(=O)R³, S(=O)₂R³, un groupe alkyle, alcoxy ou thioalkyle en chaîne droite qui comporte de 1 à 20 atome(s) de C ou un groupe alkyle, alcoxy ou thioalkyle ramifié ou cyclique qui comporte de 3 à 20 atomes de C ou un groupe alkényle ou alkynyle qui comporte de 2 à 20 atomes de C, où les groupes qui ont été mentionnés ci-avant peuvent dans chaque cas être substitués par un radical ou par plusieurs radicaux R³ et où un ou plusieurs groupe(s) CH₂ dans les groupes qui ont été mentionnés ci-avant peut/peuvent être remplacé(s) par -R³C=CR³-, -C=C-, Si(R³)₂, C=O, C=S, C=NR³, -C(=O)O-, -C(=O)NR³-, NR³, P(=O)(R³), -O-, -S-, SO ou SO₂ et où un ou plusieurs atome(s) de H dans les groupes qui ont été mentionnés ci-avant peut/peuvent être remplacé(s) par D, F, Cl, Br, I, CN ou NO₂, ou un système de cycle aromatique qui comporte de 6 à 30 atomes de cycle aromatique, lequel peut dans chaque cas être substitué par un radical ou par plusieurs radicaux R³ ;
R³ est pour chaque occurrence, de manière identique ou différente, H, D, F ou un radical organique aliphatique ou aromatique qui comporte de 1 à 20 atome(s) de C, où, en outre, un ou plusieurs atome(s) de H peut/peuvent être remplacé(s) par D ou par F ;
n est pour chaque occurrence, de manière identique ou différente, 0, 1, 2, 3, 4 ou 5 ;
m est pour chaque occurrence, de manière identique ou différente, 0, 1, 2 ou 3 ;
où les trois groupes qui sont liés à l'atome d'azote central, considérés en tant que globalité, ne sont pas tous identiques,
où la somme des indices n est au moins égale à 2, et
où les composés qui suivent sont exclus du cadre de la revendication :

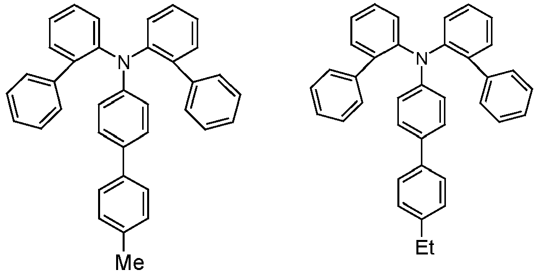

2. Composé selon la revendication 1, **caractérisé en ce qu'**il ne contient pas d'autre groupe arylamino indépendamment du groupe triarylamino qui est représenté au niveau de la formule (I).

3. Composé selon la revendication 1 ou 2, **caractérisé en ce qu'**il ne contient ni un groupe hétéroaryle, ni un système de cycle hétéroaromatique.

4. Composé selon une ou plusieurs des revendications 1 à 3, **caractérisé en ce que** le groupe W est égal à CH.

5. Composé selon une ou plusieurs des revendications 1 à 4, **caractérisé en ce que** 0, 1, 2 ou 3 groupe(s) Z dans un cycle est/sont égal/ égaux à N, où pas plus de deux groupes Z adjacents sont égaux à N.

6. Composé selon une ou plusieurs des revendications 1 à 5, **caractérisé en ce que** Ar¹ est pour chaque occurrence, de manière identique ou différente, un système de cycle aromatique qui comporte de 6 à 18 atomes de cycle aromatique, lequel peut être substitué par un radical ou par plusieurs radicaux R¹.

7. Composé selon une ou plusieurs des revendications 1 à 6, **caractérisé en ce qu'**au moins l'un des groupes Ar¹ dans le composé de la formule (I) est lié au cycle à six éléments au niveau de la position méta sur la liaison sur l'atome d'azote central.

8. Composé selon une ou plusieurs des revendications 1 à 7, **caractérisé en ce que** Ar² est pour chaque occurrence, de manière identique ou différente, un système de cycle aromatique qui comporte de 6 à 12 atomes de cycle aromatique, lequel peut être substitué par un radical ou par plusieurs radicaux R¹.

9. Composé selon une ou plusieurs des revendications 1 à 8, **caractérisé en ce que** n est égal à 1 ou 2.

10. Composé selon une ou plusieurs des revendications 1 à 9, **caractérisé en ce que** m est égal à 0 ou 1, de préférence à 0.

11. Composé selon une ou plusieurs des revendications 1 à 10, **caractérisé en ce qu'**aucun groupe Ar¹ n'est lié au cycle à six éléments au niveau de la position ortho sur la liaison sur l'atome d'azote central.

12. Procédé pour la préparation d'un composé selon une ou plusieurs des revendications 1 à 11, **caractérisé en ce qu'**un composé aryle qui contient un groupe amino et un composé aryle qui contient un groupe partant sont liés l'un à l'autre par l'intermédiaire d'une réaction de couplage catalysée par métal/métaux de transition.

13. Oligomère, polymère ou dendrimère contenant un ou plusieurs composé(s) selon une ou plusieurs des revendications 1 à 11, où les liaisons sur l'oligomère, le polymère ou le dendrimère peuvent être localisées au niveau de n'importe quelles positions souhaitées au niveau de la formule (I) qui sont substituées par R¹.

14. Formulation comprenant au moins un composé selon une ou plusieurs des revendications 1 à 11 ou au moins un polymère, un oligomère ou un dendrimère selon la revendication 13 et au moins un solvant.

15. Dispositif électronique sélectionné parmi les circuits intégrés organiques (O-IC), les transistors à effet de champ organiques (O-FET), les transistors à film mince organiques (O-TFT), les transistors à émission de lumière organiques (O-LET), les cellules solaires organiques (O-SC), les détecteurs optiques organiques, les photorécepteurs organiques, les dispositifs à extinction de champ organiques (O-FQD), les cellules électrochimiques à émission de lumière (LEC), les diodes laser organiques (O-laser) et les dispositifs électroluminescents organiques (OLED), **caractérisé en ce qu'**il contient au moins un composé selon une ou plusieurs des revendications 1 à 11 ou au moins un polymère, un oligomère ou un dendrimère selon la revendication 13.

16. Dispositif électronique selon la revendication 15, sélectionné parmi les dispositifs électroluminescents organiques, **caractérisé en ce que** le composé selon une ou plusieurs des revendications 1 à 11 est utilisé dans une ou plusieurs des couches qui suivent :
- dans une couche de transport de trous, une couche d'injection de trous ou une couche de blocage d'électrons en tant que matériau de transport de trous,
- dans une couche d'émission en tant que matériau de matrice en combinaison avec un ou plusieurs dopant(s) fluorescent(s) ou phosphorescent(s).
